# EUROPEAN PATENT SPECIFICATION

(11) **EP 4 022 624 B1**
(45) Date of publication and mention of the grant of the patent: **11.03.2026**
(21) Application number: 20761818.2
(22) Date of filing: 26.08.2020
(51) Int. Cl.: G16B 25/10, G16B 40/20

(54) **ASSESSING ANTIGEN RETRIEVAL AND TARGET RETRIEVAL PROGRESSION QUANTITATION WITH VIBRATIONAL SPECTROSCOPY**
BEWERTUNG DER ANTIGENWIEDERAUFFINDUNGS- UND ZIELWIEDERAUFFINDUNGSQUANTISIERUNG MITTELS VIBRATIONSSPEKTROSKOPIE
ÉVALUATION DE PRÉLÈVEMENT D'ANTIGÈNE ET QUANTIFICATION DE LA PROGRESSION DE PRÉLÈVEMENT DE CIBLE PAR SPECTROSCOPIE VIBRATIONNELLE

(30) Priority: 28.08.2019 US 201962892688 P
(43) Date of publication of application: 06.07.2022
(73) Proprietor: Ventana Medical Systems, Inc., Tucson, Arizona 85755 (US)
(72) Inventor: BAUER, Daniel, Tucson, Arizona 85755 (US); CHAFIN, David, Tucson, Arizona 85755 (US); DVORAK, Bohuslav, Tucson, Arizona 85755 (US); FERRERI, Gianni, Tucson, Arizona 85755 (US); PHILLIPS-PORTILLO, James, Tucson, Arizona 85755 (US)
(74) Representative: Altmann Stößel Dick Patentanwälte PartG mbB
(86) International application number: PCT/EP2020/073781
(87) International publication number: WO 2021/037869

(56) References cited:
- WO-A1-2017/072320
- US-A1- 2014 186 858
- US-A1- 2014 270 457
- SHI SHAN-RONG ET AL: "Antigen retrieval immunohistochemistry: review and future prospects in research and diagnosis over two decades", JOURNAL OF HISTOCHEMISTRY AND CYTOCHEMISTRY, HISTOCHEMICAL SOCIETY, NEW YORK, NY, US, vol. 59, no. 1, 1 January 2011 (2011-01-01), pages 13 - 32, XP009148078, ISSN: 0022-1554
- SHIM M G ET AL: "THE EFFECTS OF EX VIVO HANDLING PROCEDURES ON THE NEAR-INFRARED RAMAN SPECTRA OF NORMAL MAMMALIAN TISSUES", PHOTOCHEMISTRY AND PHOTOBIOLOGY, WILEY-BLACKWELL PUBLISHING, INC, US, vol. 63, no. 5, 1 May 1996 (1996-05-01), pages 662 - 671, XP009030166, ISSN: 0031-8655, DOI: 10.1111/J.1751-1097.1996.TB05671.X

## Description

### BACKGROUND OF THE DISCLOSURE

Immunohistochemical (IHC) slide staining can be utilized to identify proteins in cells of a tissue section and hence is widely used in the study of different types of cells, such as cancerous cells and immune cells in biological tissue. Thus, IHC staining may be used in research to understand the distribution and localization of the differentially expressed biomarkers of immune cells (such as T-cells or B-cells) in a cancerous tissue for an immune response study. For example, tumors often contain infiltrates of immune cells, which may prevent the development of tumors or favor the outgrowth of tumors.

In-situ hybridization (ISH) can be used to look for the presence of a genetic abnormality or condition such as amplification of cancer-causing genes specifically in cells that, when viewed under a microscope, morphologically appear to be malignant. In situ hybridization (ISH) employs labeled DNA or RNA probe molecules that are anti-sense to a target gene sequence or transcript to detect or localize targeted nucleic acid target genes within a cell or tissue sample. ISH is performed by exposing a cell or tissue sample immobilized on a glass slide to a labeled nucleic acid probe which is capable of specifically hybridizing to a given target gene in the cell or tissue sample. Several target genes can be simultaneously analyzed by exposing a cell or tissue sample to a plurality of nucleic acid probes that have been labeled with a plurality of different nucleic acid tags. By utilizing labels having different emission wavelengths, simultaneous multicolored analysis may be performed in a single step on a single target cell or tissue sample.

Thin tissue sections are used in histology in order to obtain representative information about a tissue sample. The quality of the thin section should meet a number of characteristics in order to be properly representative of the overall tissue region where excision of the sample was performed. Although guidelines can vary according to tissue type and use, the size of the thin section generally should not be less than 2 µm. Typically, tissue sections are prepared in the range between 2 and 5 µm and should not vary in thickness by more than 50% over the lateral extent of the thin section in order to allow for appropriate further processing.

Fixation of tissue and cell samples is used to help ensure that the morphology of the sample and the spatial distribution of biomolecules is preserved and thus enable a diagnosis by a pathologist. Fixation of samples is routinely accomplished using neutral-buffered formalin (NBF). It is believed that the formaldehyde in NBF preserves tissue and cell morphology by forming cross-links between reactive groups on proteins and nucleic acids in the sample, and these cross-links can lead to certain portions of the molecules being rendered undetectable. For example, formaldehyde preserves or fixes tissue or cells predominantly by cross-linking primary amine groups in proteins with other nearby nitrogen atoms in protein or DNA through a -CH₂- linkage. The process of tissue fixation, however, frequently masks antigens on specific proteins for which detection is desirable for diagnostic and prognostic purposes. As such, for immunohistochemical procedures and *in-situ* hybridization procedures, an unmasking process (also known as "antigen retrieval" or "target retrieval" for IHC and ISH, respectively) is often used make protein antigens or nucleic acid targets accessible to detection reagents such as antibodies or probes.

A variety of methods are used to reverse the effects of formalin fixation and provide access to antigens and targets in fixed biological samples. One traditional method comprises treating the tissue with a proteolytic enzyme, which opens up the tissue to allow the antibodies access to the antigens. For some specific antibodies, this method is the preferred antigen retrieval procedure, but it suffers from some limitations. Since the proteolytic enzymes degrade the tissue-proteins, there is a significant risk of destroying the specific epitope in the protein that is recognized by the antibody, thus the antigenicity may be lost rather than being restored. Furthermore, prolonged proteolytic treatment may cause the proteins to lose their three-dimensional structure, which is also significant for antibody-antigen recognition. These factors obviously necessitate close control with incubation times; however, the required incubation time may be dependent on the activity of the enzyme.

Another factor that affects the efficiency of proteolytic target retrieval is the length of fixation of the tissue. It is believed that the degree of cross-linking in the tissue increases with the fixation time and thus tissue that has been fixed for 72 hours will need longer proteolytic treatment than one that has been fixed for 24 hours. Frequently, however the information concerning fixation time is not available, in which case it is not possible to make the corresponding adjustment in enzyme incubation time.

A fundamentally different method comprises heat induced antigen retrieval. The high temperature accelerates hydrolysis of the aldehyde-induced modifications and the aldehyde released is diluted in the water, thus preventing it from reacting with the proteins again. Another method for retrieving the immunoreactivity of formaldehyde cross-linked antigens involves the thermal processing using heat or high energy treatment of the samples. Such a method is described in e.g. U.S. Pat. No. 5,244,787, wherein formaldehyde-fixed tissue preparations are submersed in water and subjected to microwave energy. Yet another method for retrieving antigens from formaldehyde-fixed tissues is the use of a pressure cooker, either in combination with a microwave or in the form of an autoclave, such as described in Norton, 1994. J. Pathos. 173(4):371-9 and Taylor et al. 1996. Biotech Histochem 71(5):263-70.

In addition to pure distilled water, many reagents are used routinely for antigen retrieval including Tris, Urea, EDTA, Citrate and saline buffers. Furthermore, many solutions contain detergents, the selection comprising both ionic and non-ionic surfactants. In spite of the extensive research in the area of staining enhancement, no universal method or reagent has been identified that works with all subsequent immunohistochemical or immunocytochemical procedures.

WO 2017/072320 A1 method of evaluating the quality state (such as a fixation status) of a cellular sample is described. A MIR spectrum of the sample is obtained, and a classification or quantification algorithm is applied to the MIR spectrum to identify features indicative of the quality state and/or to classify the sample. The quality state may then be used to determine whether the sample is appropriate for an analytical method and/or whether remedial processing (such as further fixation) is appropriate.

### BRIEF SUMMARY OF THE DISCLOSURE

Antigen retrieval has become a mainstay of modern immunohistochemistry because it unmasks protein epitopes so that they can be detected by IHC based detection. Likewise, target retrieval is important in unmasking nucleic acids for ISH based detection. Although the exact mechanism of the unmasking of protein epitopes and nucleic acids remains speculative, it is a critical chemical process that enables antigens and nucleic to be detected in formalin-fixed paraffin-embedded tissues. Thus, unmasking is thus fundamentally critical in detecting biomarkers, such as cancer biomarkers. Additionally, the degree to which a tissue is unmasked can significantly impact detected protein and/or nucleic acid expression levels. To date, there exist no analytical methods to measure the quality of an unmasking process or the degree to which any unmasking process is carried out.

To meet this critical and unmet need, Applicant has developed systems and methods of identifying features in acquired spectral data derived from biological specimens and, based on those identified features, predict an unmasking status of the biological specimen. In some embodiments, the systems and methods of the present disclosure identify an "unmasking signature" within acquired spectral data and compute a predicted unmasking status based on that identified unmasking signature. Applicant demonstrates that changes in the molecular composition (e.g. changes in tissue biostructure and/or the availability of immunorecognition sites based on conformational changes of those recognition sites) of a biological specimen may be manifested in acquired vibrational spectral data of the biological specimen, and that shifts in the vibrational spectral data may be exploited to predict an unmasking status (e.g. unmasking duration, unmasking temperature, etc.) of the biological specimen.

In view of the foregoing, Applicant provides a system and method which advantageously enables the quantitative and/or qualitative prediction of the unmasking status of a biological specimen. For example, the systems and methods facilitate the quantitative determination of a degree of unmasking of a biological specimen, where the degree of unmasking may correspond to the conditions of any unmasking process (e.g. temperature, duration, reagents employed, pressure, the presence of introduced energy other than heat, or any combination thereof). Not only does Applicant demonstrate herein that the disclosed systems and methods may accurately predict an unmasking status of a biological specimen, but that the systems and methods enable accurate predictions to be made for various tissue types (e.g. breast tissue, tonsil tissue). Thus, the presently disclosed systems and methods are applicable to any type of histology or cytology specimen. In addition, Applicant further demonstrates that the systems and methods of the present disclosure are applicable to both antigen retrieval processes and target retrieval processes. Overall, Applicant submits that the discloses systems enable quick and accurate prediction of an unmasking status of a biological specimen through the use certain algorithms, e.g. of machine learning algorithms or other automated algorithms, ultimately providing for improved IHC and/or ISH assay results and patient care.

In view of the foregoing, a first aspect of the present disclosure is a system for predicting an unmasking status of a test biological specimen treated in an unmasking process for retrieving antigens or targets and/or improving detection of antigens, amino acids, peptides, proteins, nucleic acids, and/or other targets in fixed tissue, the system comprising: (i) one or more processors, and (ii) one or more memories coupled to the one or more processors, the one or more memories to store computer-executable instructions that, when executed by the one or more processors, cause the system to perform operations comprising: (a) obtaining test vibrational spectral data from the test biological specimen, wherein the test spectral data comprises vibrational spectral data derived from at least a portion of the biological specimen; (b) deriving unmasking features from the obtained test spectral data using a trained unmasking status estimation engine wherein the unmasking status estimation engine is trained using one or more training spectral data sets, wherein each training spectral data set of the one or more training spectral data sets comprises a plurality of training vibrational spectra derived from a plurality of differentially unmasked training tissue samples, and wherein each training spectral data set comprises one or more class labels, wherein the one or more class labels are selected from a known unmasking duration, a known unmasking temperature, and a qualitative assessment of an unmasking state; and (c) predicting the unmasking status of the test biological specimen based on the derived unmasking features. The unmasking status comprises a predicted duration of unmasking and/or a predicted temperature of unmasking. In some embodiments, the unmasking process is an antigen retrieval process. In some embodiments, unmasking process is a target retrieval process.

In some embodiments, the unmasking status further comprises an estimate of tissue quality.

In some embodiments, each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions. In some embodiments, the different unmasking conditions comprise holding a temperature of unmasking constant while varying the duration of unmasking. In some embodiments, the different unmasking conditions comprise holding a duration of unmasking constant while varying the temperature of unmasking. Exemplarily, the different unmasking conditions comprise varying both the duration of unmasking and the temperature of unmasking. Exemplarily, the different unmasking conditions comprise varying the unmasking agent used for unmasking. Exemplarily, the different unmasking conditions comprise varying the pressure used for unmasking.

In some embodiments, the obtained test spectral data comprises an averaged vibrational spectrum derived from a plurality of normalized and corrected vibrational spectra. In some embodiments, the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region. In some embodiments, the acquired vibrational spectrum from each individual region is corrected by: (i) compensating each acquired vibrational spectrum for atmospheric effects to provide an atmospheric corrected vibrational spectrum; and (ii) compensating the atmospheric corrected vibrational spectrum for scattering.

In some embodiments, the trained unmasking status estimation engine comprises a machine learning algorithm based on dimensionality reduction. In some embodiments, the dimensionality reduction comprises a projection onto latent structure regression model. In some embodiments, the dimensionality reduction comprises a principal component analysis plus discriminant analysis. In some embodiments, the trained unmasking status estimation engine comprises a neural network.

In some embodiments, the system further comprises operations for assessing whether test biological specimen is suitable for labeling with one or more specific binding entities. In some embodiments, the obtained test spectral data comprises vibrational spectral information for at least an amide I band. In some embodiments, the obtained test spectral data comprises vibrational spectral information for wavelengths ranging from between about 3200 to about 3400 cm-1. In some embodiments, the obtained test spectral data comprises vibrational spectral information for wavelengths ranging from between about 2800 to about 2900 cm-1. In some embodiments, the obtained test spectral data comprises vibrational spectral information for wavelengths ranging from between about 1020 to about 1100 cm-1. In some embodiments, the obtained test spectral data comprises vibrational spectral information for wavelengths ranging from between about 1520 to about 1580 cm-1.

A second aspect of the present disclosure is a non-transitory computer-readable medium storing instructions for predicting an unmasking status of a test biological specimen treated in an unmasking process for retrieving antigens or targets and/or improving detection of antigens, amino acids, peptides, proteins, nucleic acids, and/or other targets in fixed tissue comprising: (a) obtaining test spectral data from the test biological specimen, wherein the test spectral data comprises vibrational spectral data derived from at least a portion of the biological specimen; (b) deriving unmasking features from the obtained test spectral data using a trained unmasking status estimation engine, wherein the unmasking status estimation engine is trained using training spectral data sets acquired from a plurality of differentially unmasked training biological specimens and wherein the training spectral data sets comprise class labels of at least unmasking duration and unmasking temperature; and (c) predicting the unmasking status of the test biological specimen treated in the unmasking process based on the derived unmasking features. The unmasking status comprises a predicted duration of unmasking and/or a predicted temperature of unmasking.

In some embodiments, the unmasking status comprises an estimate of tissue quality.

In some embodiments, each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions. In some embodiments, the different unmasking conditions comprise holding a temperature of unmasking constant while varying the duration of unmasking. In some embodiments, the different unmasking conditions comprise holding a duration of unmasking constant while varying the temperature of unmasking. In some embodiments, the different unmasking conditions comprise varying both the duration of unmasking and the temperature of unmasking. Exemplarily, the different unmasking conditions comprise varying the unmasking agent used for unmasking. Exemplarily, the different unmasking conditions comprise varying the pressure used for unmasking.

Exemplarily, the training biological specimens comprise the same tissue type as the test biological specimen. Exemplarily, the training biological specimens comprise a different tissue type than the test biological specimen. In some embodiments, the unmasking process is an antigen retrieval process. In some embodiments, the unmasking process is a target retrieval process. Exemplarily, the test biological specimen is unstained. Exemplarily, the test biological specimen is stained for the presence of one or more biomarkers.

Exemplarily, the obtained test spectral data comprises vibrational spectral information for at least an amide I band. Exemplarily, the obtained test spectral data comprises vibrational spectral information for wavelengths ranging from between about 3200 to about 3400 cm-1. Exemplarily, the obtained test spectral data comprises vibrational spectral information for wavelengths ranging from between about 2800 to about 2900 cm-1. Exemplarily, the obtained test spectral data comprises vibrational spectral information for wavelengths ranging from between about 1020 to about 1100 cm-1. Exemplarily, the obtained test spectral data comprises vibrational spectral information for wavelengths ranging from between about 1520 to about 1580 cm-1.

A third aspect of the present disclosure is a method for predicting an unmasking status of a test biological specimen treated in an unmasking process for retrieving antigens or targets and/or improving detection of antigens, amino acids, peptides, proteins, nucleic acids, and/or other targets in fixed tissue comprising: (a) obtaining test spectral data from the test biological specimen, wherein the obtained test spectral data comprises vibrational spectral data derived from at least a portion of the biological specimen; (b) deriving unmasking features from the obtained test spectral data using a trained unmasking status estimation engine, wherein the unmasking status estimation engine is trained using training spectral data sets acquired from a plurality of differentially unmasked training biological specimens and wherein the training spectral data sets comprise class labels of at least unmasking duration and unmasking temperature; and (c) predicting at least one of a duration or a temperature of the unmasking process to which the test unmasked biological specimen was subjected based on the derived unmasking features. The unmasking status comprises a predicted duration of unmasking ans/or a predicted temperature of unmasking. In some embodiments, the unmasking process is an antigen retrieval process. In some embodiments, unmasking process is a target retrieval process.

In some embodiments, the unmasking status further comprises at least one of an estimate of tissue quality and an estimate of damage incurred during the unmasking process. In some embodiments, the trained unmasking status estimation engine comprises a machine learning algorithm based on a projection onto latent structure regression model. In some embodiments, the trained unmasking status estimation engine comprises a machine learning algorithm based on principal component analysis and discriminate analysis. In some embodiments, the trained unmasking status estimation engine comprises a neural network.

Exemplarily, the obtained test spectral data comprises vibrational spectral information for at least an amide I band. Exemplarily, the obtained test spectral data comprises vibrational spectral information for wavelengths ranging from between about 3200 to about 3400 cm-1. Exemplarily, the obtained test spectral data comprises vibrational spectral information for wavelengths ranging from between about 2800 to about 2900 cm-1. Exemplarily, the obtained test spectral data comprises vibrational spectral information for wavelengths ranging from between about 1020 to about 1100 cm-1. Exemplarily, the obtained test spectral data comprises vibrational spectral information for wavelengths ranging from between about 1520 to about 1580 cm-1.

### BRIEF DESCRIPTION OF THE FIGURES

For a general understanding of the features of the disclosure, reference is made to the drawings. In the drawings, like reference numerals have been used throughout to identify identical elements.
FIG. 1 illustrates a representative digital pathology system including an image acquisition device and a computer system in accordance with one embodiment of the present disclosure.
FIG. 2 sets forth various modules that can be utilized in a system or within a digital pathology workflow to quantitatively or qualitatively predict an unmasking status of a test biological sample in accordance with one embodiment of the present disclosure.
FIG. 3 sets forth a flowchart illustrating the various steps of quantitatively or qualitatively predicting an unmasking status of a test biological specimen using a trained unmasking status estimation engine in accordance with one embodiment of the present disclosure.
FIG. 4A illustrates the process of obtaining a plurality of training tissue samples for differential unmasking from two different training biological specimens in accordance with one embodiment of the present disclosure.
FIG. 4B illustrates the differential unmasking of a plurality of training tissue samples obtained from two different training biological specimens in accordance with one embodiment of the present disclosure.
FIG. 4C sets forth a flowchart illustrating the various steps of acquiring vibrational spectra for a training biological specimen in accordance with one embodiment of the present disclosure.
FIG. 5 sets forth a flowchart illustrating the various steps of acquiring an averaged vibrational spectrum for a test biological specimen in accordance with one embodiment of the present disclosure.
FIG. 6 sets forth a flowchart illustrating the various steps correcting, normalizing, and averaging acquired spectra derived from a biological specimen, including test biological specimens and training biological specimens, in accordance with one embodiments of the present disclosure.
FIG. 7A sets forth a graph of the first principal component (PC1) versus the second principal component (PC2) of the mid-IR spectra from differentially retrieved tissue samples. The solid lines represent the output from the discriminate analysis, which calculates boundary lines to separate two different retrieval conditions.
FIG. 7B sets forth confusion matrices for the PCA+DA with increasing numbers of principal components. It is believed that the best classification accuracy is achieved with 5 principal components.
FIG. 8 provides an example of tonsil tissues labeled with antisera raised against Ki-67. Image analysis was conducted only on tonsil tissue (circled portion in left image). Connective tissue that sometimes showed high background but was not present in other sections was excluded.
FIG. 9 provides a visible image of example tissue section having multiple regions identified. The figure further provides an example of a collected, averaged, processed, and normalized vibrational spectrum from the indicated region in visible image.
FIG. 10A provides mid-IR absorption spectra, specifically illustrating a protein band of within the acquired mid-IR spectra,
FIG. 10B sets forth the peak location of the Amide I band's first derivative versus the band's FWHM, which elucidates that un-retrieved spectra have a significantly different spectra than the other retrieved tissues.
FIG. 11 sets forth an example of training an unmasking status estimation engine, and specifically a PLSR machine learning algorithm. Initially, the model is trained with input vibrational spectra with a known classification, and a model is developed which assigns a weight to each wavelength corresponding roughly to how correlated (or anticorrelated) each wavelength is to the response (e.g. unmasking time). Finally, the model is applied to the vibrational spectral data it was trained on to assess how accurately it predicts unmasking time.
FIG. 12 sets forth experimental unmasking time versus unmasking status estimation engine predicted unmasking time. The unmasking status estimation engine was trained on "X" data points (accuracy = 2 minutes) and performance was evaluated on blinded spectra as indicated with purple circles (accuracy = 8 minutes).
FIG. 13 provides a photograph of four tissues imaged with mid-IR in the time-temperature course. The unmasking status estimation engine was trained on the tissues in the bottom row and those in the right side (circled portion including three tissue sections) and the predictive power of the unmasking status estimation engine was evaluated with the tissue in the upper left section of the image (circled portion including only a single tissue section).
FIG. 14 illustrates experimental unmasking temperatures versus predicted unmasking temperatures (such as predicted using a trained unmasking status estimation engine) exclusively for blinded tonsil (shown in FIG. 13). The average predictive accuracy was about 1.3°C.
FIG. 15 illustrates a serial section derived from a tonsil sample that were retrieved either for 60 minutes at 98.6°C. Overlaid points illustrate the sampling pattern used to collect mid-IR spectra for analysis.

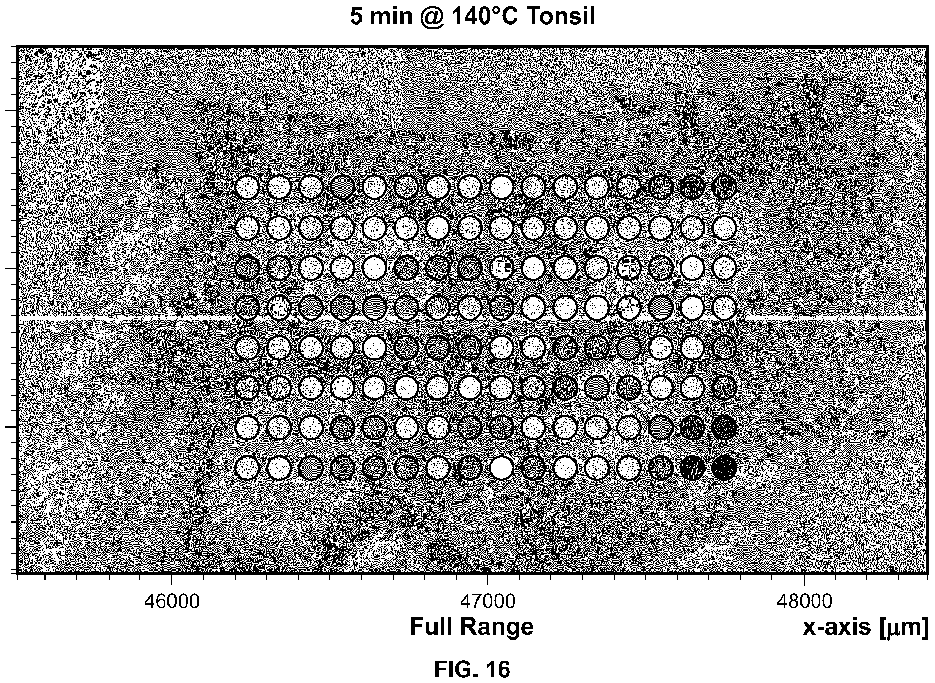

FIG. 16 illustrates a serial section derived from a tonsil sample that were retrieved either for 5 minutes at 140°C. Overlaid points illustrate the sampling pattern used to collect mid-IR spectra for analysis.
FIG. 17A provides a graph which illustrates how many components are needed in the PLSR model to capture the variance in the X and Y variables.
FIG. 17B provides a graph which illustrates how the mean squared predictive error of the model (MSPE) increases with number of components.
FIG. 17C provides a graph which illustrates the performance of the model on the training set and the hold-out validation data. The predictive error is identical for both types of data indicating a well-trained model that has identified the true signature of retrieval in the mid-IR spectra.
FIG. 18 illustrates differential bands from tonsil samples retrieved with low and high temperature retrieval. Each graph is labeled with the likely molecular origins of mid-IR absorption in each respective mid-IR region.
FIG. 19 illustrates that the training unmasking status estimation engine was able to distinguish low versus high temperature retrieval, which also stain differently for C4d.
FIG. 20 provides graphs of the mid-IR spectra (top row) and mid-IR first derivative (bottom row) centered on the Amide I region (left column) and the Amide A region (right column) in which visible difference are observed between the different unmasking conditions.
FIGS. 21A and 21B illustrate amide I Peak location of the Amide I band's first derivative versus the band's FWHM for all duplicates of an unmasking time (FIG. 21A) and the average deformation for each respective unmasking temperature (FIG. 21B).
FIG. 22 provides experimental unmasking temperature versus model predicted unmasking temperature. The unmasking status estimation engine was trained on "Training" spectra and performance was evaluated on "Holdout" spectra (accuracy = 2.02°C). Displayed statistics are exclusively between holdout data as determined by a two-tail ranksum test.
FIG. 23A provides mid-IR absorption spectra of breast tissue after CC1 and CC2 based retrievals.
FIG. 23B provides a difference in absorption spectra between the average of CC1 and CC2 based breast tissue of FIG. 23A.
FIG. 24 provides mid-IR first derivative of CC1 and CC2 based retrievals (A); a difference in mid-IR first derivatives (B). Zoomed in views of bands centered near: 1200 cm-1 (C), 2880cm-1 (D), and E) 1600cm-1 (E).
FIG. 25 sets forth zoomed in views of several bands that are well-differentiated between CC1 and CC2 based retrievals (top row). The figure also sets forth a distribution of spectra for CC1 and CC2 retrieval at wavenumbers of (A) 1265cm-1, (B) 1472cm-1, (C) 1664 cm -1, and (D) 2862cm-1 (bottom row).
FIG. 26 illustrates typical FT-IR and Raman spectra for collagen.
FIG. 27 provides a table setting forth the infrared and Raman characteristic frequencies of biological samples.

### DETAILED DESCRIPTION

It should also be understood that, unless clearly indicated to the contrary, in any methods claimed herein that include more than one step or act, the order of the steps or acts of the method is not necessarily limited to the order in which the steps or acts of the method are recited.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described.

As used herein, the singular terms "a," "an," and "the" include plural referents unless context clearly indicates otherwise. Similarly, the word "or" is intended to include "and" unless the context clearly indicates otherwise. The term "includes" is defined inclusively, such that "includes A or B" means including A, B, or A and B.

As used herein in the specification and in the claims, "or" should be understood to have the same meaning as "and/or" as defined above. For example, when separating items in a list, "or" or "and/or" shall be interpreted as being inclusive, for example, the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (for example "one or the other but not both") when preceded by terms of exclusivity, such as "either," "one of," "only one of" or "exactly one of." "Consisting essentially of," when used in the claims, shall have its ordinary meaning as used in the field of patent law.

The terms "comprising," "including," "having," and the like are used interchangeably and have the same meaning. Similarly, "comprises," "includes," "has," and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a device having components a, b, and c" means that the device includes at least components a, b and c. Similarly, the phrase: "a method involving steps a, b, and c" means that the method includes at least steps a, b, and c. Moreover, while the steps and processes may be outlined herein in a particular order, the skilled artisan will recognize that the ordering steps and processes may vary.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

As used herein, the term "antigen" refers to a substance to which an antibody, an antibody analog (e.g. an aptamer), or antibody fragment binds. Antigens may be endogenous whereby they are generated within the cell as a result of normal or abnormal cell metabolism, or because of viral or intracellular bacterial infections. Endogenous antigens include xenogenic (heterologous), autologous and idiotypic or allogenic (homologous) antigens. Antigens may also be tumor-specific antigens or presented by tumor cells. In this case, they are called tumor-specific antigens (TSAs) and, in general, result from a tumor-specific mutation. Antigens may also be tumor-associated antigens (TAAs), which are presented by tumor cells and normal cells. Antigen also includes CD antigens, which refers any of a number of cell-surface markers expressed by leukocytes and can be used to distinguish cell lineages or developmental stages. Such markers can be identified by specific monoclonal antibodies and are numbered by their cluster of differentiation.

As used herein, the term "biological specimen," "sample," or "tissue sample" refers to any sample including a biomolecule (such as a protein, a peptide, a nucleic acid, a lipid, a carbohydrate, or a combination thereof) that is obtained from any organism including viruses. Other examples of organisms include mammals (such as humans; veterinary animals like cats, dogs, horses, cattle, and swine; and laboratory animals like mice, rats and primates), insects, annelids, arachnids, marsupials, reptiles, amphibians, bacteria, and fungi. Biological specimens include tissue samples (such as tissue sections and needle biopsies of tissue), cell samples (such as cytological smears such as Pap smears or blood smears or samples of cells obtained by microdissection), or cell fractions, fragments or organelles (such as obtained by lysing cells and separating their components by centrifugation or otherwise). Other examples of biological specimens include blood, serum, urine, semen, fecal matter, cerebrospinal fluid, interstitial fluid, mucous, tears, sweat, pus, biopsied tissue (for example, obtained by a surgical biopsy or a needle biopsy), nipple aspirates, cerumen, milk, vaginal fluid, saliva, swabs (such as buccal swabs), or any material containing biomolecules that is derived from a first biological specimen. In certain embodiments, the term "biological specimen" as used herein refers to a sample (such as a homogenized or liquefied sample) prepared from a tumor or a portion thereof obtained from a subject.

As used herein, the terms "biomarker" or "marker" refer to a measurable indicator of some biological state or condition. In particular, a biomarker may be a protein or peptide, e.g. a surface protein, that can be specifically stained, and which is indicative of a biological feature of the cell, e.g. the cell type or the physiological state of the cell. An immune cell marker is a biomarker that is selectively indicative of a feature that relates to an immune response of a mammal. A biomarker may be used to determine how well the body responds to a treatment for a disease or condition or if the subject is predisposed to a disease or condition. In the context of cancer, a biomarker refers to a biological substance that is indicative of the presence of cancer in the body. A biomarker may be a molecule secreted by a tumor or a specific response of the body to the presence of cancer. Genetic, epigenetic, proteomic, glycomic, and imaging biomarkers can be used for cancer diagnosis, prognosis, and epidemiology. Such biomarkers can be assayed in non-invasively collected biofluids like blood or serum. Several gene and protein based biomarkers have already been used in patient care including but, not limited to, AFP (Liver Cancer), BCR- ABL (Chronic Myeloid Leukemia), BRCA1 / BRCA2 (Breast/Ovarian Cancer), BRAF V600E (Melanoma/Colorectal Cancer), CA-125 (Ovarian Cancer), CA19.9 (Pancreatic Cancer), CEA (Colorectal Cancer), EGFR (Non-small-cell lung carcinoma), HER-2 (Breast Cancer), KIT (Gastrointestinal stromal tumor), PSA (Prostate Specific Antigen), S100 (Melanoma), and many others. Biomarkers may be useful as diagnostics (to identify early stage cancers) and/or prognostics (to forecast how aggressive a cancer is and/or predict how a subject will respond to a particular treatment and/or how likely a cancer is to recur). Examples of other biomarkers are disclosed herein.

As used herein, the term "cytological sample" refers to a cellular sample in which the cells of the sample have been partially or completely disaggregated, such that the sample no longer reflects the spatial relationship of the cells as they existed in the subject from which the cellular sample was obtained. Examples of cytological samples include tissue scrapings (such as a cervical scraping), fine needle aspirates, samples obtained by lavage of a subject, et cetera.

As used herein, the term "immunohistochemistry" refers to a method of determining the presence or distribution of an antigen in a sample by detecting interaction of the antigen with a specific binding agent, such as an antibody. A sample is contacted with an antibody under conditions permitting antibody-antigen binding. Antibody-antigen binding can be detected by means of a detectable label conjugated to the antibody (direct detection) or by means of a detectable label conjugated to a secondary antibody, which binds specifically to the primary antibody (indirect detection). In some instances, indirect detection can include tertiary or higher antibodies that serve to further enhance the detectability of the antigen. Examples of detectable labels include enzymes, fluorophores and haptens, which in the case of enzymes, can be employed along with chromogenic or fluorogenic substrates.

As used herein, the term "slide" refers to any substrate (e.g., substrates made, in whole or in part, glass, quartz, plastic, silicon, etc.) of any suitable dimensions on which a biological specimen is placed for analysis, and more particularly to a "microscope slide" such as a standard 3 inch by 1 inch microscope slide or a standard 75 mm by 25 mm microscope slide. Examples of biological specimens that can be placed on a slide include, without limitation, a cytological smear, a thin tissue section (such as from a biopsy), and an array of biological specimens, for example a tissue array, a cellular array, a DNA array, an RNA array, a protein array, or any combination thereof. Thus, in one embodiment, tissue sections, DNA samples, RNA samples, and/or proteins are placed on a slide at particular locations. In some embodiments, the term slide may refer to SELDI and MALDI chips, and silicon wafers.

As used herein, the term "specific binding entity" refers to a member of a specific-binding pair. Specific binding pairs are pairs of molecules that are characterized in that they bind each other to the substantial exclusion of binding to other molecules (for example, specific binding pairs can have a binding constant that is at least 103 M-1 greater, 104 M-1 greater or 105 M-1 greater than a binding constant for either of the two members of the binding pair with other molecules in a biological sample). Particular examples of specific binding moieties include specific binding proteins (for example, antibodies, lectins, avidins such as streptavidins, and protein A). Specific binding moieties can also include the molecules (or portions thereof) that are specifically bound by such specific binding proteins.

As used herein, the term "spectra data" encompasses raw image spectral data acquired from a biological specimen or any portion thereof, such as with a spectrometer.

As used herein, the term "spectrum" refers to information (absorption, transmission, reflection) obtained "at" or within a certain wavelength or wavenumber range of electromagnetic radiation. A wavenumber range can be as large as 4000 cm-1 or as narrow as 0.01 cm-1. Note that a measurement at a so-called "single laser wavelength" will typically cover a small spectral range (e.g., the laser linewidth) and will hence be included whenever the term "spectrum" is used throughout this manuscript. A transmission measurement at a fixed wavelength setting of a quantum cascade laser, for example, shall hereby fall under the term spectrum throughout this application.

As used herein, the terms "stain," "staining," or the like as used herein generally refers to any treatment of a biological specimen that detects and/or differentiates the presence, location, and/or amount (such as concentration) of a particular molecule (such as a lipid, protein or nucleic acid) or particular structure (such as a normal or malignant cell, cytosol, nucleus, Golgi apparatus, or cytoskeleton) in the biological specimen. For example, staining can provide contrast between a particular molecule or a particular cellular structure and surrounding portions of a biological specimen, and the intensity of the staining can provide a measure of the amount of a particular molecule in the specimen. Staining can be used to aid in the viewing of molecules, cellular structures and organisms not only with bright-field microscopes, but also with other viewing tools, such as phase contrast microscopes, electron microscopes, and fluorescence microscopes. Some staining performed by the system can be used to visualize an outline of a cell. Other staining performed by the system may rely on certain cell components (such as molecules or structures) being stained without or with relatively little staining other cell components. Examples of types of staining methods performed by the system include, without limitation, histochemical methods, immunohistochemical methods, and other methods based on reactions between molecules (including non-covalent binding interactions), such as hybridization reactions between nucleic acid molecules. Particular staining methods include, but are not limited to, primary staining methods (e.g., H&E staining, Pap staining, etc.), enzyme-linked immunohistochemical methods, and in situ RNA and DNA hybridization methods, such as fluorescence in situ hybridization (FISH).

As used herein, the term "target" refers to any molecule for which the presence, location and/or concentration is or can be determined. Examples of target molecules include proteins, epitopes, nucleic acid sequences, and haptens, such as haptens covalently bonded to proteins. Target molecules are typically detected using one or more conjugates of a specific binding molecule and a detectable label.

As used herein, the term "tissue sample" shall refer to a cellular sample that preserves the cross-sectional spatial relationship between the cells as they existed within the subject from which the sample was obtained. "Tissue sample" shall encompass both primary tissue samples (for example, cells and tissues produced by the subject) and xenografts (for example foreign cellular samples implanted into a subject).

As used herein, the terms "unmask", or "unmasking" are commonly used in the field of tissue diagnostics and refer to retrieving antigens or targets and/or improving the detection of antigens, amino acids, peptides, proteins, nucleic acids, and/or other targets in fixed tissue. For example, it is believed that antigenic sites that can otherwise go undetected, for example, may be revealed by breaking some of the protein cross-links surrounding the antigen during the unmasking. In some embodiments, antigens and/or other targets are unmasked through the application of one or more unmasking agents, heat, and/or pressure. In some embodiments, only one or more unmasking agents are applied to the specimen to effectuate unmasking (for example no heating or pressure is required). In other embodiments, only heat is applied to effectuate unmasking. In some embodiments, unmasking may occur only in the presence of water and added heat. Unmasking is further described in United States Patent Publication Nos. 2009/0170152 and 2009/0104654. Examples of suitable unmasking agents are described herein.

### OVERVIEW

The present disclosure describes systems and methods for predicting an unmasking status of a biological specimen. As used herein, the term "unmasking status" refers to the unmasking state of a biological specimen subjected to one or more unmasking processes. It is believed that the unmasking status is largely dependent on the conditions in which the biological specimen was subjected to during the unmasking process, and such conditions include temperature, time (for example the duration of the unmasking process), pressure, the one or more unmasking agents utilized to effectuate unmasking, whether external energy (UV, microwave) was supplied to the specimen, etc. Thus, the unmasking status provides insight into the degree and/or quality of an unmasking process, and any unmasking status "output" may include, for example, a predicted duration of an unmasking process or the temperature in which an unmasking process was carried out.

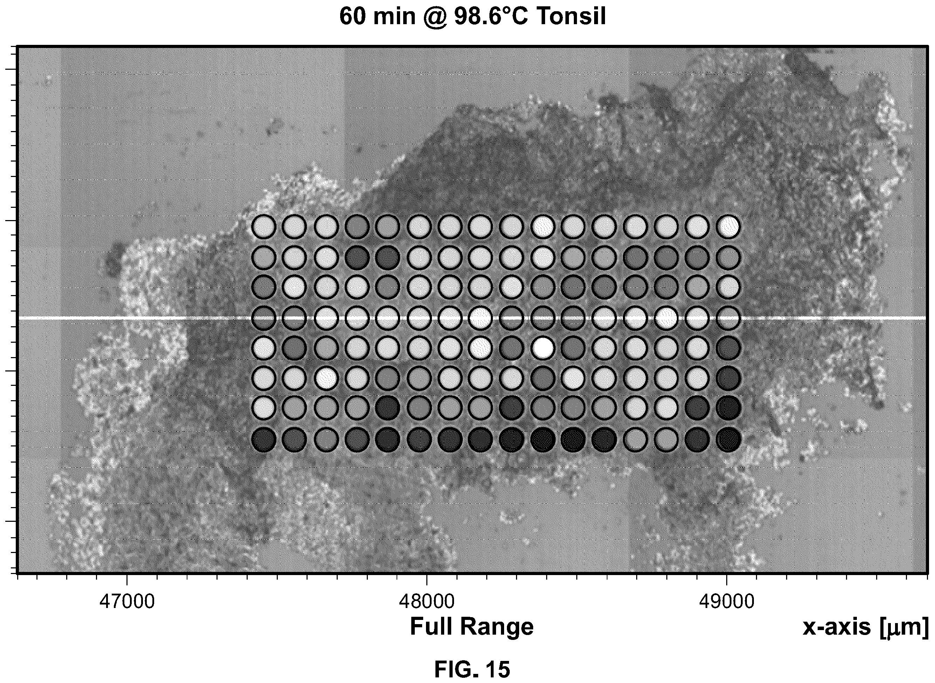

In view of the foregoing, and as described further herein, in some embodiments the predicted unmasking status may include a quantitative estimation of the temperature and/or duration of an unmasking process that the biological specimen was subjected to. In other embodiments, the predicted unmasking status may include a qualitative estimation of a degree or quality of an unmasking process, for example whether the antigen or target was not unmasked, partially unmasked, acceptably unmasked, or fully unmasked. In some embodiments, the qualitative estimation may be binary, for example unmasked or not unmasked. By way of example, and with reference to FIG. 15B, the datapoint at about 98.6°C / about 100 °C may be considered as a data point that is "under retrieved;" while the data point at about 140°C / about 140°C may be considered as a data point that is "over retrieved." Such data points may be used to train an unmasking status estimation engine.

In some embodiments, the unmasking status of a biological specimen is predicted based on an identification of an unmasking features in spectral data acquired from the biological specimen. In some embodiments, the unmasking features within the spectral data acquired from the biological specimen are identified using a trained unmasking status estimation engine; and an unmasking status may be derived based on those identified unmasking features. In some embodiments, the unmasking features identified correspond to conformational changes in the composition of the biological specimen as a result of the unmasking process. For example, unmasking features may be identified which correspond to tissue biostructure and/or the availability of immunorecognition sites within the tissue. It is believed that the availability of immunorecognition sites is tied to the conformational state of a protein or nucleic acid, as that protein or nucleic acid conformational state is altered through the course of an unmasking process.

The present disclosure also describes systems and methods for training an unmasking status estimation engine to enable a quantitative and/or qualitative determination of an unmasking status based on ground truth data, e.g. training spectral data including class labels. As described further herein. In some embodiments, the training spectral data includes differentially unmasked biological specimens.

### Systems

At least some embodiments of the present disclosure relate to computer systems and methods for analyzing spectral data acquired from biological specimens which have been subjected at least partially to an unmasking process, where the unmasking process may be either an antigen retrieval process (a process utilized to make antigenic sites recognizable by a specific binding entity, such as an antibody probe) or a target retrieval process (a process utilized to make nucleic acids recognizable by a specific binding entity, such as a nucleic acid probe). In accordance with the present disclosure, a trained unmasking status estimation engine may be used to provide a quantitative and/or qualitative estimate of an unmasking status of a test biological specimen, e.g. the systems of the present disclosure may receive as input test spectral data from a test biological specimen and may then provide as an output an unmasking status, e.g. a quantitative estimate of an unmasking temperature, a quantitative estimate of an unmasking duration, and/or a qualitative estimate of an unmasking of a degree or quality of unmasking.

In other embodiments, the output provided by the system and method of the present disclosure may be used to assess whether a particular test biological specimen has been sufficiently unmasked prior to the application of one or more specific binding entities or prior to conducting any further downstream processing steps. It is believed that different biomarkers may show different responses to increasing unmasking treatments. For example, and as described in the examples and figures herein, Ki-67 increases in stain intensity and number of labeled cells to a point after which intensity and positivity decrease. Conversely, C4d continues to increase in intensity and positivity through the duration of an applied unmasking process, even under unmasking conditions which would otherwise damage the biological specimen. Thus, in some embodiments, the presently disclosed systems enable the determination of whether an unmasking process applied to a biological specimen provides for a specimen that was sufficiently unmasked prior to a downstream operation, e.g. prior to staining in an IHC or ISH process. Indeed, and in the context of staining with Ki-67 as noted above, the skilled artisan will be able to interpret the results of actual Ki-67 staining in view of the predicted unmasking status of the biological specimen based on an actual unmasking state of the specimen (regardless of the unmasking conditions (time and duration) reported by a lab).

In some embodiments, the output, for example the predicted unmasking status, may be used, for example, to verify a reported unmasking status of a sample. For example, if a test biological specimen is received in a lab along with a report indicating that the test biological specimen was subjected to an unmasking process at about 120°C for about 30 minutes, the output from the system and method of the present disclosure may be used to validate the reported unmasking status.

In some embodiments, the output may be in the form of a generated report. In other embodiments, the output may be an overlay superimposed over an image of a test biological specimen. In yet other embodiments, any output may be stored in a memory coupled to the system (e.g. storage system 240) and that output may be associated with the test biological specimen and/or other patient data.

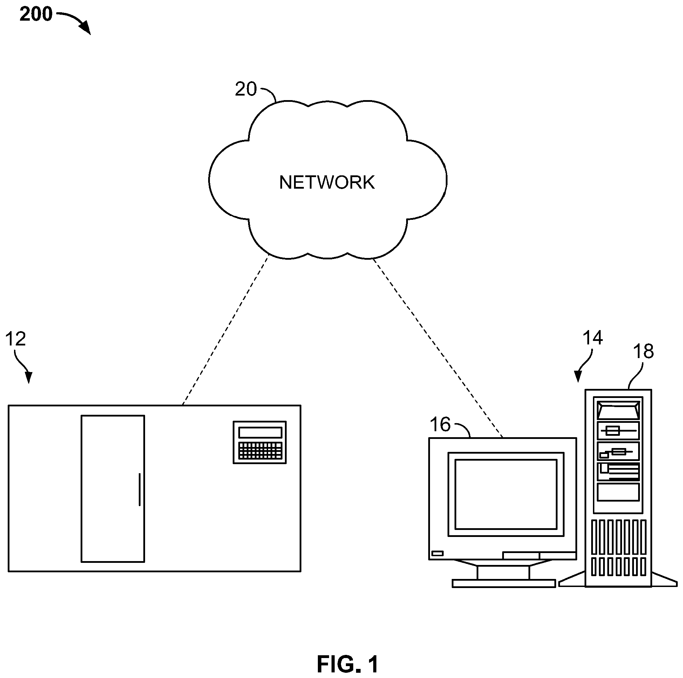

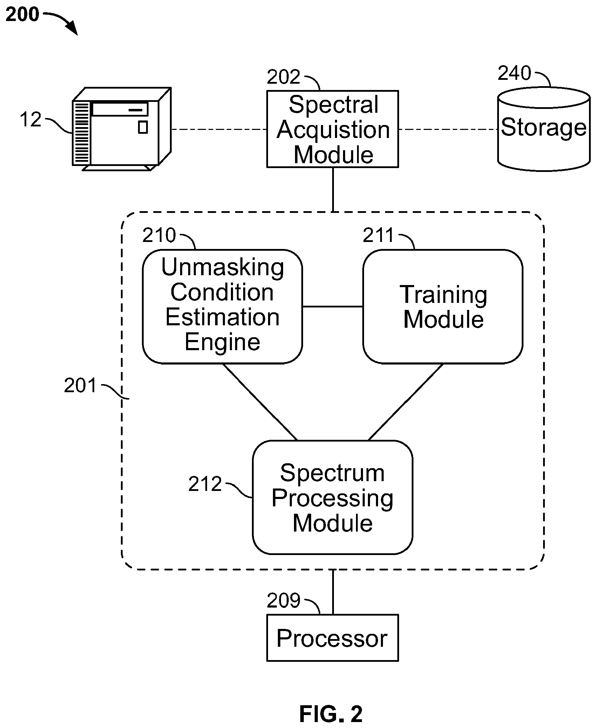

A system 200 for acquiring spectra data, e.g. vibrational spectral data, and analyzing biological specimens (including test biological specimens and training biological specimens) is illustrated in FIGS. 1 and 2. The system may include a spectral acquisition device 12, such as one configured to acquire a vibrational spectrum (e.g. a mid-IR spectrum or a RAMAN spectrum) of a biological specimen (or any portion thereof), and a computer 14, whereby the spectral acquisition device 12 and computer may be communicatively coupled together (e.g. directly, or indirectly over a network 20). The computer system 14 can include a desktop computer, a laptop computer, a tablet, or the like, digital electronic circuitry, firmware, hardware, memory 201, a computer storage medium (240), a computer program or set of instructions (e.g. where the program is stored within the memory or storage medium), one or more processors (209) (including a programmed processor), and any other hardware, software, or firmware modules or combinations thereof (such as described further herein). For example, the system 14 illustrated in FIG. 1 may comprise a computer with a display device 16 and an enclosure 18. The computer system can store acquired spectral data locally, such as in a memory, on a server, or another network connected device.

Vibrational spectroscopy is concerned with the transitions due to absorption or emission of electromagnetic radiation. These transitions are believed to appear in the range of 102 to 104 cm-1 and originate from the vibration of nuclei constituting the molecules in any given sample. It is believed that a chemical bond in a molecule can vibrate in many ways, and each vibration is called vibrational mode. There are two types of molecular vibrations, stretching and bending. A stretching vibration is characterized by movement along the bond axis with increasing or decreasing of the interatomic distances, whereas a bending vibration consists of a change in bond angles with respect to the remainder of the molecule. The two widely used spectroscopic techniques based on vibrational energy are the Raman spectroscopy and the infrared spectroscopy. Both methods give complementary information and are based on the fact that within any molecules the atoms vibrate with a few definite sharply defined frequency characteristics of that molecule. When a sample is eradiated to a beam of incident radiation, it absorbs energy at frequencies characteristic to that of the frequency of the vibration of chemical bonds present in the molecules. This absorption of energy through the vibration of chemical bond results in an infrared spectrum.

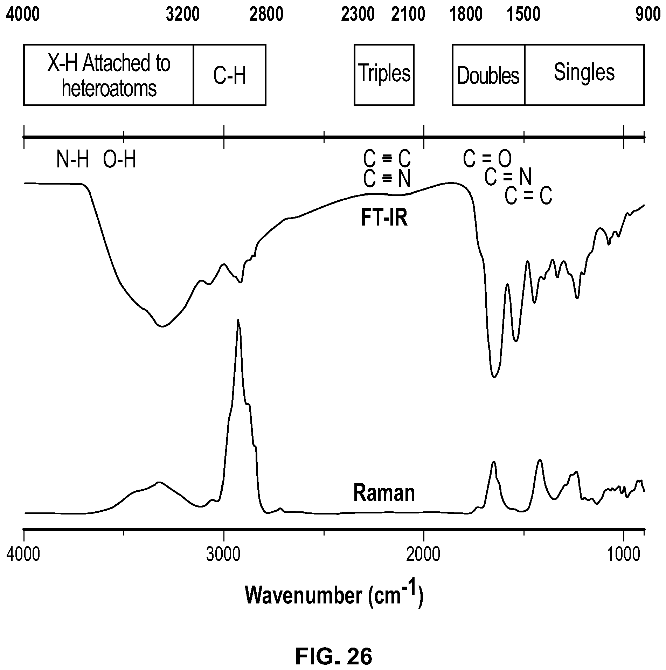

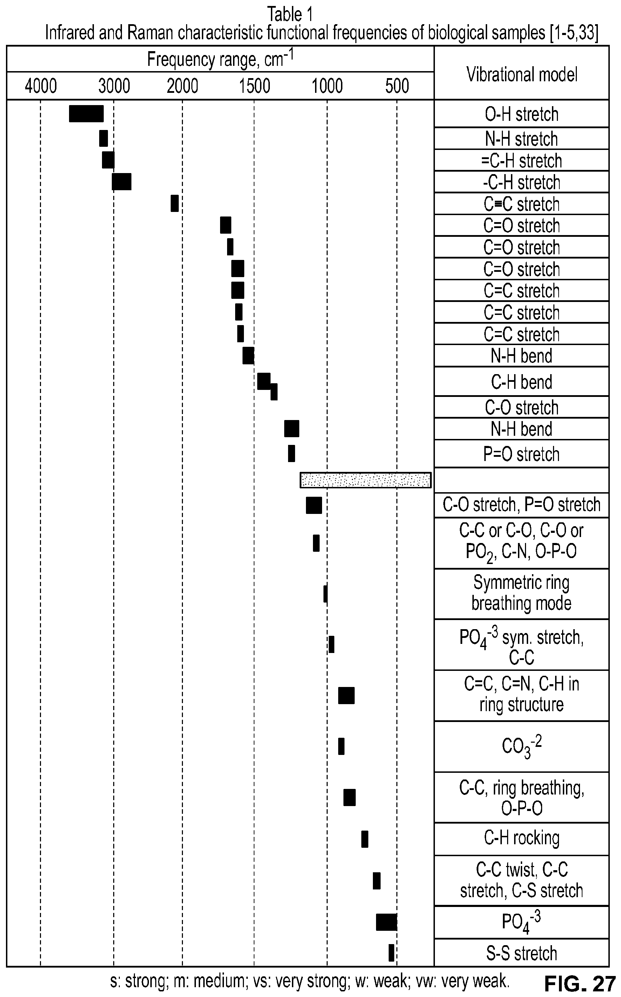

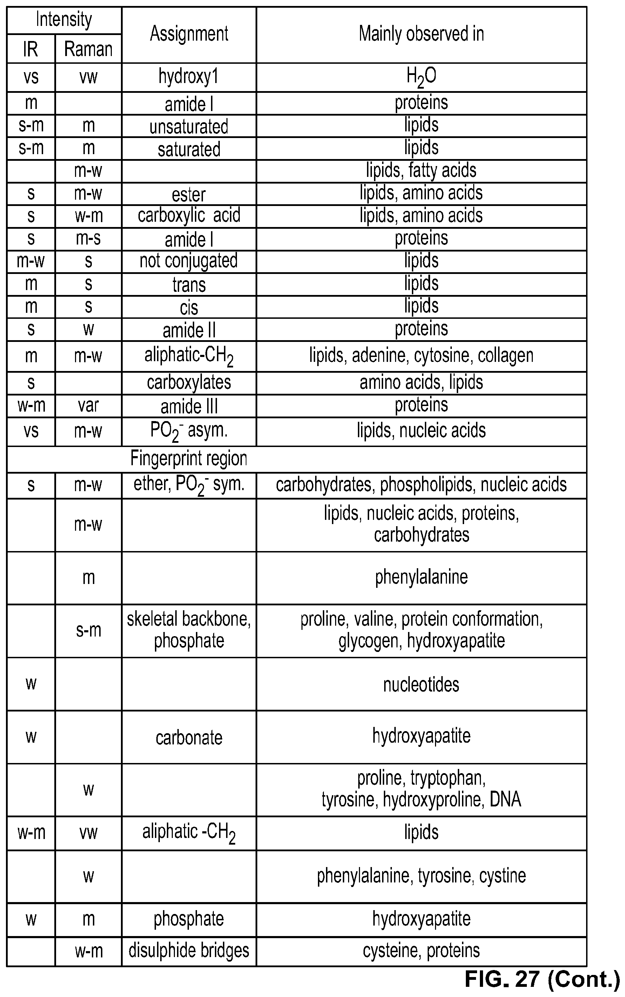

Although IR and Raman spectroscopies measure the vibrational energies of molecules, both methods are dependent on different selection rules, for example, an absorption process and a scattering effect. Although their contrast mechanisms are different and each methodology has respective strengths and weaknesses, the resultant spectra from each modality are often correlated (see, e.g. FIG. 26 and 27).

IR spectroscopy is based on the absorption of electromagnetic radiation, whereas Raman spectroscopy relies upon inelastic scattering of electromagnetic radiation. Infrared spectroscopy offers a number of analytical tools, from absorption to reflection and dispersion techniques, extended in a large range of wave numbers and including the near, middle, and far infrared regions in which the different bonds present in the sample molecules offer numerous generic and characteristic bands suitable to be employed for both qualitative and quantitative purposes. The sample is radiated by IR light in IR spectroscopy, and the vibrations induced by electrical dipole moment are detected.

Raman spectroscopy is a scattering phenomenon and arises due to the difference between the incident and scattered radiation frequencies. It utilizes scattered light to gain knowledge about molecular vibration, which can provide information regarding the structure, symmetry, electronic environment, and bonding of the molecule. In Raman spectroscopy, the sample is illuminated by a monochromatic visible or near IR light from a laser source and its vibrations during the electrical polarizability changes are determined.

Any spectral acquisition device may be utilized in the systems of the present disclosure. Examples of suitable spectral acquisition devices or components of such devices for use in acquiring mid-infrared spectra are described in US Patent Publication Nos.: 2018/0109078a and 2016/0091704; and in US Patent Nos.: 10,041,832, 8,036,252, 9,046,650, 6,972,409, and 7,280,576.

Any method suitable for generating a representative mid-IR spectrum for the samples may be used. Fourier-transform Infrared Spectroscopy and its biomedical applications are discussed in, for example, in P. Lasch, J. Kneipp (Eds.) Biomedical Vibrational Spectroscopy" 2008 (John Wiley&Sons). More recently, however, tunable quantum cascade lasers have enabled the rapid spectroscopy and microscopy of biomedical specimen (see N. Kröger et al., in: Biomedical Vibrational Spectroscopy VI: Advances in Research and Industry, edited by A. Mahadevan-Jansen, W. Petrich, Proc. of SPIE Vol. 8939, 89390Z; N. Kröger et al., J. Biomed. Opt. 19 (2014) 111607; N. Kröger-Lui et al., Analyst 140 (2015) 2086) by virtue of their high spectral power density. It is believed that this work constitutes a major breakthrough (as compared to foregoing Infrared microscopy setups) towards applicability in that the investigation is much faster (e.g. 5 minutes instead of 18 hours), does not need liquid nitrogen cooling and provides more many more pixels per image at substantially lower cost. One particular advantage of QCL-based microscopy in the context of the quality assessment of unstained tissue is the larger field of view (as compared to FT-IR imaging) which is enabled by the microbolometer array detector with e.g. 640 x 480 pixels.

In some embodiments, spectra may be obtained over broad wavelength ranges, one or more narrow wavelength ranges, or even at merely a single wavelength, or a combination thereof. For example, spectra may be acquired for an Amide I band and Amide II band. By way of another example, the spectra may be acquired over a wavelength ranging from about 3200 to about 3400 cm-1, about 2800 to about 2900 cm-1, about 1020 to about 1100 cm-1, and/or about 1520 to about 1580 cm-1. In some embodiments, the spectra may be acquired over a wavelength ranging from about 3200 to about 3400 cm-1. In some embodiments, the spectra may be acquired over a wavelength ranging from about 2800 to about 2900 cm-1.In some embodiments, the spectra may be acquired over a wavelength ranging from about 1020 to about 1100 cm-1. In some embodiments, the spectra may be acquired over a wavelength ranging from about 1520 to about 1580 cm-1. It is believed that narrowing down the spectral range is usually advantageous in terms of the acquisition speed, especially when using quantum cascade lasers. In some embodiments, a single tunable laser is tuned to the respective wavelengths one after the other. Alternatively, a set of non-tunable lasers at fixed frequency could be used such that the wavelength selection is done by switching on and off whichever laser is needed for a measurement at a particular frequency.

The spectra may be acquired using, for example, transmission or reflection measurements. For transmission measurements, barium fluorite, calcium fluoride, silicon, thin polymer films, or zinc selenide are usually used as substrate. For the reflection measurements, gold- or silver-plated substrates are common as well as standard microscope glass slides, or glass slides which are coated with a mid-IR-reflection coating (e.g. multilayer dielectric coating or thin sliver-coating). In addition, means for using surface enhancement (e.g. SEIRS) may be implemented such as structured surfaces like nanoantennas.

The skilled artisan will appreciate that other computer devices or systems may be utilized and that the computer systems described herein may be communicatively coupled to additional components, e.g. microscopes, imaging devices, scanner, other imaging systems, automated slide preparation equipment, etc. Some of these additional components and the various computers, networks, etc. that may be utilized are described further herein.

For example, in some embodiments the system 200 may further include an imaging device and any images captured from the imaging device may be stored in binary form, such as locally or on a server. In some embodiments, the images captured may be stored along with the unmasking status estimates and/or any patient data, such as in storage sub-system 240. The captured digital images can also be divided into a matrix of pixels. The pixels can include a digital value of one or more bits, defined by the bit depth. In general, the imaging apparatus (or other image source including pre-scanned images stored in a memory) can include, without limitation, one or more image capture devices. Image capture devices can include, without limitation, a camera (e.g., an analog camera, a digital camera, etc.), optics (e.g., one or more lenses, sensor focus lens groups, microscope objectives, etc.), imaging sensors (e.g., a charge-coupled device (CCD), a complimentary metal-oxide semiconductor (CMOS) image sensor, or the like), photographic film, or the like. In digital embodiments, the image capture device can include a plurality of lenses that cooperate to prove on-the-fly focusing. An image sensor, for example, a CCD sensor can capture a digital image of the specimen.

In some embodiments, the imaging device is a brightfield imaging system, a multispectral imaging (MSI) system or a fluorescent microscopy system. The digitized tissue data may be generated, for example, by an image scanning system, such as a VENTANA DP200 scanner by VENTANA MEDICAL SYSTEMS, Inc. (Tucson, Arizona) or other suitable imaging equipment. Additional imaging devices and systems are described further herein. The skilled artisan will appreciate that the digital color image acquired by the imaging apparatus is conventionally composed of elementary color pixels. Each colored pixel can be coded over three digital components, each comprising the same number of bits, each component corresponding to a primary color, generally red, green or blue, also denoted by the term "RGB" components.

FIG. 2 provides an overview of the system 200 of the present disclosure and the various modules utilized within the system. In some embodiments, the system 200 employs a computer device or computer-implemented method having one or more processors 209 and one or more memories 201, the one or more memories 201 storing non-transitory computer-readable instructions for execution by the one or more processors to cause the one or more processors to execute certain instructions as described herein.

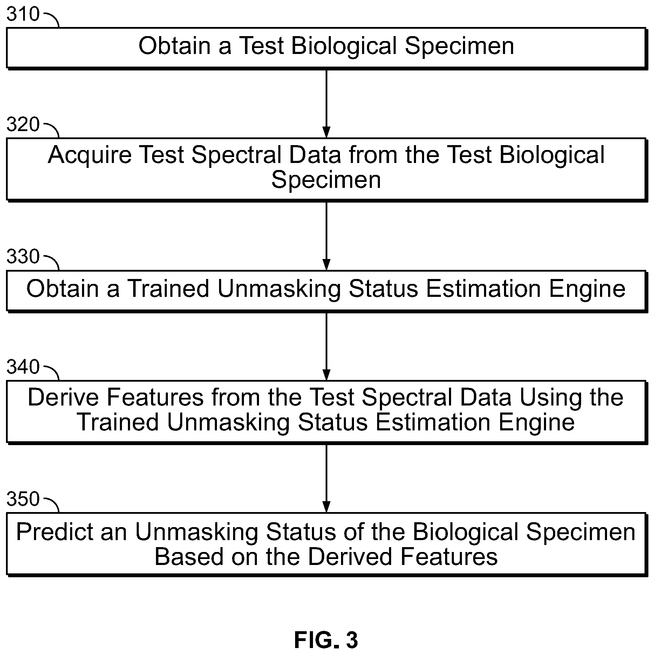

In some embodiments, and as noted above, the system includes a spectral acquisition module 202 for acquiring vibrational spectra, such as mid-IR spectra or RAMAN spectra, of an obtained biological specimen (see, e.g., step 310 of FIG. 3) or any portion thereof (see, e.g., step 320 of FIG. 3). In some embodiments, the system 200 further includes a spectrum processing module 212 adapted to process acquired spectral data. In some embodiments, the spectrum processing module 212 is configured to pre-process spectral data. In some embodiments, the spectrum processing module 212 corrects and/or normalizes the acquired spectra, or to convert acquired transmission spectra to absorption spectra. In other embodiments, the spectrum processing module 212 is configured to average a plurality of acquired spectra from a single biological specimen. In yet other embodiments, the spectrum processing module 212 is configured to further process any acquired spectrum, such as to compute a first derivative, a second derivative, etc. of an acquired spectrum.

In some embodiments, the system 200 further includes a training module 211 adapted to receive training spectral data and to use the received training spectral data to train an unmasking status estimation engine 210.

In some embodiments, the system 200 includes an unmasking status estimation engine 210 which is trained to detect unmasking features within test spectral data (see, e.g., step 340 of FIG. 3) and provide an estimate of an unmasking status of a biological specimen based on the detected unmasking features (see, e.g., step 350 of FIG. 3). In some embodiments, the unmasking status estimation engine 210 includes one or more machine-learning algorithms. In some embodiments, one or more machine-learning algorithms is based on dimensionality reduction as described further herein. In some embodiments, the dimensionality reduction utilized principal component analysis, such as principal component analysis with discriminate analysis. In other embodiments, the dimensionality reduction is a projection onto latent structure regression. In some embodiments, the unmasking status estimation engine 210 includes a neural network. In other embodiments, the unmasking status estimation engine 210 includes a classifier, such as a support vector machine.

The skilled artisan will also appreciate that additional modules may be incorporated into the workflow or into system 200. In some embodiments, an image acquisition module be run to acquire digital images of a biological specimen or any portion thereof. In other embodiments, an automated image analysis algorithm may be run such that cells may be detected, classified and/or scored (see, e.g., U.S. Patent Publication No. 2017/0372117).

### SPECTRAL ACQUISITION MODULE AND ACQUIRED SPECTRAL DATA

With reference to FIG. 2, in some embodiments, the system 200 runs a spectral acquisition module 202 to acquire vibrational spectra (e.g. using an spectra imaging apparatus 12, such as any of those described above) from at least a portion of a biological specimen (e.g. a test biological specimen or a training biological specimen). In some embodiments, the biological specimen is unstained, for example it does not include any stains indicative of the presence of a biomarker. In other embodiments, the biological specimen may also include one or more stains, e.g. primary stains, or stains indicative of the presence of one or more biomarkers (e.g. proteins, nucleic acids). Once the spectra are acquired using the spectral acquisition module 202, the acquired spectra may be stored in a storage module 240 (e.g. a local storage module or a networked storage module).

In some embodiments, the vibrational spectra may be acquired from a portion of the biological specimen (and this is regardless of whether the specimen is a training biological specimen or a test biological specimen, as described further herein). In such a case, the spectral acquisition module 202 may be programmed to acquire the vibrational spectra from a predefined portion of the sample, for example by random sampling or by sampling at regular intervals across a grid covering the entire sample. This can also be useful where only specific regions of the sample are relevant for analysis. For example, a region of interest may include a certain type of tissue or a comparatively higher population of a certain type of cell as compared with another region of interest. For example, a region of interest may be selected that includes tonsil tissue but excludes connective tissue. In such a case, the spectral acquisition module 202 may be programmed to collect the vibrational spectra from a predefined portion of a region of interest, for example by random sampling of the region of interest or by sampling at regular intervals across a grid covering the entire region of interest. In embodiments where the sample includes one or more stains, vibrational spectra may be obtained from those regions of interest that do not include any stain or include comparatively less stain than other regions.

In some embodiments, at least two regions of the biological specimen are sampled, and vibrational spectra are acquired for each of the at least two regions (and again, this is regardless of whether the specimen is a training biological specimen or a test biological specimen). In other embodiments, at least 10 regions of the biological specimen are sampled, and vibrational spectra are acquired for each of the at least 10 regions. In yet other embodiments, at least 30 regions of the biological specimen are sampled, and vibrational spectra are acquired for each of the at least 30 regions. In further embodiments, at least 60 regions of the biological specimen are sampled, and vibrational spectra are acquired for each of the at least 60 regions. In yet further embodiments, at least 90 regions of the biological specimen are sampled, and vibrational spectra are acquired for each of the at least 90 regions. In even further embodiments, between about 30 regions and about 150 regions of the biological specimen are sampled, and vibrational spectra are acquired for each of the regions.

In some embodiments, a single vibrational spectrum is acquired per region of the biological specimen. In other embodiments, at least two vibrational spectra are acquired per region of the biological specimen. In yet other embodiments, at least three vibrational spectra are acquired per region of the biological specimen.

In some embodiments, the acquired spectra or acquired spectral data (used interchangeably herein) which are stored in storage module 240 include "training spectral data." In some embodiments, the training spectral data is derived from training biological specimens, where the training biological specimens may be histological specimens, cytological specimens, or any combination thereof. In some embodiments, the training spectral data are used to train an unmasking status estimation engine 210, such as through use of the training module 211 as described herein. In some embodiments, the training spectral data includes class labels, such as unmasking time, unmasking duration, relative quality information (e.g. un-retrieved, fully retrieved, partially retrieved), etc. In some embodiments, the training spectral data includes a plurality of class labels.

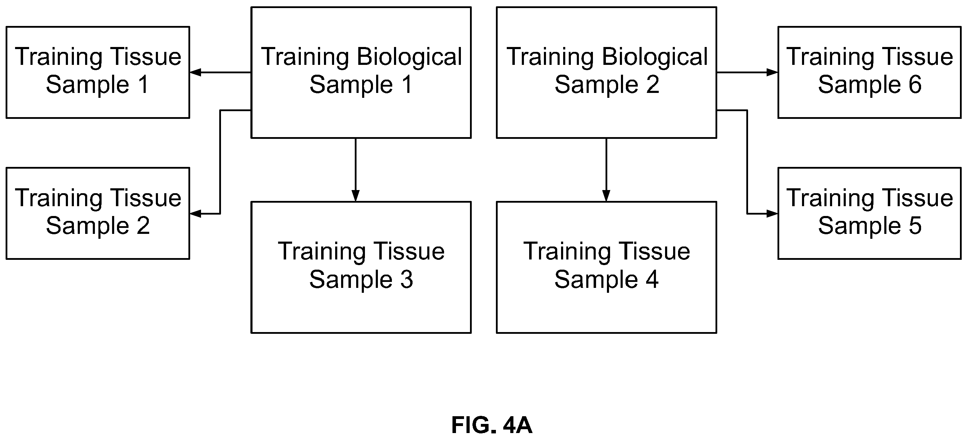

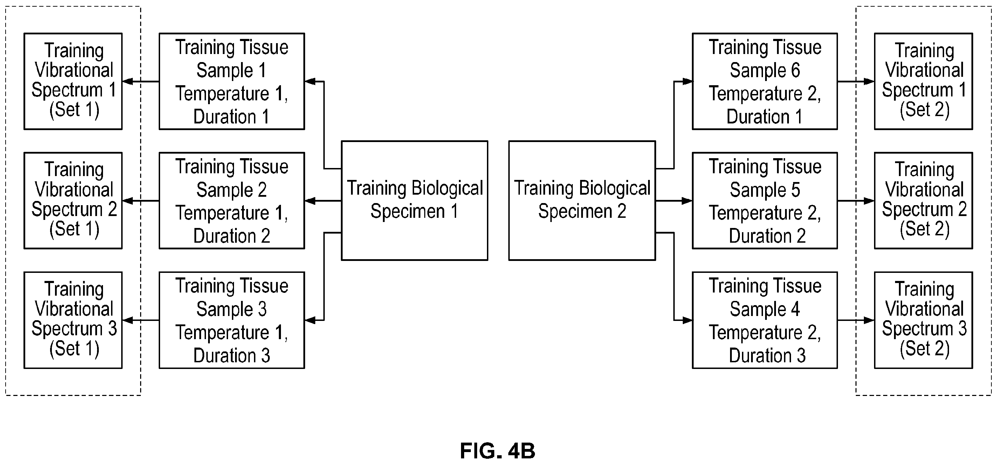

In some embodiments, the training biological specimens are differentially unmasked. Differential unmasking is a process where a single training biological specimen is divided into a plurality of parts (e.g. a first training tissue sample, a training second tissue sample, and nth training tissue sample) (see, e.g., FIG. 4A), and each part of the plurality of parts is subjected to a different unmasking process (see, e.g., FIG. 4B). For example, a single tonsil tissue sample may be divided into 10 or more parts, and each part may be unmasked for a pre-determined amount of time and at a pre-determined temperature. In some embodiments, a sample may sectioned into 3 or more parts, and each part may be unmasked under a different set of unmasking conditions. In other embodiments, a sample may sectioned into 5 or more parts, and each part may be unmasked under a different set of unmasking conditions. In yet other embodiments, a sample may sectioned into 7 or more parts, and each part may be unmasked under a different set of unmasking conditions. In further embodiments, a sample may sectioned into 9 or more parts, and each part may be unmasked under a different set of unmasking conditions. This process is illustrated in FIG. 4A.

In some embodiments, a plurality of training samples derived from a single training biological specimen are each unmasked at the same temperature, but for different durations. For example, each training sample derived from a single training biological specimen could be unmasked at the same temperature (e.g. about 98.6°C) but where the duration of unmasking could vary (about 5 minutes, about 30 minutes, about 60 minutes, etc.).

In other embodiments, a plurality of training samples derived from a single biological specimen are each unmasked for the same duration, but at different temperatures. For example, each training sample could be unmasked for the same duration (e.g. 10 minutes) but where the temperature of the unmasking is varied (about 98.6°C, about 110°C, about 120°C, about 130°C, etc.).

Of course, the skilled artisan will recognize that unmasking time and temperature could both be varied. As in the embodiments described above, a first set of training samples could be unmasked at a first temperature but for different durations, providing a first set of training samples. A second set and a third set of training samples can be unmasked at a second temperature and a third temperature, respectively, and again for different durations, providing second and third sets of training samples. This process is illustrated in FIG. 4B. This process may be repeated for more than one biological specimen, such as biological specimens having the same or different tissue types so as to build a database (such as a database stored within storage module 240) comprising a plurality of training spectral data sets which may be used in training an unmasking status estimation engine. The skilled artisan will also appreciate that temperature and duration are not the only unmasking conditions that may be varied. Indeed, different unmasking reagents, pressures, etc. may be utilized in any unmasking process, and such conditions may be varied to provide training spectral data sets that take into consideration such process conditions.

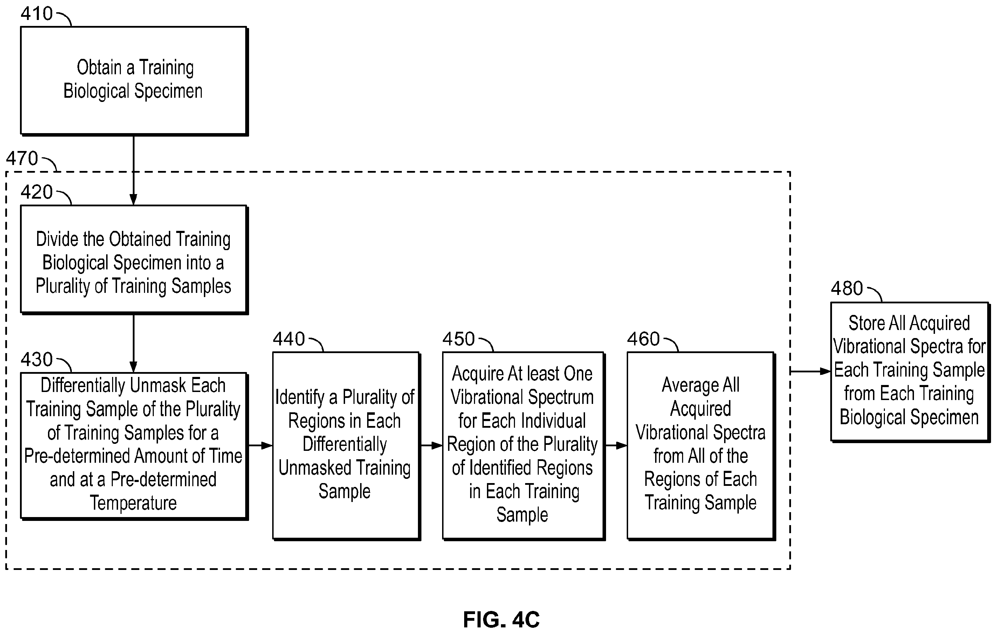

The process of differential unmasking and of acquiring spectral data from the differentially unmasked samples is further illustrated in FIG. 4C. As noted above, one or more training biological specimens are first acquired (step 410). Each of the one or more training biological specimens are then divided into at least two parts (step 420). In this way, each of the one or more training biological specimens provide at least two "training samples." Each of these training samples may be differentially unmasked, for example each may be unmasked for a different pre-determined amount of time or for a different pre-determined duration (step 430). Following the differential unmasking of the at least two training samples, a plurality or regions in each of the at least two training samples are identified (step 440).

Next, at least one vibrational spectrum is acquired for each of the identified regions of the plurality of identified regions (step 450). The average of each acquired vibrational spectrum from each identified region (or a further processed variant thereof as described further below) is computed to provide an averaged vibrational spectrum for that training sample (step 460). Steps 400 through 460 may be repeated for a plurality of different training biological specimens (see dotted line 470). In some embodiments, the averaged vibrational spectra from all training samples from all training biological specimens (referred to as "training spectral data") are stored (step480), such as in storage module 240. In this way, the training spectral data may be retrieved from the storage module 240 by the training module 211 for training of an unmasking status estimation engine 210. In addition to storing the average vibrational spectra from all training samples, the storage module 240 is also adapted to store any class labels associated with the averaged vibrational spectra (e.g. unmasking temperature, unmasking duration, etc.).

In some embodiments, each of the training samples is stained for the presence of one or more biomarkers such that functional staining intensity may be evaluated for each training sample. In some embodiments, each training sample is stained for the presence of a single biomarker and then images of the samples are captured using an imaging device and analyzed (such as for staining intensity and/or percent positivity). In other embodiments, each training sample is stained for the presence of two or more biomarkers and then images of the samples are captured using an imaging device and analyzed (such that the staining intensity and/or percent positivity of each of the two or more biomarkers are independently analyzed). In some embodiments, multiple biomarkers are tested for functional staining information since each biomarker may have a different response to an unmasking treatment. For example, Ki-67 and C4d biomarkers may behave differently when introduced to samples which were unmasked under different conditions. For example, it is believed that Ki-67 increases in stain intensity as unmasking extent increases, but only up to a certain point, after which intensity and positivity decrease. Conversely, C4d continues to increase in intensity and positivity even through unmasking conditions which would otherwise damage the sample.

The processes described above for preparing training biological specimens and acquiring spectra data from such specimens may be repeated for a plurality of different training biological specimens, where each of the plurality of different training biological specimens may be of the same tissue type or may of a different tissue type (e.g. tonsil tissue or breast tissue). The Example section herein further describes the methods of preparing training biological specimens and the acquisition of spectral data for use in training an unmasking status estimation engine 210, such as for predicting an unmasking status of test biological specimens derived from both tonsil tissue and breast tissue. Moreover, the processes described above may be repeated for different unmasking reagents, or for antigen retrieval processes and target retrieval processes (and the Example provided herein illustrates that the systems and methods of the present disclosure are equally applicable to antigen retrieval processes and target retrieval processes, with accurate results being provided in both use cases).

In some embodiments, the acquired spectral data stored in the storage module 240 include "test spectral data." In some embodiments, the test spectral data is derived from test biological specimens, such as specimens derived from a subject (e.g. a human patient), where the test biological specimens may be histological specimens, cytological specimens, or any combination thereof.

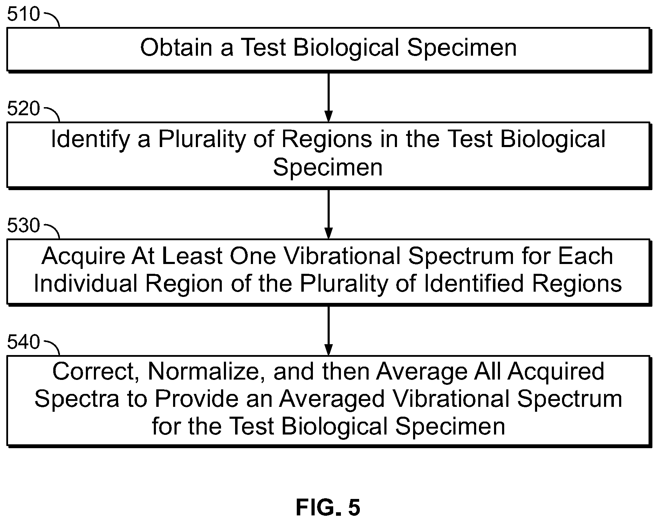

With reference to FIG. 5, a test biological specimen may be obtained (step 510), and then a plurality of spatial regions within the test biological specimen may be identified (step 520). At least one vibrational spectrum may be acquired for each identified region (step 530). The acquired vibrational spectra from all of the regions may then be corrected, normalized, and averaged to provide an averaged vibrational spectrum for the test biological specimen ("test spectral data"). As described further herein, the test spectral data may be supplied to a trained unmasking status estimation engine 210 such that an unmasking status of the test biological specimen may be predicated. The predicated unmasking status may then be used in downstream processes or downstream decision making, e.g. to determine whether the test specimen was adequately unmasked before the deposition of one or more specific binding entities.

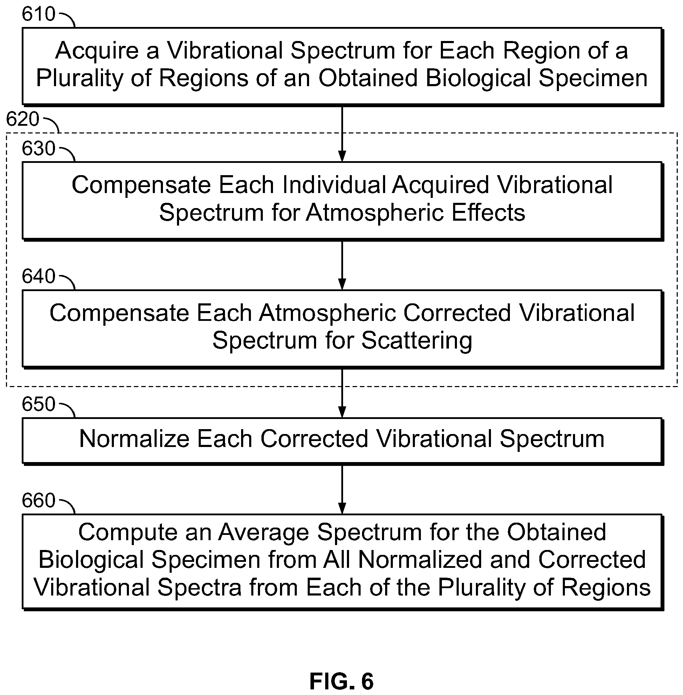

As noted above, and regardless of whether the spectral data is acquired from a training or test biological specimen, a plurality of vibrational spectra are acquired for each biological specimen, e.g. to account for spatial the spatial heterogeneity of the sample. In some embodiments, the spectral processing module 212 is first utilized to covert each acquired vibrational transmission spectrum to a vibrational absorption spectrum. In some embodiments, transmission spectra and absorbance spectra are directly related via the equation Absorbance = ln(blank transmission / transmission through the tissue) and thus acquired transmission spectra may be converted to absorption spectra. Once all of the vibrational spectra are converted from transmission to absorbance, in some embodiments, the spectral processing module 212 averages all of the acquired spectra from all of the various regions, and it is the averaged vibrational spectrum that is used for downstream analysis, for example for training or predicting an unmasking status. In some embodiments, and with reference to FIG. 6, the vibrational spectra acquired from each of the plurality of spatial regions are first normalized and/or corrected prior to their averaging. In some embodiments, vibrational spectrum from each region is individually corrected (step 620) to provide a corrected vibrational spectrum. For example, the correction may include compensating each acquired vibrational spectrum for atmospheric effects (step 630) and then compensating each atmospheric corrected vibrational spectrum for scattering (step 640). Next, each corrected vibrational spectrum is normalized, e.g. to a maximum value of 2 to mitigate differences in specimen thickness and tissue density (step 650). Subsequently, the collective of the amplitude normalized spectra are averaged (step 660).

### UNMASKING STATUS ESTIMATION ENGINE

The systems and methods of the present disclosure employ machine learning techniques to mine spectra data. In the case of an unmasking status estimation engine in a training mode, the unmasking status estimation engine may learn features from a plurality of acquired and processed training spectra (such as training spectra stored within storage module 240) and correlate those learned features with class labels associated with the training spectra (e.g. known unmasking temperatures, known unmasking duration, tissue quality, etc.). In the case of a trained unmasking status estimation engine, the trained unmasking status engine may derive unmasking features from a test biological specimen and, based on the learned datasets, predict an unmasking status of the test biological specimen based on the derived unmasking features.

Machine learning can be generally defined as a type of artificial intelligence (AI) that provides computers with the ability to learn without being explicitly programmed. Machine learning focuses on the development of computer programs that can teach themselves to grow and change when exposed to new data. In other words, machine learning can be defined as the subfield of computer science that gives computers the ability to learn without being explicitly programmed. Machine learning explores the study and construction of algorithms that can learn from and make predictions on data-such algorithms overcome following strictly static program instructions by making data driven predictions or decisions, through building a model from sample inputs. The machine learning described herein may be further performed as described in "Introduction to Statistical Machine Learning," by Sugiyama, Morgan Kaufmann, 2016, 534 pages; "Discriminative, Generative, and Imitative Learning," Jebara, MIT Thesis, 2002, 212 pages; and "Principles of Data Mining (Adaptive Computation and Machine Learning)," Hand et al., MIT Press, 2001, 578 pages. The embodiments described herein may be further configured as described in these references.

In some embodiments, the unmasking status estimation engine 210 employs "supervised learning" for the task of predicting an unmasking status of a test spectrum derived from a test biological specimen. Supervised learning is the machine learning task of learning a function that maps an input to an output based on example input-output pairs. It infers a function from labeled training data (here, the unmasking status is the label associated with training spectral data) consisting of a set of training examples (here training spectra). In supervised learning, each example is a pair consisting of an input object (typically a vector) and a desired output value (also called the supervisory signal). A supervised learning algorithm analyzes the training data and produces an inferred function, which can be used for mapping new examples. An optimal scenario allows for the algorithm to correctly determine the class labels for unseen instances.

The unmasking status estimation engine 210 may include any type of machine learning algorithm known to those of ordinary skill in the art. Suitable machine learning algorithms include regression algorithms, similarity-based algorithms, feature selection algorithms, regularization method-based algorithms, decision tree algorithms, Bayesian models, kernel-based algorithms (e.g. support vector machines), clustering-based methods, artificial neural networks, deep learning networks, ensemble methods, and dimensionality reduction methods. Examples of suitable dimensionality reduction methods include principal component analysis (such as principal component analysis plus discriminant analysis) and projection onto latent structure regression.

In some embodiments, the unmasking status estimation engine 210 utilizes principal component analysis. The main idea of principal component analysis (PCA) is to reduce the dimensionality of a data set consisting of many variables correlated with each other while retaining the variation present in the dataset, up to the maximum extent. The same is done by transforming the variables to a new set of variables, which are known as the principal components (or simply, the PCs) and are orthogonally ordered such that the retention of variation present in the original variables decreases as they move down in the order. In this way, the first principal component retains maximum variation that was present in the original components. The principal components are the eigenvectors of a covariance matrix, and hence they are orthogonal. Principal component analysis and methods of employing the same are described in U.S. Patent Publication No. 2005/0123202 and in U.S. Patent Nos. 6,894,639 and 8,565,488. PCA and Linear Discriminant Analysis are further described by Khan et. al., "Principal Component Analysis-Linear Discriminant Analysis Feature Extractor for Pattern Recognition," "IJCSI International Journal of Computer Sciences Issues, Vol. 8, Issue 6, No. 2, Nov. 2011.

In some embodiments, the unmasking status estimation engine 210 utilizes projection onto latent structure regression (PLSR). PLSR is a recent technique that combines features from and generalizes PCA and multiple linear regression. Its goal is to predict a set of dependent variables from a set of independent variables or predictors. This prediction is achieved by extracting from the predictors a set of orthogonal factors called latent variables which have the best predictive power. These latent variables can be used to create displays akin to PCA displays. The quality of the prediction obtained from a PLS regression model is evaluated with cross-validation techniques such as the bootstrap and jackknife. There are two main variants of PLS regression: The most common one separates the roles of dependent and independent variables; the second one-gives the same roles to dependent and independent variables. PLSR is further described by Abdi, "Partial Least Squares Regression and Projection on Latent Structure Regression (PLS Regression)," WIREs Computational Statistics, John Wiley & Sons, Inc., 2010. The Examples section provided herein describes a trained unmasking status estimation engine based on PLSR and illustrates that the PLSR-based trained unmasking status estimation engine 210 may be used to provide at least quantitative estimates of unmasking temperature and/or unmasking duration.

In some embodiments, the unmasking status estimation engine 210 utilizes reinforcement learning. Reinforcement Learning (RL) refers to a type of Machine Learning method in which the agent receives a delayed reward in the next time step to evaluate its previous action. Said another way, RL is model-free machine learning paradigm concerned with how software agents ought to take actions in an environment so as to maximize some notion of cumulative reward. Typically, a RL setup is composed of two components, an agent and an environment. The environment refers to the object that the agent is acting on, while the agent represents the RL algorithm. The environment starts by sending a state to the agent, which then based on its knowledge to take an action in response to that state. After that, the environment sends a pair of next state and reward back to the agent. The agent will update its knowledge with the reward returned by the environment to evaluate its last action. The loop keeps going on until the environment sends a terminal state, which ends to episode. Reinforcement learning algorithms are further described in U.S. Patent Nos. 10,279,474 and 7,395,252.

In some embodiments, the machine learning algorithm is a Support Vector Machine ("SVM"). In general, an SVM is a classification technique, which is based on statistical learning theory where a nonlinear input data set is converted into a high dimensional linear feature space via kernels for the non-linear case. A support vector machines project a set of training data, E, that represents two different classes into a high-dimensional space by means of a kernel function, K. In this transformed data space, nonlinear data are transformed so that a flat line can be generated (a discriminating hyperplane) to separate the classes so as to maximize the class separation. Testing data are then projected into the high-dimensional space via K, and the test data (such as the features or metrics enumerated below) are classified on the basis of where they fall with respect to the hyperplane. The kernel function K defines the method in which data are projected into the high-dimensional space.

In some embodiments, the unmasking status estimation engine 210 includes a neural network. In some embodiments, the neural network is configured as a deep learning network. Generally speaking, "deep learning" is a branch of machine learning based on a set of algorithms that attempt to model high level abstractions in data. Deep learning is part of a broader family of machine learning methods based on learning representations of data. An observation can be represented in many ways such as a vector of intensity values per pixel, or in a more abstract way as a set of edges, regions of particular shape, etc. Some representations are better than others at simplifying the learning task. One of the promises of deep learning is replacing handcrafted features with efficient algorithms for unsupervised or semi-supervised feature learning and hierarchical feature extraction.

In some embodiments, the neural network is a generative network. A "generative" network can be generally defined as a model that is probabilistic in nature. In other words, a "generative" network is not one that performs forward simulation or rule-based approaches. Instead, the generative network can be learned (in that its parameters can be learned) based on a suitable set of training data (e.g. a plurality of training spectral data sets). In some embodiments, the neural network is configured as a deep generative network. For example, the network may be configured to have a deep learning architecture in that the network may include multiple layers, which perform a number of algorithms or transformations.

In some embodiments, the neural network includes an autoencoder. An autoencoder neural network is an unsupervised learning algorithm that applies backpropagation, setting the target values to be equal to the inputs. The aim of an autoencoder is to learn a representation (encoding) for a set of data, typically for dimensionality reduction, by training the network to ignore signal "noise." Along with the reduction side, a reconstructing side is learnt, where the autoencoder tries to generate from the reduced encoding a representation as close as possible to its original input. Additional information regarding autoencoders can be found at http://ufldl.stanford.edu/tutorial/unsupervised/Autoencoders/,.

In some embodiments, the neural network may be a deep neural network with a set of weights that model the world according to the data that it has been fed to train it. Neural networks typically consist of multiple layers, and the signal path traverses from front to back between the layers. Any neural network may be implemented for this purpose. Suitable neural networks include LeNet, AlexNet, ZFnet, GoogLeNet, VGGNet, VGG16, DenseNet, and the ResNet. In some embodiments, a fully convolutional neural network is utilized, such as described by Long et al., "Fully Convolutional Networks for Semantic Segmentation," Computer Vision and Pattern Recognition (CVPR), 2015 IEEE Conference, June 20015 (INSPEC Accession Number: 15524435).

In some embodiments, the neural network is configured as an AlexNet. For example, the classification network structure can be AlexNet. The term "classification network" is used herein to refer to a CNN, which includes one or more fully connected layers. In general, an AlexNet includes a number of convolutional layers (e.g., 5) followed by a number of fully connected layers (e.g., 3) that are, in combination, configured and trained to classify data.

In other embodiments, the neural network is configured as a GoogleNet. While the GoogleNet architecture may include a relatively high number of layers (especially compared to some other neural networks described herein), some of the layers may be operating in parallel, and groups of layers that function in parallel with each other are generally referred to as inception modules. Other of the layers may operate sequentially. Therefore, a GoogleNet is different from other neural networks described herein in that not all of the layers are arranged in a sequential structure. Examples of neural networks configured as GoogleNets are described in "Going Deeper with Convolutions," by Szegedy et al., CVPR 2015.

In other embodiments, the neural network is configured as a VGG network. For example, the classification network structure can be VGG. VGG networks were created by increasing the number of convolutional layers while fixing other parameters of the architecture. Adding convolutional layers to increase depth is made possible by using substantially small convolutional filters in all of the layers.

In other embodiments, the neural network is configured as a deep residual network. For example, the classification network structure can be a Deep Residual Net or ResNet. Like some other networks described herein, a deep residual network may include convolutional layers followed by fully connected layers, which are, in combination, configured and trained for detection and/or classification. In a deep residual network, the layers are configured to learn residual functions with reference to the layer inputs, instead of learning unreferenced functions. In particular, instead of hoping each few stacked layers directly fit a desired underlying mapping, these layers are explicitly allowed to fit a residual mapping, which is realized by feedforward neural networks with shortcut connections. Shortcut connections are connections that skip one or more layers. A deep residual net may be created by taking a plain neural network structure that includes convolutional layers and inserting shortcut connections which thereby takes the plain neural network and turns it into its residual learning counterpart. Examples of deep residual nets are described in "Deep Residual Learning for Image Recognition" by He et al., NIPS 2015. The neural networks described herein may be further configured as described in this reference.

### TRAINING AN UNMASKING STATUS ESTIMATION ENGINE

In some embodiments, the unmasking status estimation engine 210 is adapted to operate in a training mode. In some embodiments, the training module 211 may operate to provide training spectral data to the unmasking status estimation engine 210 and to operate the unmasking status estimation engine 210 in its training mode in accordance with any suitable training algorithm. In some embodiments, a training module 211 is in communication with the unmasking status estimation engine 210 and is configured to receive training spectral data (or a further processed variants of the training absorbance spectra data, e.g. a first or second derivative of the training spectral data, magnitudes of individual bands within the training spectra data, the integral of bands within the training spectral data, the ratio of two or more band intensities within the training spectral data, the ratios from second and third order derivatives of the training spectral data, etc.) and supply the training spectral data to the unmasking status estimation engine 210. In some embodiments, the training module 211 is also adapted to supply the class labels associated with the training spectral data.

In some embodiments, the training algorithms utilize a known set of training spectral data (such as described herein) and a corresponding set of known output class labels (e.g. unmasking condition, etc.), and are configured to vary internal connections within the unmasking status estimation engine 210 such that processing of input training spectral data provides the desired corresponding class labels.

The unmasking status estimation engine 210 may be trained in accordance with any methods known to those of ordinary skill in the art. For example, any of the training methods disclosed in U.S. Patent Publication Nos. 2018/0268255, 2019/0102675, 2015/0356461, 2016/0132786, 2018/0240010, and 2019/0108344.

In some embodiments, the unmasking status estimation engine 210 is trained using a cross-validation method. Cross-validation is a technique that can be used to aid in model selection and/or parameter tuning when developing a classifier. Cross-validation uses one or more subsets of cases from the set of labeled cases as a test set. For example, in k-fold cross-validation, a set of labeled cases is equally divided into k "folds," for example K-fold cross-validation is a resampling procedure used to evaluate machine learning models. A series of train-then-test cycles is performed, iterating through the k folds such that in each cycle a different fold is used as a test set while the remaining folds are used as the training set. Since each fold is used as the test set at some point, non-randomly selected cases in the set of labeled cases would seemingly bias the cross-validation. For example, in the scenario of 5-fold cross validation (k=5), the data set is split into 5 folds. In the first iteration, the first fold is used to test the model and the rest are used to train the model. In the second iteration, second fold is used as the testing set while the rest serve as the training set. This process is repeated until each fold of the 5 folds have been used as the testing set. Methods of performing k-fold cross validation are further described in US Patent Publication Nos.: 2014/0279734 and 2005/0234753.

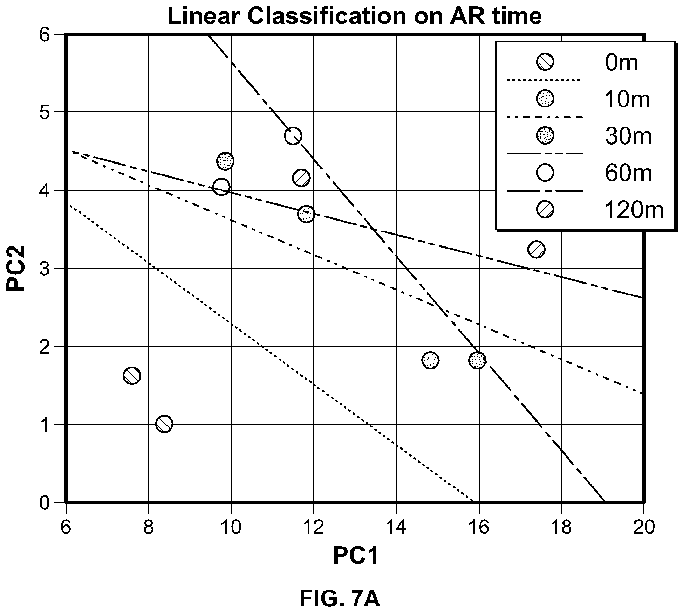

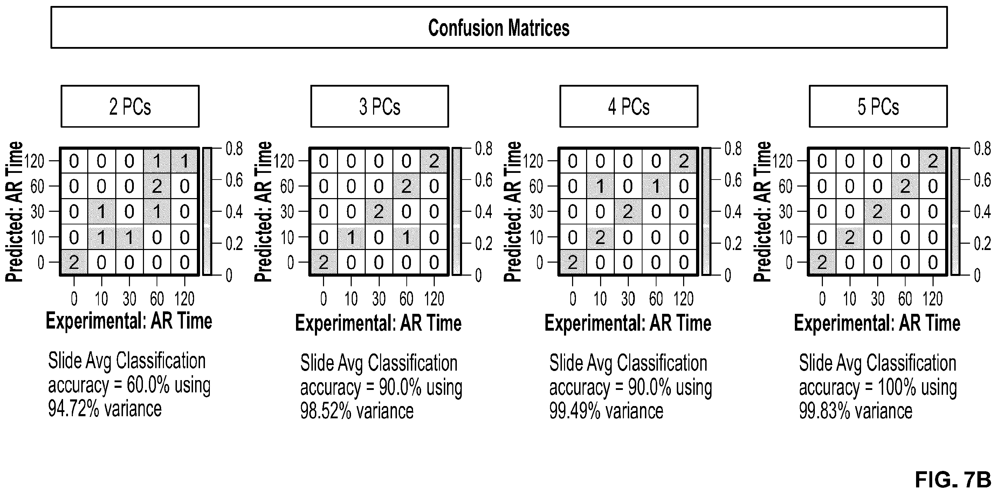

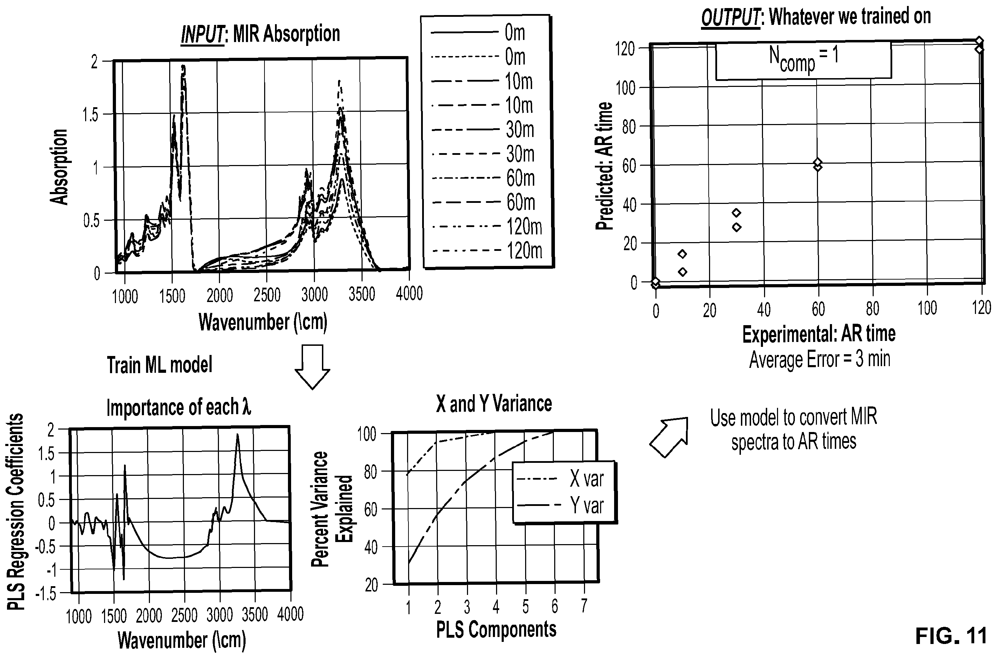

In the context of an unmasking status estimation engine 210 which utilizes a machine learning algorithm based on PLSR, FIGS. 7 and 11 illustrate how the PLSR model is trained to mine the vibrational spectra for features within the training spectra, such as features associated with the conformational states of immunorecognition sites, and how these features change as the unmasking conditions or as the unmasking process proceeds. In some embodiments, the PLSR model is also trained to recognize the changes in these features for different types of tissues and/or for different types of molecules (proteins, nucleic acids). In some embodiments, the PLSR algorithm takes the vibrational spectral data (e.g. absorption spectra, first derivative, second derivative) and creates a model that is used to determine which features (wavelengths) are most predictive of the response variable (unmasking time/duration, etc.). In some embodiments, the generated model may be further evaluated for performance using the same and unknown vibrational spectral data for performance evaluation and optimization.

In the context of an unmasking status estimation engine 210 which utilizes a machine learning algorithm based on principal component analysis, a PCA is performed on an initial training data set of a default sample size to generate a PCA transform matrix. A second PCA is performed on a combined data set which includes the initial training data set and a test data set. The number of samples in initial training data set is then incremented to generate an expanded training data set. A PCA of the expanded training data set is performed to determine if the PCA number for the expanded training data set is the same as for the initial training data set. If so, the error between the initial test data set and the expanded test data set is assessed based on the PCA signals and PCA transform matrix to estimate a final solution error. The PCA matrix of the combined data set is transformed back to the initial training data set domain (e.g., spectral domain) using the transform matrix from the first PCA to generate a test data set estimate. The method iterates with the size of the training matrix expanding until the PCA number converges and a final error target is achieved. Upon reaching the error target, the training data set of the identified size adequately represents the training target function information contained in the specified input parameter range. A machine learning system (e.g. the unmasking status estimation engine 210) may then be trained with the training matrix of the identified size. Additional aspects of training using PCA are disclosed in U.S. Patent Nos. 8,452,718 and 7,734,087.

In embodiments where the unmasking status estimation engine 210 includes a neural network, a back-propagation algorithm may be used for training the unmasking status estimation engine 210. Back propagation is an iterative process in which the connections between network nodes are given some random initial values, and the network is operated to calculate corresponding output vectors for a set of input vectors (the training spectral data set). The output vectors are compared to the desired output of the training spectral data set and the error between the desired and actual output is calculated. The calculated error is propagated back from the output nodes to the input nodes and is used for modifying the values of the network connection weights in order to decrease the error. After each such iteration the training module 211 may calculate a total error for the entire training set and the training module 211 may then repeat the process with another iteration. The training of the unmasking status estimation engine 210 is complete when the total error reaches a minimum value. If a minimum value of the total error is not reached after a predetermined number of iterations and if the total error is not a constant the training module 211 may consider that the training process does not converge.

In the context of training with acquired spectral data derived from training biological samples differentially unmasked (for example unmasked for different durations and/or at different temperatures) as described above, each acquired training spectrum is associated with known unmasking conditions, for example the duration of the unmasking process and the temperature at which the unmasking process was carried out. For example, the two training spectral data sets illustrated in FIG. 4B (see dotted line boxes) may be provided to the training module 211 for training of the unmasking status estimation engine 210, along with any associated class labels. In other embodiments, the training spectral data may further include a class label such as un-retrieved, fully retrieved, or partially retrieved.

When the training of the unmasking status estimation engine 210 is complete, the system 200 is ready to operate for detect features within test spectral data and, based on the detected features, estimate an unmasking status of the test biological specimen, e.g. unmasking conditions including temperature and/or duration of the unmasking process applied. In some embodiments, the unmasking status estimation engine 210 may be periodically retrained to adapt for variations in input data.

### ESTIMATION OF UNMASKING STATUS

Once the unmasking status estimation engine 210 has been appropriately trained, such as described above, it may be used to detect features within test vibrational spectral data and, based on the detected features, predict an unmasking status, e.g. the duration and/or temperature of an unmasking process. In some embodiments, and with reference to FIG. 3, a test biological specimen is obtained (step 310) (such as from a subject suspected of having a certain disease or known to have a certain disease) and then test vibrational spectral data is acquired from that test biological specimen (step 320) (see also FIG. 5). In some embodiments, the test vibrational spectral data includes the absorbance spectra, the first and/or second derivatives of the absorbance spectra, magnitudes of individual bands within the training spectra data, the integral of bands within the training spectral data, the ratio of two or more band intensities within the training spectral data, the ratios from second and third order derivatives of the training spectral data, etc.

Once test vibrational spectral data and/or the variants thereof described above have been acquired and processed, unmasking features may be derived from the test spectral data using the trained unmasking status estimation engine 210 (step 340). In some embodiments, the derived unmasking features are a mapping of how relevant each wavenumber is to predicting retrieval status. For example, and as illustrated in FIG. 11, and with specific reference to the graph of PLSR coefficients, large positive coefficients mean that a wavenumber is positively correlated with retrieval status; while large negative numbers mean that a wavenumber is negatively correlated with retrieval status. Values close to zero have little significance. In some embodiments, unmasking features that may be detected include peak amplitudes, peak positions, peak ratios, a sum of spectral values (such as the integral over a certain spectral range), one or more changes in slope (first derivative) or changes in curvature (second derivative), etc. Based on the derived unmasking features, an unmasking status estimate may be computed (step 350).

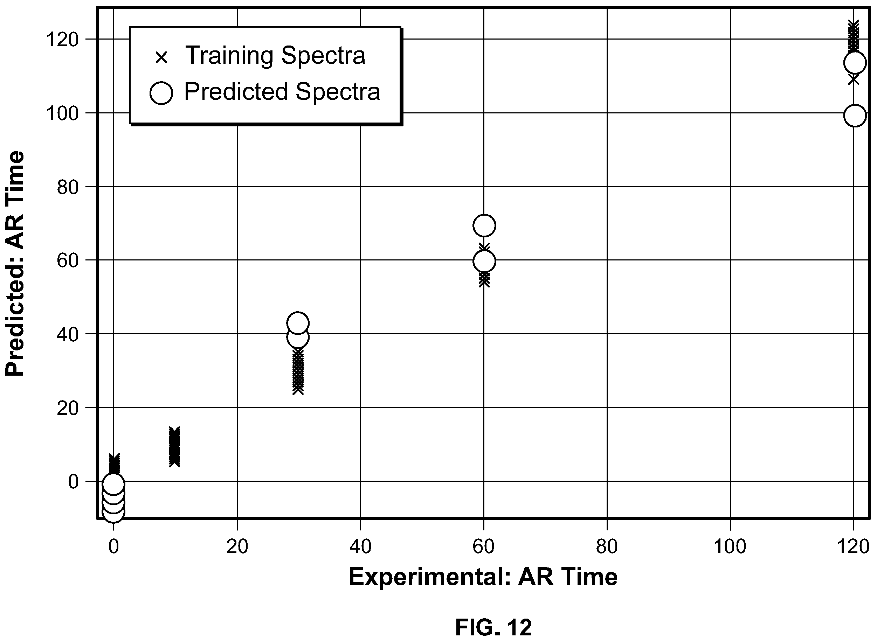

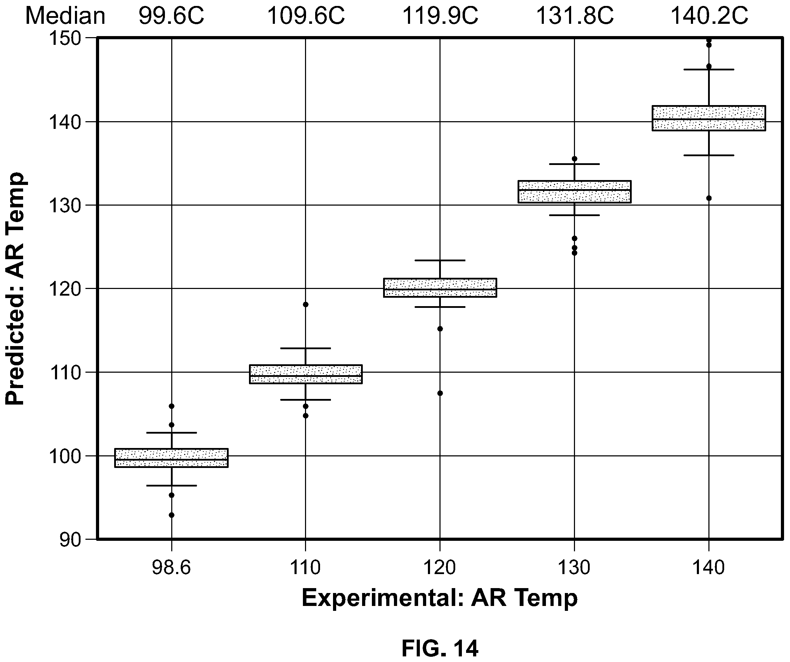

In embodiments where the unmasking status estimation engine 210 is trained with training spectral data including known unmasking temperature and duration class labels, the unmasking status estimation engine 210 may provide as output a predicted temperature and/or duration of an unmasking process to which the test biological specimen was subjected. This concept is further described in Example 1 herein. By way of example, FIGS. 12 and 14 illustrate the results achieved using a trained unmasking status estimation engine 210. FIGS. 12 and 14 comparatively illustrate the predicted unmasking durations and temperatures to known (experimental) unmasking durations and temperatures for differentially unmasked test biological specimens. As illustrated, the unmasking status estimation engine 210 is able to accurately predict unmasking times and/or unmasking durations across differentially unmasked specimens.

The skilled artisan will appreciate that the information provided as output may be used in further downstream processes and may be used to render decisions as to whether the test biological specimen should be treated with one or more specific binding entities.

### Example 1 - Estimation of Unmasking Status Using a Trained Unmasking Status Estimation Engine

### Abstract

This project utilizes mid-infrared (mid-IR) spectroscopy to interrogate the vibrational state of molecules in histological tissue sections. The testable hypothesis is that the vibrational state of the tissue, as judged by the mid-IR signal, will manifest conformational changes in the tissue composition that occur during the antigen retrieval (AR) process.

A metrology has been developed that correlates changes in the mid-IR spectra with the antigen retrieval state of the tissue. This metrology was used to examine differences between low and high temperature retrievals. The mid-IR results are corroborated by staining results that show different staining intensities resulting from low and high temperature antigen retrieval, indicating that these treatments do not leave the tissue in equivalent states.

In this work changes in the mid-IR spectra due to differentially retrieved tonsil tissues were studied. In particular time and temperature, the two largest variables that affect antigen retrieval, were independently evaluated. The identified shifts in the mid-IR spectra were correlated with immunohistochemical (IHC) staining for Ki-67 and C4d proteins. Results were validated on tonsil and breast tissue, indicating this metrology is generalizable to different types of tissues. Furthermore, it was shown that the tissue is in a drastically different state after CC1-based antigen retrieval and CC2-based target retrieval. Thus, the presented method is applicable to multiple retrieval methods.

### INTRODUCTION

Mid infrared spectroscopy (mid-IR) is a powerful optical technique that probes the vibrational state of individual molecules in the tissue and is very sensitive to the conformational state of proteins. This extreme sensitivity makes mid-IR spectroscopy ideally suited for microscopy applications because the presence and even conformational state of endogenous and exogenous materials manifest through changes in the mid-IR absorption profile of the biospecimen. Vibrational spectroscopy has even been used for diagnostic applications, for example to distinguish healthy from cancerous tissue.

Although the exact mechanism of retrieval is not fully understood, the unmasking of epitopes requires significant change to the tissue's biocomposition. It was therefore sought to investigate if changes in a biospecimen's molecular composition would manifest in changes in the mid-IR spectrum. The ultimate goal was to use the mid-IR spectra to develop a metrology that could be used to accurately determine the antigen retrieval of a tissue sample. Such a novel capability would enable the investigator to assess the retrieval status of a tissue sample with a standardized and objective metrology.

Antigen retrieval has become a mainstay of modern immunohistochemistry because it unmasks protein epitopes so that they can be detected by IHC based detection. Although the exact mechanism of antigen retrieval remains speculative it is a critical chemical process that enables IHC to be detected on FFPE tissues. Retrieval is thus fundamentally critical to detect current cancer biomarkers and even the degree to which a tissue is retrieved can significantly impact detected protein expression levels. Despite its importance the precise mechanism of antigen retrieval remains unclear and the are no analytical methods to measure the quality of antigen retrieval.

### METHOD AND MATERIALS

### Retrieval Procedure

MirrIR microscope slides (Kevley Technologies, Chesterland, OH) for reflective infrared studies were used for the mid-IR spectra measurements. Four-micron serial sections of formalin-fixed paraffin-embedded (FFPE) tonsil tissue were placed on pre-treated MirrIR slides. Deparaffinization of tonsil tissue was performed manually according to OP2100-025. Briefly, after xylene steps slides were hydrated through descending grades of ethanol and then transferred in the VENTANA Cell Conditioning 1 (CC1) solution to the Rapid Antigen Retrieval (RAR) test-bed.

The antigen retrieval step was performed in CC1 solution in the RAR chamber, which was pre-pressurized to 30 psi before heaters were turned on. The total heating time for any given experiment included 90 seconds ramp-up time and 2 minutes of cooling time. After the antigen retrieval step, the slides were gently washed in deionized water and air-dried at room temperature. Dried slides with intact tonsil tissues were used for the mid-IR measurements.

### IHC Staining and Quantitation

Immunoreactivity data was collected for all samples and treatments analyzed with mid-IR spectroscopy. Briefly, samples were processed using a hybrid procedure where deparaffinization and antigen retrieval were performed manually. Deparaffinization (depar) was performed using xylene followed by rehydration through a graded alcohol series. Samples were then placed in CC1 (catalog number: 950-124). After antigen retrieval samples were transferred in reaction buffer (catalog number: 950-300) to a BenchMark UTLRA instrument for subsequent processing steps from peroxide inhibitor through counterstain.

For the studies presented here, tonsil samples were labeled with antisera raised against Ki-67 (30-9) or C4d (SP91). These markers were selected because they show different responses to increasing antigen retrieval treatments. Ki-67 increases in stain intensity and number of labeled cells to a point after which intensity and positivity decrease. Conversely, C4d continues to increase in intensity and positivity through antigen retrieval conditions that otherwise damage the sample. C4d was additionally selected because it displays poor performance when treated with current retrieval methods, but clear immunoreactivity when treated with high temperature antigen retrieval (this behavior is described in detail in the addendum to D081973 entitled Stain Quality Improvements from Rapid Antigen Retrieval).

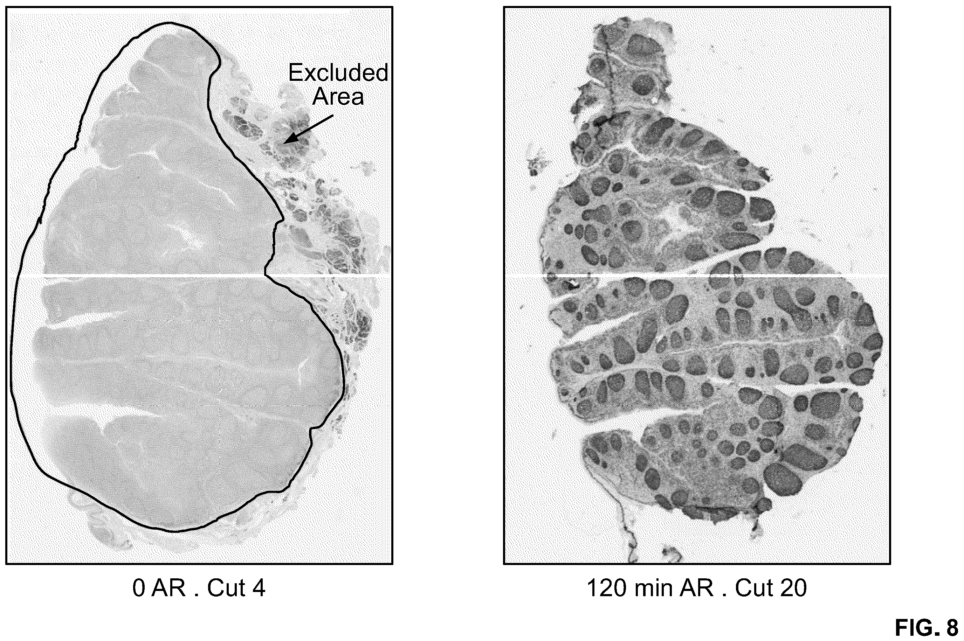

Sample slides were scanned using a Leica Aperio AT2 (Leica Biosystems, Nussloch, Germany) slide scanner and the intensity of immunoreactivity and the proportion of tissue stained was quantified using the "Positive Pixel Count v9" algorithm supplied with Aperio Imagescope software. For each tissue, a region of interest (ROI) was selected to include tonsil tissue expected to stain. Connective tissue which showed high background with some staining treatments but that was missing in others was excluded as illustrated in FIG. 8.

This method of quantification produces intensity units that are repeatable across samples and can be compared within an experiment. However, no attempt was made to map or reconcile the intensity measurements, or the percentage of positive pixels reported to pathologist scores.

### Collection of Mid-IR Data

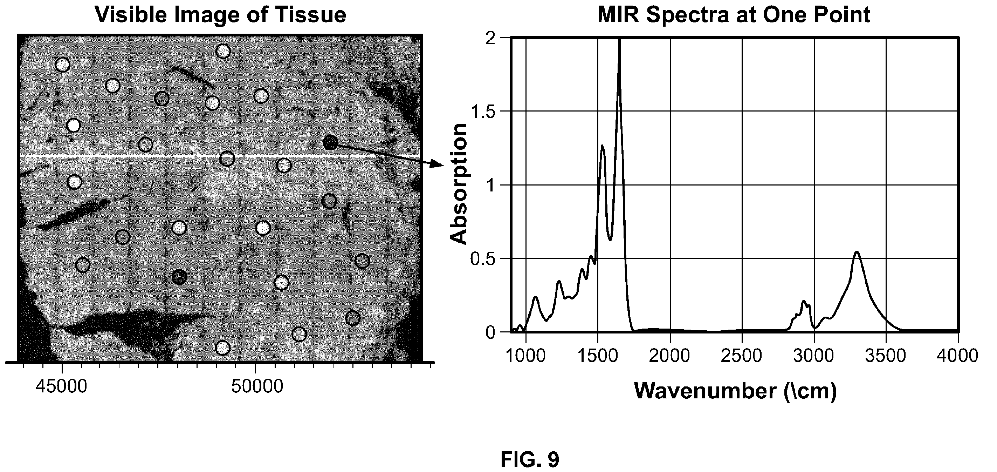

The mid-IR spectra were collected on a Fourier Transform Infrared (FTIR) microscope (Bruker Hyperion 3000, Bruker Optics, Billerica MA) with an attached optical interferometer (Vertex 70). Serial sections from tonsil blocks were sectioned 4 micrometer thick onto mid-IR reflective slides (Kevley Technologies, MirrIR), differentially retrieval, and imaged with the mid-IR microscope. Tonsils tissue sections retrieved under different experimental conditions were placed on the FTIR microscope and the entire tissue section was imaged with a visible objective by raster scanning the field of view (FOV). The Bruker software OPUS was used to randomly select regions of tissue from which mid-IR spectra were collected using a mercury-cadmium-telluride (MCT) detector. Typically, between about 20 to about 80 spectra were collected from each tissue sample. Absorption spectra were collected at a resolution of about 4 cm-1 and each selected ROI was sampled 64 times and these spectra were averaged together to yield the final spectra for a given position. An example tissue image, sampling pattern and the resulting average spectra for a single ROI are shown below in FIG. 9. All spectra were collected with a 15X IR objective producing roughly a 200µm x 200µm FOV.

### Preprocessing mid-IR Data

Collected spectra were preprocessed to remove artifacts, standardize the format of the spectra, and to isolate the mid-IR absorption of the tissue. The microscope directly measures mid-IR transmission. To convert transmission spectra to absorption spectra a reference transmission spectrum was collected at a spatial location outside of the sample and used to divide the spectrum collected through the tissue. This calculation provides the amount of light attenuated (absorbed + scattered) by the tissue. Next, atmospheric absorption, primarily from water vapor and carbon dioxide, were removed using algorithms in the OPUS software. Baseline correction was then used to correct for tissue scattering using a concave rubberband correction (8 iterations, 64 baseline points). The resulting spectrum represents absorption by the sample tissue. Finally, all spectra were normalized to a maximum value of 2 to mitigate differences in section thickness and tissue density.

### EXPERIMENTAL DESIGN AND RESULTS

### Variation of the Antigen Retrieval Time at Constant Antigen Retrieval Temperature

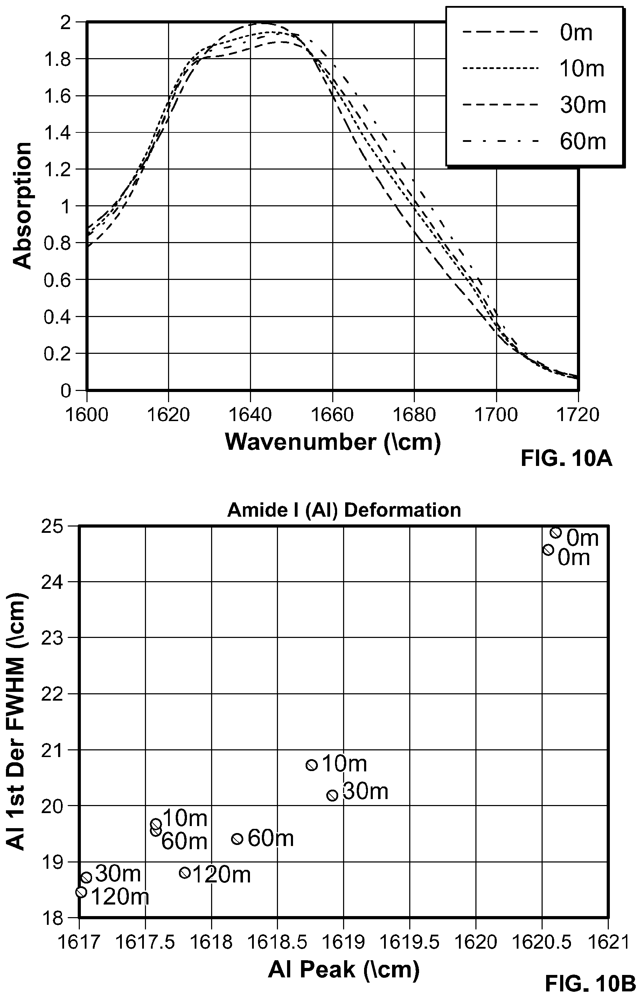

In this experiment, antigen retrieval was performed on tonsil tissue at about 98.6°C for either about 0, about 10, about 30, about 60, or about 120 minutes. Each treatment was run on duplicate samples. The mid-IR spectra show a conspicuous shift in the primary protein band, referred to as the Amide I band, that was loosely correlated with antigen retrieval treatment. Examples of this Amide I shift are shown in FIG. 10A. Quantification of the Amide I band's peak wavelength versus full width at half maximum (FWHM) enables course discrimination of antigen retrieval treatment into un-retrieved and partially, fully, and over retrieved (FIG. 10B).

Multiple other metrics were evaluated throughout this project including principal component analysis, integration of the Amide I band, normalization to several bands to correct for scattering, and quantitation of the methyl and methylene peaks. Unfortunately, none of these other metrics were able to improve the level of stratification of the antigen retrieval status of tissue. In the end, a supervised machine learning model was established to make use of non-obvious signatures in the mid-IR spectra that indicated the antigen retrieval status of the tissue. These small differences in spectra were identified using the projection onto latent structure regression (PLSR) method. This algorithm takes the mid-IR signal (e.g. absorption spectra, first derivative, second derivative) and creates a model that is used to determine which features (wavelengths) are most predictive of the response variable (antigen retrieval status, target retrieval status, etc.). The generated model was then evaluated for performance using the same and unknown mid-IR data for performance evaluation and optimization. FIG. 11 illustrates how the PLSR model is trained to mine the mid-IR spectra for the antigen retrieval signature. In this experiment the model had an accuracy of 3 minutes.

These studies demonstrate that the supervised machine learning model is able to mine the data and develop a method that can be used to determine the antigen retrieval time in a tonsil sample. To further verify that the model recognizes a true antigen retrieval signature, a series of spectra that the algorithm was not trained on were given to the model to determine how well it could make blinded predictions. As seen in FIG. 12, the model was able to predict the antigen retrieval time of unknown spectra (purple dots) to within 8 minutes, demonstrating that the model found a robust antigen retrieval signature in the mid-IR spectra.

### Variation of the Antigen Retrieval Time and Temperature

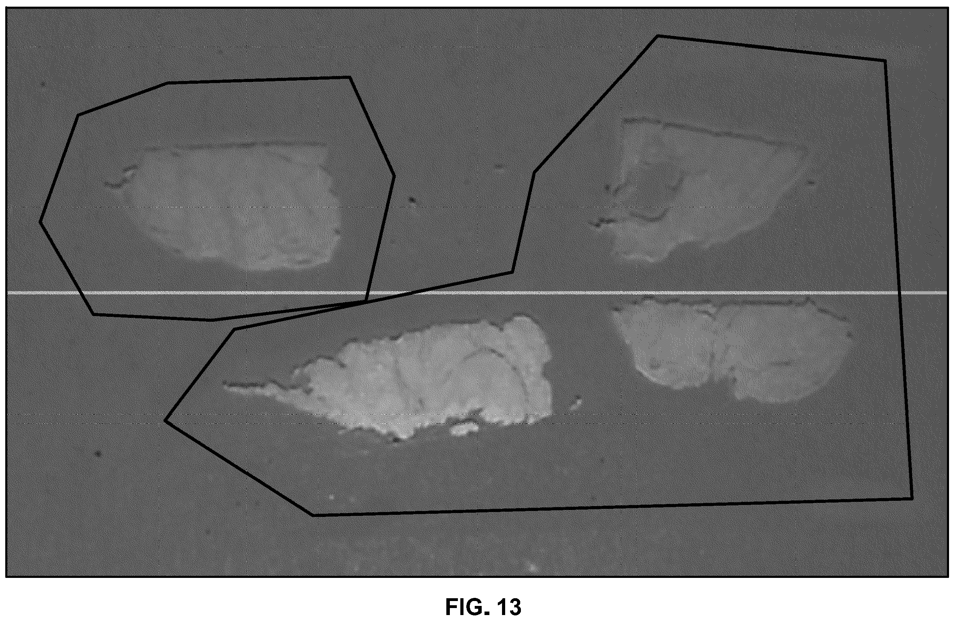

In this study, it was investigated how well the mid-IR spectra coupled with machine learning models could be used to estimate antigen retrieval time and temperature. Five multi-tissue slides with four separate tonsil tissues were retrieved for 5 minutes at temperatures between about 98.6°C and about 140°C. The mid-IR spectra from three tonsil tissues (FIG. 13, circled portion including three tissue specimens) were used to train the PLSR model. This model was then used to infer the antigen retrieval conditions in the "unknown" tonsil tissue (FIG. 13).

The model's performance on the blinded (FIG. 13, circled portion including only a single tissue specimen) tonsil sample was additionally tested with the same temperature course performed at about 30 and about 60 minutes of antigen retrieval (see FIG. 14).

The results from FIG. 9 demonstrate, at least in tonsil tissue, that across all times and temperatures the mid-IR spectra coupled with PLSR is able to accurately quantify the degree to which an unknown sample is retrieved. This is of critical importance because time and temperature are the two most important variables that impact antigen retrieval.

### Comparison of Two Extreme Antigen Retrieval Conditions

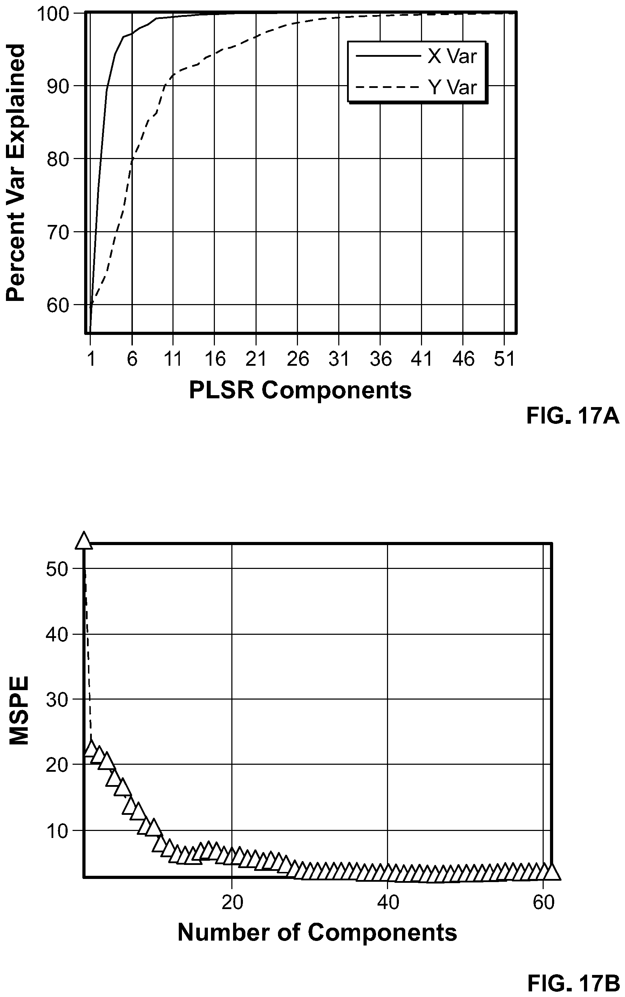

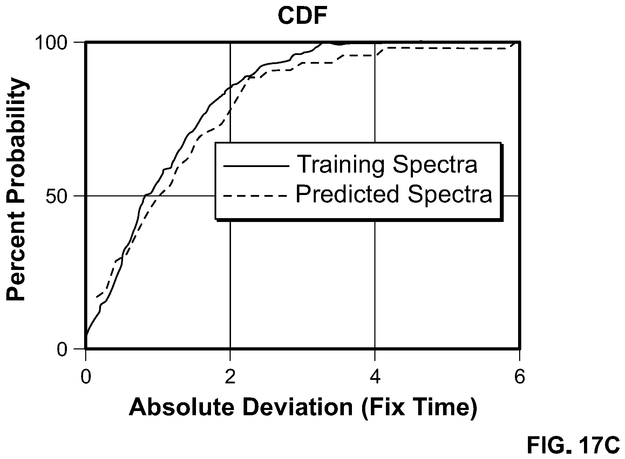

In this experiment, two completely different antigen retrieval conditions were compared using the mid-IR spectra and IHC staining for C4d protein. The antigen retrieval step in tonsil tissues was performed in the RAR testbed. The first antigen retrieval treatment was 60 minutes at about 98.6°C, which mimics the antigen retrieval conditions typically used on the Ventana BenchMark ULTRA platform. The second antigen retrieval condition tested was 5 minutes at 140°C. For this study, each sample was imaged about 128 times in a grid pattern near the center of each tissue as shown in FIGS. 17A and 17B.

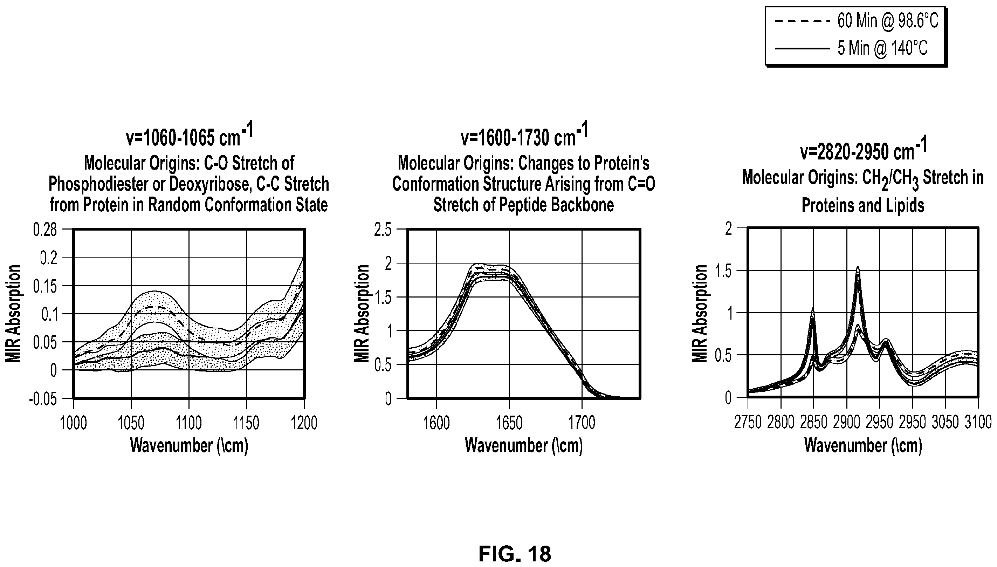

Visual inspection of the mid-IR spectra from the two differently treated samples reveals significant differences (FIG. 18). For instance, the mid-IR absorption near 1060 cm-1 (FIG. 18, left) exhibits a distinct absorption band for the low temperature/long time antigen retrieval sample (dashed trace) that is not present in the high temperature/short time sample (solid trace). This probably indicates that the nucleic acids of the tissue are in a different conformation state and that the low temperature nucleic acids are able to absorb mid-IR energy. Similarly, the primary protein band located between about 1600 to about 1700 cm-1 (FIG. 18, center) also has large differences with a noticeable deformation in the low temperature/long time tissue that is not present with the high temperature/short time antigen retrieval samples. Finally, substantial differences are observed in the methyl and methylene absorption bands between about 2800 to about 2950 cm-1 (FIG. 18, right). These bands are related to the mid-IR absorption of proteins and lipids. Further studies are necessary to understand the molecular basis of these different signals; however, this data supports the theory that tissue specimen are in very different conformational states after low and high temperature antigen retrieval.

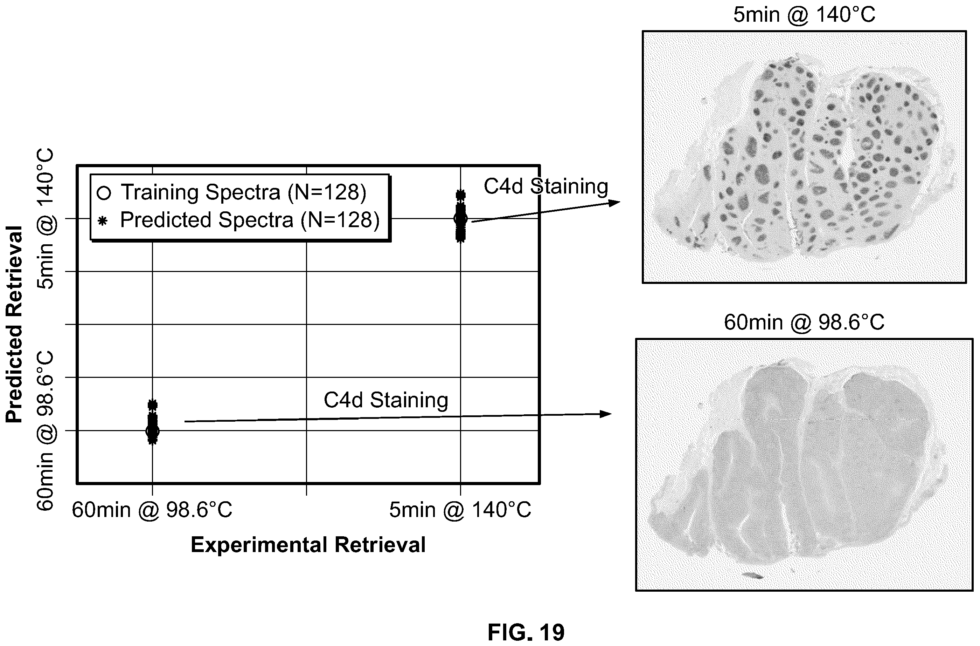

Additionally, these two antigen retrieval protocols were distinguishable using the machine learning algorithm both with training and blinded spectra. The apparent differences in the mid-IR spectra are mirrored by distinct IHC staining for C4d (FIG. 19). Little if any immunoreactivity is visible in the sample retrieved for an hour at about 98.6°C while the sample treated with about 5 minutes of antigen retrieval at about 140°C shows significant C4d immunoreactivity in germinal centers.

### Antigen Retrieval of Breast Tissue

This section expands on the results achieved in tonsil tissue by applying the same methodology to breast tissue. Four different pieces of breast tissue were differentially retrieved at a temperature of about 98.6°C, about 110°C, about 120°C, about 130°C, or about 140°C all for a constant time of 5 minutes. Each tissue was cut in quadruplicate so that four average spectra could be obtained for each antigen retrieval temperature.

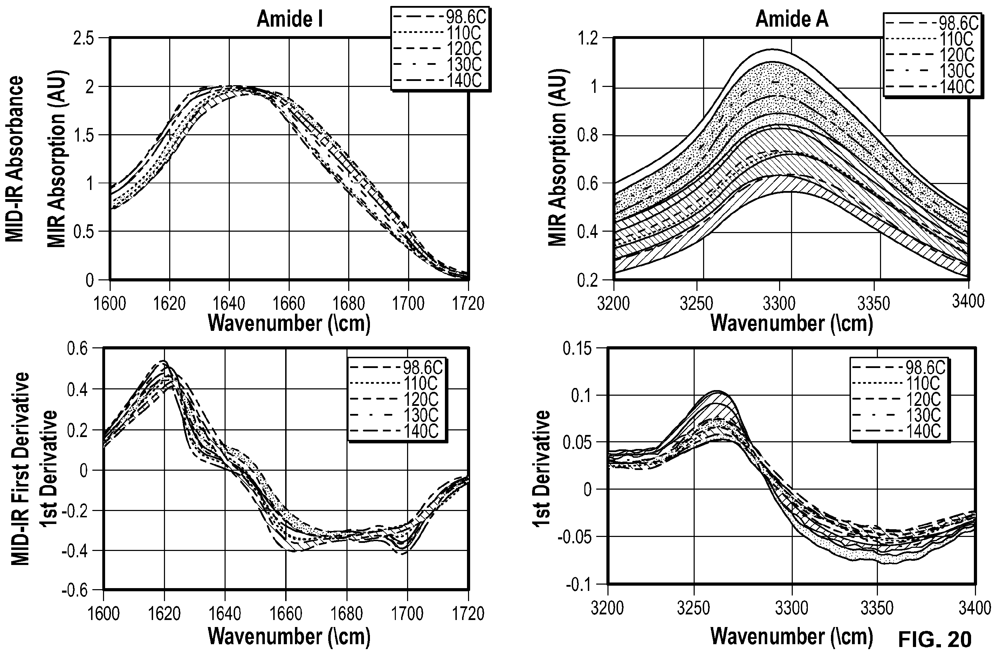

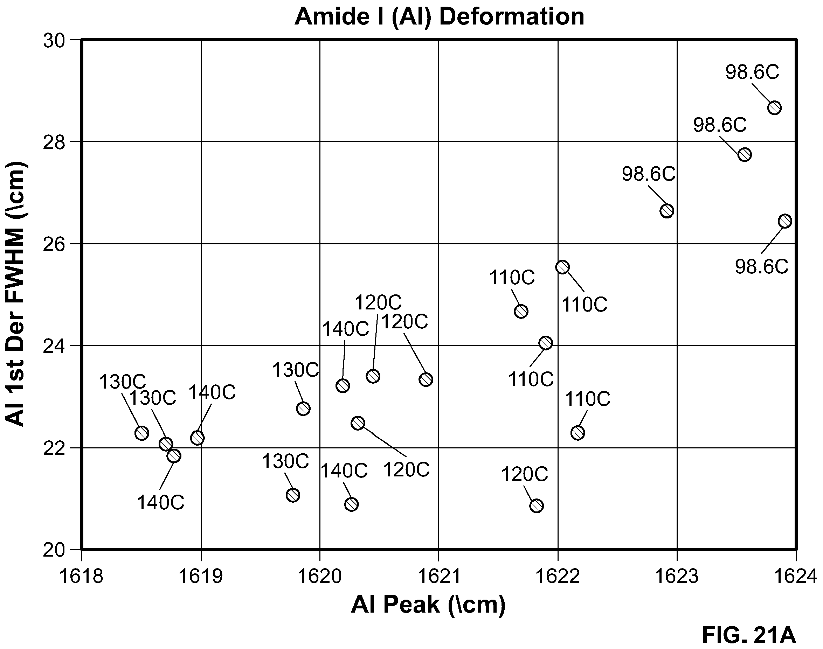

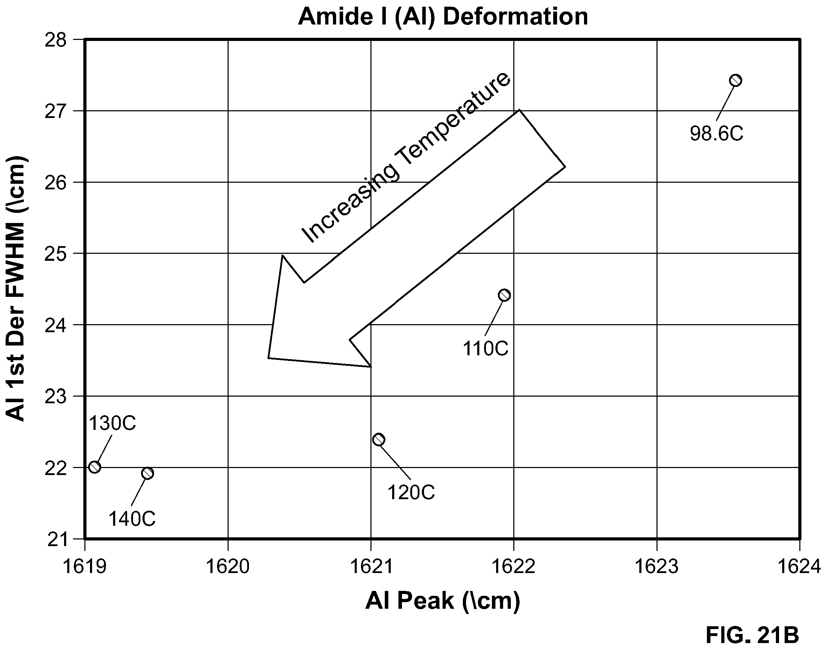

Visible differences can be seen in the mid-IR absorbance spectra as well as the first derivative spectra of differentially retrieved tissues as can be seen in FIG. 20. FIGS. 21A and 21B present a quantitative view of the Amide I band's first derivative peak location versus FWHM. There is a clear trend toward narrower bands and lower peak wavenumbers as antigen retrieval progresses. This method was able to coarsely resolve antigen retrieval temperature although it failed to work for antigen retrieval time. Several other analysis methods were investigated that also failed to reliably and accurately differentiate samples based on the antigen retrieval status including, looking at the magnitudes of individual bands, the integral of bands, ratio of about 2 or more bands intensities, as well as magnitudes and ratios from the 2nd and 3rd order derivative spectra.

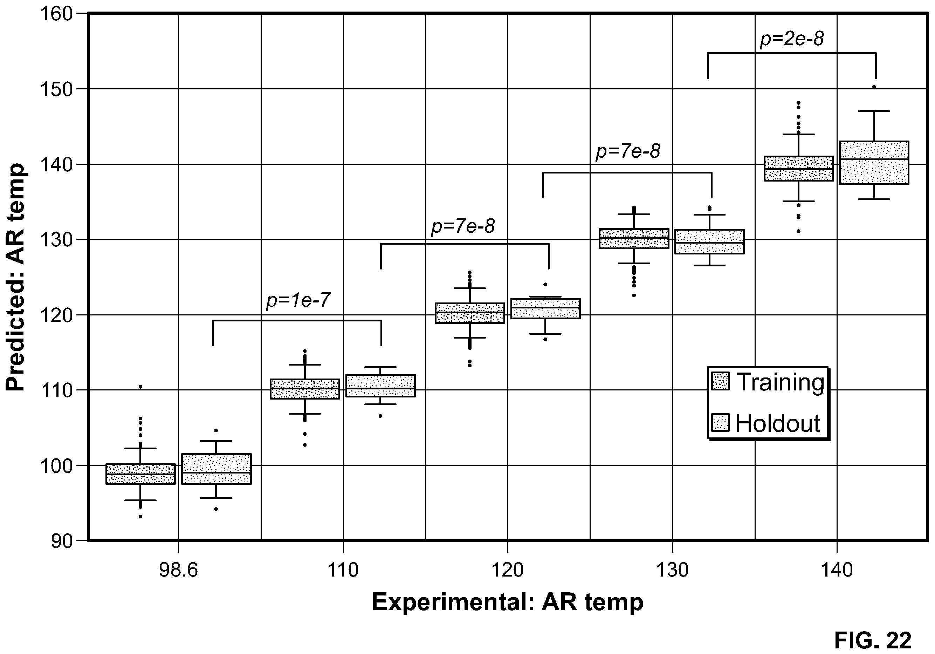

To achieve more accurate results which would be needed for a practical embodiment of this technology a PLSR model was trained and tested on blinded spectra, results are shown in FIG. 22. Looking exclusively at the blinded spectra the trained model was capable of determining the antigen retrieval temperature of tissue to about 2°C.

### Target Retrieval in Breast Tissue

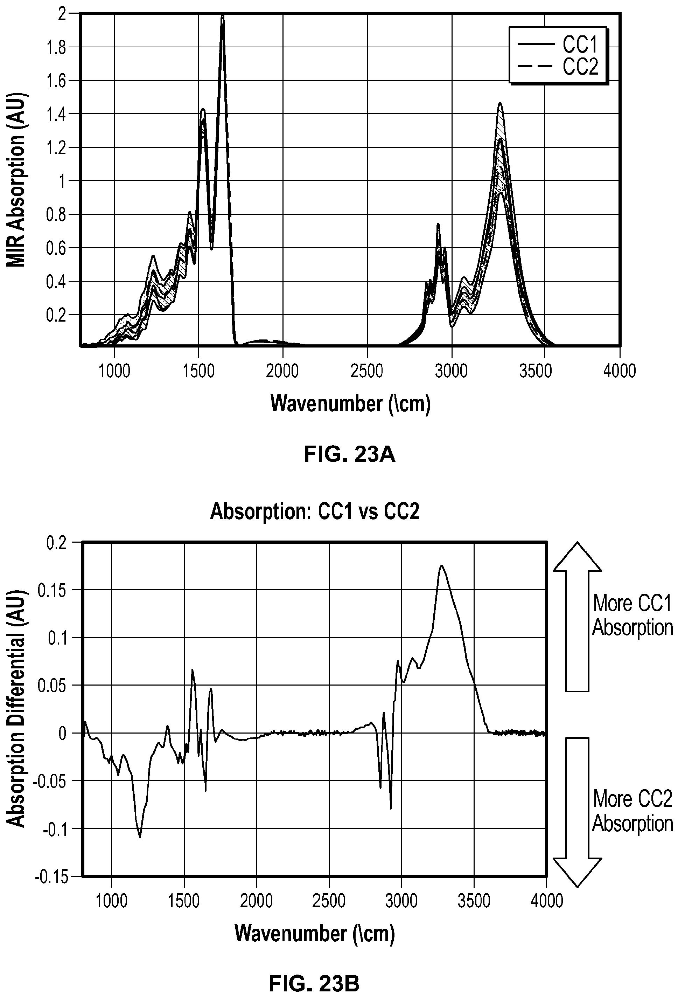

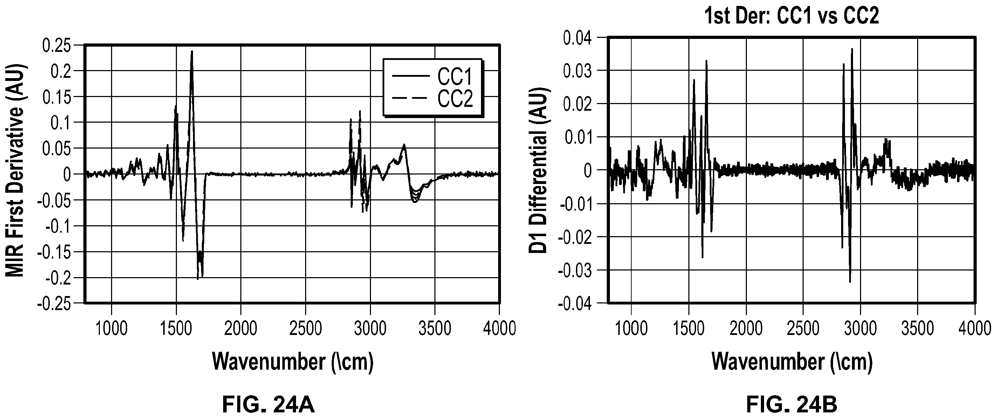

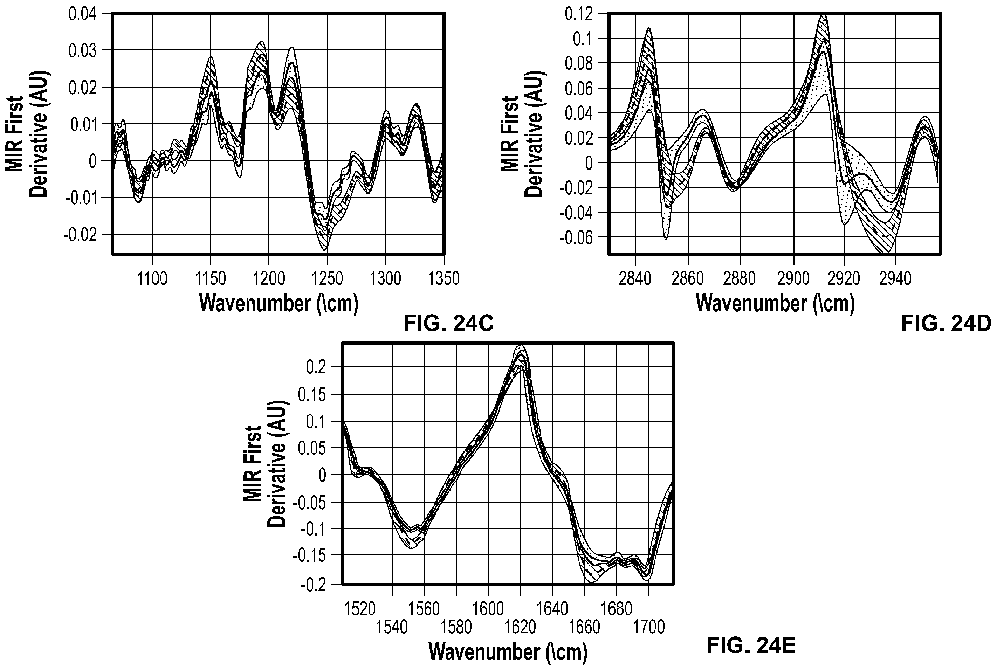

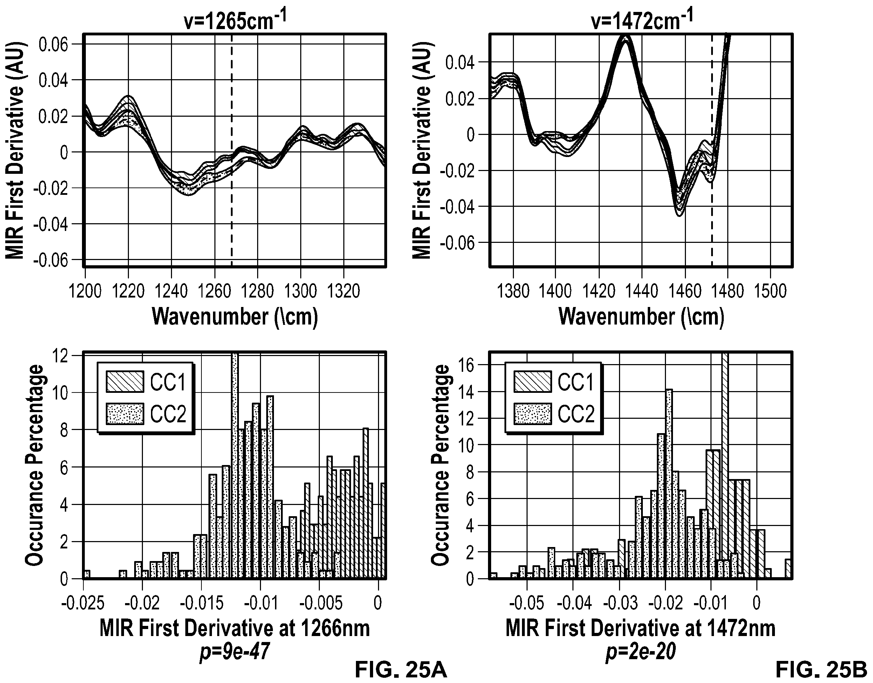

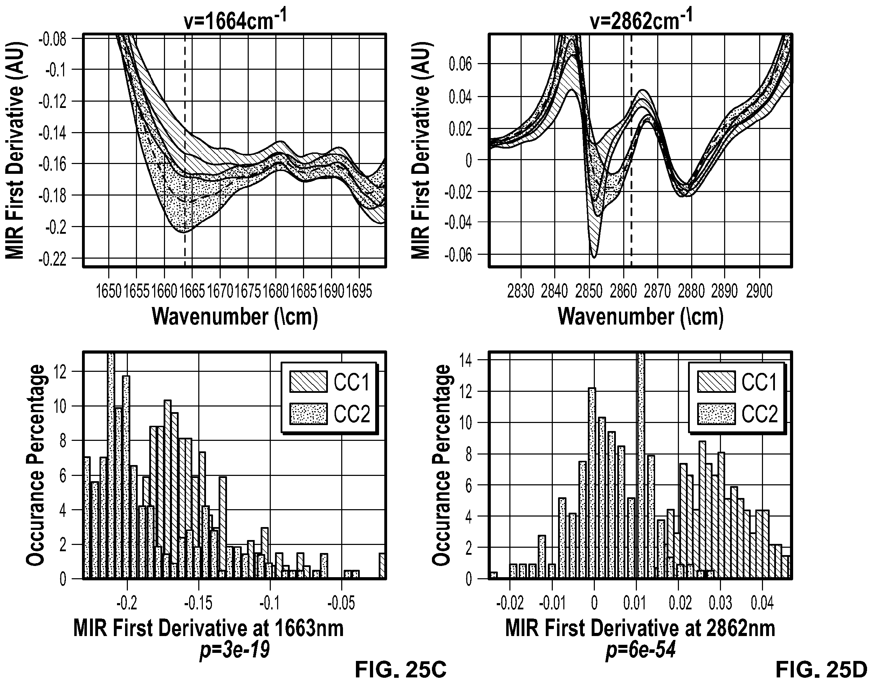

This section presents proof-of-principle data that tissue is in a significantly different configuration after CC1-based antigen retrieval and CC2-based target retrieval Serial sections of breast tissue was retrieved was retrieved using either CC1 of CC2. The average absorption spectra are plotted in FIGS. 23A and 23B with clear differences observed throughout the spectra. FIG. 24 displays the first derivative spectra at several different bands. Statistical analysis confirms that the spectra are significantly different at bands throughout the electromagnetic range. This indicates the tissue is in drastically different states after CC1 versus CC2 retrieval and that mid-IR spectroscopy can detect and resolve these spectral differences. FIG. 25 provides yet further views of differences between the use of CC1 and CC2.

### SUMMARY AND DISCUSSION

This work establishes a proof-of-concept for the hypothesis that mid-IR spectroscopy can be used to quantitate the antigen retrieval process when driven by time or temperature. It also established an independent metrology, which is corroborated by staining results, that indicates that certain antibodies have different immunorecognition of targets subjected to low temperature versus high temperature retrieval conditions. The mid-IR spectra thus provided proof that histological tissue sections are not in identical states after low and high temperature retrieval. Findings were verified in tonsil and breast tissue.

### Example 2 - Training an Unmasking Status Estimation Engine

In one embodiment a principal component analysis plus discriminate analysis (PCDA) algorithm is used to determine the retrieval status of a tissue. Mid-IR spectra were collected from roughly one hundred locations throughout a tissue sample, so the average spectrum is representative of the average of the tissue. All spectra were atmospherically corrected to remove CO2 contamination, baselinecorrected using concave rubber band correction with 10 iterations and 64 baseline points, then the acquired spectra were amplitude normalized. Finally, all the spectra from each tissue were averaged together. Next, a PCDA model was used to classify a group in which a given spectrum belonged to. The two main variables in this algorithm were the number of principal components that were used for classification purposes and the type of discriminate analysis (linear or quadratic). One example is shown in FIG. 7A in which the first two principal components are plotted for a series of slides retrieval for different amounts of time. The discriminate analysis thus determined the optimal break points and classified each spectrum as from one group of retrieval condition. The skilled artisan will appreciate that as the number of principal components is increased, this approach can be a viable option to determine the retrieval condition of a slide based on its MID-IR spectra. Confusion matrices are depicted in FIGS. 7B illustrating that classification performance may be further improved as more components are utilized during training.

### Other System Components

The system 200 of the present disclosure may be tied to a specimen processing apparatus that can perform one or more preparation processes on the tissue specimen. The preparation process can include, without limitation, deparaffinizing a specimen, conditioning a specimen (e.g., cell conditioning), staining a specimen, performing antigen retrieval, performing immunohistochemistry staining (including labeling) or other reactions, and/or performing in situ hybridization (e.g., SISH, FISH, etc.) staining (including labeling) or other reactions, as well as other processes for preparing specimens for microscopy, microanalyses, mass spectrometric methods, or other analytical methods.

The processing apparatus can apply fixatives to the specimen. Fixatives can include cross-linking agents (such as aldehydes, e.g., formaldehyde, paraformaldehyde, and glutaraldehyde, as well as non-aldehyde cross-linking agents), oxidizing agents (e.g., metallic ions and complexes, such as osmium tetroxide and chromic acid), protein-denaturing agents (e.g., acetic acid, methanol, and ethanol), fixatives of unknown mechanism (e.g., mercuric chloride, acetone, and picric acid), combination reagents (e.g., Carnoy's fixative, methacarn, Bouin's fluid, B5 fixative, Rossman's fluid, and Gendre's fluid), microwaves, and miscellaneous fixatives (e.g., excluded volume fixation and vapor fixation).

If the specimen is a sample embedded in paraffin, the sample can be deparaffinized using appropriate deparaffinizing fluid(s). After the paraffin is removed, any number of substances can be successively applied to the specimen. The substances can be for pretreatment (e.g., to reverse protein-crosslinking, expose cells acids, etc.), denaturation, hybridization, washing (e.g., stringency wash), detection (e.g., link a visual or marker molecule to a probe), amplifying (e.g., amplifying proteins, genes, etc.), counterstaining, coverslipping, or the like.

The specimen processing apparatus can apply a wide range of substances to the specimen. The substances include, without limitation, stains, probes, reagents, rinses, and/or conditioners. The substances can be fluids (e.g., gases, liquids, or gas/liquid mixtures), or the like. The fluids can be solvents (e.g., polar solvents, non-polar solvents, etc.), solutions (e.g., aqueous solutions or other types of solutions), or the like. Reagents can include, without limitation, stains, wetting agents, antibodies (e.g., monoclonal antibodies, polyclonal antibodies, etc.), antigen recovering fluids (e.g., aqueous- or non-aqueous-based antigen retrieval solutions, antigen recovering buffers, etc.), or the like. Probes can be an isolated cells acid or an isolated synthetic oligonucleotide, attached to a detectable label or reporter molecule. Labels can include radioactive isotopes, enzyme substrates, cofactors, ligands, chemiluminescent or fluorescent agents, haptens, and enzymes.

Staining may performed with a histochemical staining module or separate platform, such as an automated IHC/ISH slide stainer. Automated IHC/ISH slide stainers typically include at least: reservoirs of the various reagents used in the staining protocols, a reagent dispense unit in fluid communication with the reservoirs for dispensing reagent to onto a slide, a waste removal system for removing used reagents and other waste from the slide, and a control system that coordinates the actions of the reagent dispense unit and waste removal system. In addition to performing staining steps, many automated slide stainers can also perform steps ancillary to staining (or are compatible with separate systems that perform such ancillary steps), including: slide baking (for adhering the sample to the slide), dewaxing (also referred to as deparaffinization), antigen retrieval, counterstaining, dehydration and clearing, and coverslipping. Prichard, Overview of Automated Immunohistochemistry, Arch Pathol Lab Med., Vol. 138, pp. 1578-1582 (2014), describes several specific examples of automated IHC/ISH slide stainers and their various features, including the intelliPATH (Biocare Medical), WAVE (Celerus Diagnostics), DAKO OMNIS and DAKO AUTOSTAINER LINK 48 (Agilent Technologies), BENCHMARK (Ventana Medical Systems, Inc.), Leica BOND, and Lab Vision Autostainer (Thermo Scientific) automated slide stainers. Additionally, Ventana Medical Systems, Inc. is the assignee of a number of United States patents disclosing systems and methods for performing automated analyses, including U.S. Pat. Nos. 5,650,327, 5,654,200, 6,296,809, 6,352,861, 6,827,901 and 6,943,029, and U.S. Published Patent Application Nos. 20030211630 and 20040052685.

Commercially-available staining units typically operate on one of the following principles: (1) open individual slide staining, in which slides are positioned horizontally and reagents are dispensed as a puddle on the surface of the slide containing a tissue sample (such as implemented on the DAKO AUTOSTAINER Link 48 (Agilent Technologies) and intelliPATH (Biocare Medical) stainers); (2) liquid overlay technology, in which reagents are either covered with or dispensed through an inert fluid layer deposited over the sample (such as implemented on VENTANA BenchMark and DISCOVERY stainers); (3) capillary gap staining, in which the slide surface is placed in proximity to another surface (which may be another slide or a coverplate) to create a narrow gap, through which capillary forces draw up and keep liquid reagents in contact with the samples (such as the staining principles used by DAKO TECHMATE, Leica BOND, and DAKO OMNIS stainers). Some iterations of capillary gap staining do not mix the fluids in the gap (such as on the DAKO TECHMATE and the Leica BOND). In variations of capillary gap staining termed dynamic gap staining, capillary forces are used to apply sample to the slide, and then the parallel surfaces are translated relative to one another to agitate the reagents during incubation to effect reagent mixing (such as the staining principles implemented on DAKO OMNIS slide stainers (Agilent)). In translating gap staining, a translatable head is positioned over the slide. A lower surface of the head is spaced apart from the slide by a first gap sufficiently small to allow a meniscus of liquid to form from liquid on the slide during translation of the slide. A mixing extension having a lateral dimension less than the width of a slide extends from the lower surface of the translatable head to define a second gap smaller than the first gap between the mixing extension and the slide. During translation of the head, the lateral dimension of the mixing extension is sufficient to generate lateral movement in the liquid on the slide in a direction generally extending from the second gap to the first gap. See WO 2011-139978 A1. It has recently been proposed to use inkjet technology to deposit reagents on slides. See WO 2016-170008 A1. This list of staining technologies is not intended to be comprehensive, and any fully or semi-automated system for performing biomarker staining may be incorporated into the histochemical staining platform.

Where a morphologically-stained sample is also desired, an automated H&E staining platform may be used. Automated systems for performing H&E staining typically operate on one of two staining principles: batch staining (also referred to as "dip 'n dunk") or individual slide staining. Batch stainers generally use vats or baths of reagents in which many slides are immersed at the same time. Individual slide stainers, on the other hand, apply reagent directly to each slide, and no two slides share the same aliquot of reagent. Examples of commercially available H&E stainers include the VENTANA SYMPHONY (individual slide stainer) and VENTANA HE 600 (individual slide stainer) series H&E stainers from Roche; the Dako CoverStainer (batch stainer) from Agilent Technologies; the Leica ST4020 Small Linear Stainer (batch stainer), Leica ST5020 Multistainer (batch stainer), and the Leica ST5010 Autostainer XL series (batch stainer) H&E stainers from Leica Biosystems Nussloch GmbH.

After the specimens are stained, the stained samples can be manually analyzed on a microscope, and/or digital images of the stained samples can be acquired for archiving and/or digital analysis. Digital images can be captured via a scanning platform such as a slide scanner that can scan the stained slides at 20x, 40x, or other magnifications to produce high resolution whole-slide digital images. At a basic level, the typical slide scanner includes at least: (1) a microscope with lens objectives, (2) a light source (such as halogen, light emitting diode, white light, and/or multispectral light sources, depending on the dye), (3) robotics to move glass slides around or to move the optics around the slide or both, (4) one or more digital cameras for image capture, (5) a computer and associated software to control the robotics and to manipulate, manage, and view digital slides. Digital data at a number of different X-Y locations (and in some cases, at multiple Z planes) on the slide are captured by the camera's charge-coupled device (CCD), and the images are joined together to form a composite image of the entire scanned surface. Common methods to accomplish this include:
(1) Tile based scanning, in which the slide stage or the optics are moved in very small increments to capture square image frames, which overlap adjacent squares to a slight degree. The captured squares are then automatically matched to one another to build the composite image; and
(2) Line-based scanning, in which the slide stage moves in a single axis during acquisition to capture a number of composite image "strips." The image strips can then be matched with one another to form the larger composite image.

A detailed overview of various scanners (both fluorescent and brightfield) can be found at Farahani et al., Whole slide imaging in pathology: advantages, limitations, and emerging perspectives, Pathology and Laboratory Medicine Int'l, Vol. 7, p. 23-33 (June 2015). Examples of commercially available slide scanners include: 3DHistech PANNORAMIC SCAN II; DigiPath PATHSCOPE; Hamamatsu NANOZOOMER RS, HT, and XR; Huron TISSUESCOPE 4000, 4000XT, and HS; Leica SCANSCOPE AT, AT2, CS, FL, and SCN400; Mikroscan D2; Olympus VS120-SL; Omnyx VL4, and VL120; PerkinElmer LAMINA; Philips ULTRA-FAST SCANNER; Sakura Finetek VISIONTEK; Unic PRECICE 500, and PRECICE 600x; VENTANA ISCAN COREO and ISCAN HT; and Zeiss AXIO SCAN.Z1. Other exemplary systems and features can be found in, for example, WO2011-049608) or in U.S. Patent Application No. 61/533,114, filed on Sep. 9, 2011, entitled IMAGING SYSTEMS, CASSETTES, AND METHODS OF USING THE SAME.

In some embodiments, any imaging may be accomplished using any of the systems disclosed in U.S. Patent Nos. 10,317,666 and 10,313,606. The imaging apparatus may be a brightfield imager such as the iScan Coreo^{™} brightfield scanner or the DP200 scanner sold by Ventana Medical Systems, Inc.

In some cases, the images may be analyzed on an image analysis system. Image analysis system may include one or more computing devices such as desktop computers, laptop computers, tablets, smartphones, servers, application-specific computing devices, or any other type(s) of electronic device(s) capable of performing the techniques and operations described herein. In some embodiments, image analysis system may be implemented as a single device. In other embodiments, image analysis system may be implemented as a combination of two or more devices together achieving the various functionalities discussed herein. For example, image analysis system may include one or more server computers and a one or more client computers communicatively coupled to each other via one or more local-area networks and/or wide-area networks such as the Internet. The image analysis system typically includes at least a memory, a processor, and a display. Memory may include any combination of any type of volatile or non-volatile memories, such as random-access memories (RAMs), read-only memories such as an Electrically-Erasable Programmable Read-Only Memory (EEPROM), flash memories, hard drives, solid state drives, optical discs, and the like. It is appreciated that memory can be included in a single device and can also be distributed across two or more devices. Processor may include one or more processors of any type, such as central processing units (CPUs), graphics processing units (GPUs), specialpurpose signal or image processors, field-programmable gate arrays (FPGAs), tensor processing units (TPUs), and so forth. It is appreciated that processor can be included in a single device and can also be distributed across two or more devices. Display may be implemented using any suitable technology, such as LCD, LED, OLED, TFT, Plasma, etc. In some implementations, display may be a touchsensitive display (a touchscreen). Image analysis system also typically includes a software system stored on the memory comprising a set of instructions implementable on the processor, the instructions comprising various image analysis tasks, such as object identification, stain intensity quantification, and the like. Exemplary commercially-available software packages useful in implementing modules as disclosed herein include VENTANA VIRTUOSO; Definiens TISSUE STUDIO, DEVELOPER XD, and IMAGE MINER; and Visopharm BIOTOPIX, ONCOTOPIX, and STEREOTOPIX software packages.

After the specimens are processed, a user can transport specimenbearing slides to the imaging apparatus. In some embodiments, the imaging apparatus is a brightfield imager slide scanner. One brightfield imager is the iScan Coreo brightfield scanner sold by Ventana Medical Systems, Inc. In automated embodiments, the imaging apparatus is a digital pathology device as disclosed in International Patent Application No.: PCT/US2010/002772 (Patent Publication No.: WO/2011/049608) entitled IMAGING SYSTEM AND TECHNIQUES or disclosed in U.S. Patent Application No. 61/533,114, filed on Sep. 9, 2011, entitled IMAGING SYSTEMS, CASSETTES, AND METHODS OF USING THE SAME. International Patent Application No. PCT/US2010/002772 and U.S. Patent Application No. 61/533,114.

Embodiments of the subject matter and the operations described in this specification can be implemented in digital electronic circuitry, or in computer software, firmware, or hardware, including the structures disclosed in this specification and their structural equivalents, or in combinations of one or more of them. Embodiments of the subject matter described in this specification can be implemented as one or more computer programs, for example, one or more modules of computer program instructions, encoded on computer storage medium for execution by, or to control the operation of, data processing apparatus. Any of the modules described herein may include logic that is executed by the processor(s). "Logic," as used herein, refers to any information having the form of instruction signals and/or data that may be applied to affect the operation of a processor. Software is an example of logic.

A computer storage medium can be, or can be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or can be included in, one or more separate physical components or media (e.g., multiple CDs, disks, or other storage devices). The operations described in this specification can be implemented as operations performed by a data processing apparatus on data stored on one or more computer-readable storage devices or received from other sources.

The term "programmed processor" encompasses all kinds of apparatus, devices, and machines for processing data, including by way of example a programmable microprocessor, a computer, a system on a chip, or multiple ones, or combinations, of the foregoing. The apparatus can include special purpose logic circuitry, e.g., an FPGA (field programmable gate array) or an ASIC (application-specific integrated circuit). The apparatus also can include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing, and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, subprograms, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random-access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

To provide for interaction with a user, embodiments of the subject matter described in this specification can be implemented on a computer having a display device, e.g., an LCD (liquid crystal display), LED (light emitting diode) display, or OLED (organic light emitting diode) display, for displaying information to the user and a keyboard and a pointing device, e.g., a mouse or a trackball, by which the user can provide input to the computer. In some implementations, a touch screen can be used to display information and receive input from a user. Other kinds of devices can be used to provide for interaction with a user as well; for example, feedback provided to the user can be in any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks). For example, the network 20 of FIG. 1 can include one or more local area networks.

The computing system can include any number of clients and servers. A client and server are generally remote from each other and typically interact through a communication network. The relationship of client and server arises by virtue of computer programs running on the respective computers and having a client-server relationship to each other. In some embodiments, a server transmits data (e.g., an HTML page) to a client device (e.g., for purposes of displaying data to and receiving user input from a user interacting with the client device). Data generated at the client device (e.g., a result of the user interaction) can be received from the client device at the server.

### Examples of Biomarkers

Non-limiting examples of biomarkers which may be unmasked are identified herein. Certain markers are characteristic of particular cells, while other markers have been identified as being associated with a particular disease or condition. Examples of known prognostic markers include enzymatic markers such as, for example, galactosyl transferase II, neuron specific enolase, proton ATPase-2, and acid phosphatase. Hormone or hormone receptor markers include human chorionic gonadotropin (HCG), adrenocorticotropic hormone, carcinoembryonic antigen (CEA), prostate-specific antigen (PSA), estrogen receptor, progesterone receptor, androgen receptor, gC1q-R/p33 complement receptor, IL-2 receptor, p75 neurotrophin receptor, PTH receptor, thyroid hormone receptor, and insulin receptor.

Lymphoid markers include alpha-1-antichymotrypsin, alpha-1-antitrypsin, B cell marker, bcl-2, bcl-6, B lymphocyte antigen 36 kD, BM1 (myeloid marker), BM2 (myeloid marker), galectin-3, granzyme B, HLA class I Antigen, HLA class II (DP) antigen, HLA class II (DQ) antigen, HLA class II (DR) antigen, human neutrophil defensins, immunoglobulin A, immunoglobulin D, immunoglobulin G, immunoglobulin M, kappa light chain, kappa light chain, lambda light chain, lymphocyte/histocyte antigen, macrophage marker, muramidase (lysozyme), p80 anaplastic lymphoma kinase, plasma cell marker, secretory leukocyte protease inhibitor, T cell antigen receptor (JOVI 1), T cell antigen receptor (JOVI 3), terminal deoxynucleotidyl transferase, unclustered B cell marker.

Tumor markers include alpha fetoprotein, apolipoprotein D, BAG-1 (RAP46 protein), CA19-9 (sialyl lewisa), CA50 (carcinoma associated mucin antigen), CA125 (ovarian cancer antigen), CA242 (tumor associated mucin antigen), chromogranin A, clusterin (apolipoprotein J), epithelial membrane antigen, epithelial-related antigen, epithelial specific antigen, epidermal growth factor receptor, estrogen receptor (ER), gross cystic disease fluid protein-15, hepatocyte specific antigen, HER2, heregulin, human gastric mucin, human milk fat globule, MAGE-1, matrix metalloproteinases, melan A, melanoma marker (HMB45), mesothelin, metallothionein, microphthalmia transcription factor (MITF), Muc-1 core glycoprotein. Muc-1 glycoprotein, Muc-2 glycoprotein, Muc-5AC glycoprotein, Muc-6 glycoprotein, myeloperoxidase, Myf-3 (Rhabdomyosarcoma marker), Myf-4 (Rhabdomyosarcoma marker), MyoD1 (Rhabdomyosarcoma marker), myoglobin, nm23 protein, placental alkaline phosphatase, prealbumin, progesterone receptor, prostate specific antigen, prostatic acid phosphatase, prostatic inhibin peptide, PTEN, renal cell carcinoma marker, small intestinal mucinous antigen, tetranectin, thyroid transcription factor-1, tissue inhibitor of matrix metalloproteinase 1, tissue inhibitor of matrix metalloproteinase 2, tyrosinase, tyrosinase-related protein-1, villin, von Willebrand factor, CD34, CD34, Class II, CD51 Ab-1, CD63, CD69, Chk1, Chk2, claspin C-met, COX6C, CREB, Cyclin D1, Cytokeratin, Cytokeratin 8, DAPI, Desmin, DHP (1-6 Diphenyl-1,3,5-Hexatriene), E-Cadherin, EEA1, EGFR, EGFRvIII, EMA (Epithelial Membrane Antigen), ER, ERB3, ERCC1, ERK, E-Selectin, FAK, Fibronectin, FOXP3, Gamma-H2AX, GB3, GFAP, Giantin, GM130, Golgin 97, GRB2, GRP78BiP, GSK3 Beta, HER-2, Histone 3, Histone 3_K14-Ace [Anti-acetyl-Histone H3 (Lys 14)], Histone 3_K18-Ace [Histone H3-Acetyl Lys 18), Histone 3_K27-TriMe, [Histone H3 (trimethyl K27)], Histone 3_K4-diMe [Anti-dimethyl-Histone H3 (Lys 4)], Histone 3_K9-Ace [Acetyl-Histone H3 (Lys 9)], Histone 3_K9-triMe [Histone 3-trimethyl Lys 9], Histone 3_S10-Phos [Anti-Phospho Histone H3 (Ser 10), Mitosis Marker], Histone 4, Histone H2A.X-5139-Phos [Phospho Histone H2A.X (Ser139)antibody], Histone H2B, Histone H3_DiMethyl K4, Histone H4_TriMethyl K20-Chip grad, HSP70, Urokinase, VEGF R1, ICAM-1, IGF-1, IGF-1R, IGF-1 Receptor Beta, IGF-II, IGF-IIR, IKB-Alpha IKKE, IL6, IL8, Integrin alpha V beta 3, Integrin alpha V beta6, Integrin Alpha V/CD51, integrin B5, integrin B6, Integrin B8, Integrin Beta 1(CD 29), Integrin beta 3, Integrin beta 5 integrinB6, IRS-1, Jagged 1, Anti-protein kinase C Beta2, LAMP-1, Light Chain Ab-4 (Cocktail), Lambda Light Chain, kappa light chain, M6P, Mach 2, MAPKAPK-2, MEK 1, MEK 1/2 (Ps222), MEK 2, MEK1/2 (47E6), MEK1/2 Blocking Peptide, MET/HGFR, MGMT, Mitochondrial Antigen, Mitotracker Green F M, MMP-2, MMP9, E-cadherin, mTOR, ATPase, N-Cadherin, Nephrin, NFKB, NFKB p105/p50, NF-KB P65, Notch 1, Notch 2, Notch 3, OxPhos Complex IV, p130Cas, p38 MAPK, p44/42 MAPK antibody, P504S, P53, P70, P70 S6K, Pan Cadherin, Paxillin, P-Cadherin, PDI, pEGFR, Phospho AKT, Phospho CREB, phospho EGF Receptor, Phospho GSK3 Beta, Phospho H3, Phospho HSP-70, Phospho MAPKAPK-2, Phospho MEK1/2, phospho p38 MAP Kinase, Phospho p44/42 MAPK, Phospho p53, Phospho PKC, Phospho S6 Ribosomal Protein, Phospho Src, phospho-Akt, Phospho-Bad, Phospho-IKB-a, phospho-mTOR, Phospho-NF-kappaB p65, Phospho-p38, Phospho-p44/42 MAPK, Phospho-p70 S6 Kinase, Phospho-Rb, phospho-Smad2, PIM1, PIM2, PKC β, Podocalyxin, PR, PTEN, R1, Rb 4H1, R-Cadherin, ribonucleotide Reductase, RRM1, RRM11, SLC7A5, NDRG, HTF9C, HTF9C, CEACAM, p33, S6 Ribosomal Protein, Src, Survivin, Synapopodin, Syndecan 4, Talin, Tensin, Thymidylate Synthase, Tuberlin, VCAM-1, VEGF, Vimentin, Agglutinin, YES, ZAP-70 and ZEB.

Cell cycle associated markers include apoptosis protease activating factor-1, bcl-w, bcl-x, bromodeoxyuridine, CAK (cdk-activating kinase), cellular apoptosis susceptibility protein (CAS), caspase 2, caspase 8, CPP32 (caspase-3), CPP32 (caspase-3), cyclin dependent kinases, cyclin A, cyclin B1, cyclin D1, cyclin D2, cyclin D3, cyclin E, cyclin G, DNA fragmentation factor (N-terminus), Fas (CD95), Fas-associated death domain protein, Fas ligand, Fen-1, IPO-38, Mc1-1, minichromosome maintenance proteins, mismatch repair protein (MSH2), poly (ADP-Ribose) polymerase, proliferating cell nuclear antigen, p16 protein, p27 protein, p34cdc2, p57 protein (Kip2), p105 protein, Stat 1 alpha, topoisomerase I, topoisomerase II alpha, topoisomerase III alpha, topoisomerase II beta.

Neural tissue and tumor markers include alpha B crystallin, alphainternexin, alpha synuclein, amyloid precursor protein, beta amyloid, calbindin, choline acetyltransferase, excitatory amino acid transporter 1, GAP43, glial fibrillary acidic protein, glutamate receptor 2, myelin basic protein, nerve growth factor receptor (gp75), neuroblastoma marker, neurofilament 68 kD, neurofilament 160 kD, neurofilament 200 kD, neuron specific enolase, nicotinic acetylcholine receptor alpha4, nicotinic acetylcholine receptor beta2, peripherin, protein gene product 9, S-100 protein, serotonin, SNAP-25, synapsin I, synaptophysin, tau, tryptophan hydroxylase, tyrosine hydroxylase, ubiquitin.

Cluster differentiation markers include CD1a, CD1b, CD1c, CD1d, CD1e, CD2, CD3delta, CD3epsilon, CD3gamma, CD4, CD5, CD6, CD7, CD8alpha, CD8beta, CD9, CD10, CD11a, CD11b, CD11c, CDw12, CD13, CD14, CD15, CD15s, CD16a, CD16b, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD44R, CD45, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CDw75, CDw76, CD77, CD79a, CD79b, CD80, CD81, CD82, CD83, CD84, CD85, CD86, CD87, CD88, CD89, CD90, CD91, CDw92, CDw93, CD94, CD95, CD96, CD97, CD98, CD99, CD100, CD101, CD102, CD103, CD104, CD105, CD106, CD107a, CD107b, CDw108, CD109, CD114, CD115, CD116, CD117, CDw119, CD120a, CD120b, CD121a, CDw121b, CD122, CD123, CD124, CDw125, CD126, CD127, CDw128a, CDw128b, CD130, CDw131, CD132, CD134, CD135, CDw136, CDw137, CD138, CD139, CD140a, CD140b, CD141, CD142, CD143, CD144, CDw145, CD146, CD147, CD148, CDw149, CDw150, CD151, CD152, CD153, CD154, CD155, CD156, CD157, CD158a, CD158b, CD161, CD162, CD163, CD164, CD165, CD166, and TCR-zeta.

Other cellular markers include centromere protein-F (CENP-F), giantin, involucrin, lamin A&C [XB 10], LAP-70, mucin, nuclear pore complex proteins, p180 lamellar body protein, ran, r, cathepsin D, Ps2 protein, Her2-neu, P53, S100, epithelial marker antigen (EMA), TdT, MB2, MB3, PCNA, and Ki67.

### Suitable Unmasking Agents

As used herein, the term "unmasking agent" refers to any liquids, including solutions and mixtures, dispensed to a specimen to assist in unmasking. In some embodiments, the unmasking agent includes multiple components, such as the components recited below. In some embodiments, the unmasking agent is a buffer solution. In some embodiments, the buffer solution has a pH ranging from between about 5 and about 10. In other embodiments, the buffer solution has a pH ranging from between about 7 and about 9. In other embodiments, the buffer solution has a pH ranging from between about 7.5 and about 11. Non-liming examples of buffers include citric acid, potassium dihydrogen phosphate, boric acid, diethyl barbituric acid, piperazine-N,N'-bis(2-ethanesulfonic acid), dimethylarsinic acid, 2-(N-morpholino)ethanesulfonic acid, tris(hydroxymethyl)methylamine (TRIS), 2-(N-morpholino)ethanesulfonic acid (TAPS), N,N-bis(2-hydroxyethyl)glycine(Bicine), N-tris(hydroxymethyl)methylglycine (Tricine), 4-2-hydroxyethyl-1-piperazineethanesulfonic acid (HEPES), 2-{[tris(hydroxymethyl)methyl]amino }ethanesulfonic acid (TES), and combinations thereof. In some embodiments, the unmasking agent is water. In other embodiments, the buffer solution may be comprised of tris(hydroxymethyl)methylamine (TRIS), 2-(N-morpholino)ethanesulfonic acid (TAPS), N,N-bis(2-hydroxyethyl)glycine(Bicine), N -tris(hydroxymethyl)methylglycine (Tricine), 4-2-hydroxyethyl-1-piperazineethanesulfonic acid (HEPES), 2-{[tris(hydroxymethyl)methyl]amino }ethanesulfonic acid (TES), or a combination thereof.

In some embodiments, the unmasking agent includes a TRIS-based buffer with a basic pH. In some embodiments, the TRIS-based buffer has a pH of about 10 (such as at elevated temperatures). In yet other embodiments, the buffer solution may be a citric acid sodium phosphate buffer solution having a pH of approximately 6.0 at elevated temperatures. In other embodiments, the unmasking agent includes about 0.05% citraconic anhydride. In other embodiments, the unmasking agent includes about 100 mM TRIS and has a pH of between about 8 and about 10. In other embodiments, the unmasking agent includes about 10 mM citrate, about 2 mM EDTA, and about 0.05% Tween 20, and has a pH of about 6.2. In other embodiments, the unmasking agent includes about 0.01M citrate buffer and has a pH of about 6.0.

In some embodiments, the unmasking agent includes a component which reacts with any liberated fixative to prevent it from reacting again with the sample. Examples a such unmasking agents include purpald or dimedone. Alternatively, or in addition, the unmasking agent can include a component that reacts in a reversible manner with free amino-groups of proteins and thus protects them from reaction with any available formaldehyde, e.g. citraconic anhydride (CCA).

In other embodiments, the unmasking agent includes a chelator. Examples of chelators include, but are not limited to, ethylenediaminetetraacetic acid (EDTA), ethylene glycol-bis(β-aminoethyl ether)-N,N,N',N'-tetraacetic acid (EGTA), EGTA/AM (EGTA, Tetra(acetoxymethyl Ester)), (1,2-bis(o-aminophenoxy)ethane-N,N,N',N'-tetraacetic acid) (BAPTA), BAPTA/AM (EGTA Tetra(acetoxymethyl Ester)), 5,5'-Dimethyl-BAPTA-AM; 1,2-Bis(2-amino-5-methylphenoxy) ethane (MAPTAM), N,N,N',N'-tetrakis(2-pyridylmethyl)ethane-1,2-diamine (TPEN), citrate, or ionophores such as ionomycin or calcimycin, or any combination or mixtures thereof.

In other embodiments, the unmasking agent includes an enzyme. Non-limiting examples of enzymes include proteinases (such as trypsin, chymotrypsin, pepsin, or proteinase K), a nuclease, a glycanases, and a hyaluronidase.

In other embodiments, the unmasking agent includes a chaotropic agent. Non-limiting examples of chaotropic agents include butanol, ethanol, a guanidinium salt (for example, guanidinium hydrochloride or guanidinium thiocyanate), lithium perchlorate, lithium acetate, magnesium chloride, phenol, propanol, thiourea and urea.

In other embodiments, the unmasking agent includes a nucleophile, for example a chemical species that donates an electron pair to an electrophile. Non-limiting examples of nucleophiles include ammonia, primary amines, secondary amines and tertiary amines. Other examples of nucleophiles include hydrazines, alcohols and halogenides.

In other embodiments, the unmasking agent includes a Lewis acid. Non-limiting examples of Lewis acids include metal ions such as iron (III) ions, aluminum ions, magnesium ions or other electron-deficient compounds such as toluenesulfonic acid, boric acid, boron trifluoride, and tartaric acid.

In other embodiments, the unmasking agent can include a surfactant. As used herein, "surfactants" are classified as anionic, cationic, or nonionic, depending on their mode of chemical action. In general, surfactants reduce interfacial tension between two liquids. An example of a surfactant is sodium dodecyl sulfate. Examples of suitable nonionic surfactants include polyethylene glycol monohexadecyl ether, Cetostearyl alcohol, Cetyl alcohol, cocamide diethanolamine, cocamide monoethanolamine, Decyl glucoside, Octylphenoxypolyethoxyethanol, Polyethylene glycol monoisohexadecyl ether, Lauryl glucoside, Nonyl phenoxypolyethoxylethanol, 4-Nonylphenyl-polyethylene glycol, 1-(4-Nonylphenyl)-1,4,7,10,13,16,19,22,25-nonaoxaheptacosan-27-ol, nonoxynols, Monolaurin, Octaethylene glycol monododecyl ether, Oleyl alcohol, Polyethylene-polypropylene glycol, Polyglycerol polyricinoleate, Polysorbates, Sorbitan monostearate. Sorbitan tristearate; Stearyl alcohol; polyethylene glycol p-(1,1,3,3-tetramethylbutyl)-phenyl ether; and Polyoxyethylene (20) sorbitan monooleate. octyl-, decyl, dodecyl-glucopyranoside, -maltoside or deoxycholic acid. Exemplary surfactants are sold under the names: Brij^{®} 35, TWEEN^{®}, Tergitol^{™}, Triton^{™}, Ecosurf^{™}, Dowfax^{™}, polysorbate 80^{™}, BigCHAP, Deoxy BigCHAP, IGEPAL^{®}, Saponin, Thesit^{®}, Nonidet^{®}, Pluronic F-68, digitonin, deoxycholate, and the like. Particular disclosed working embodiments concern using surfactants selected from Brij^{®} 35, TWEEN^{®}, Tergitol^{™}, Triton^{™}. Additional antigen unmasking agents are disclosed in U.S. Patent No. 8,486,335.

In some embodiments, the unmasking agent includes deionized water, TRIS, and a chelator. In some embodiments, the unmasking agent includes deionized water, TRIS, and a chelator and has a pH ranging from between about 7 to about 9.5. In some embodiments, the unmasking agent includes deionized water, TRIS, a chelator, and a preservative. In some embodiments, the unmasking agent is CC1, available from Ventana Medical Systems, Inc., Tucson, AZ, USA. Further unmasking agents are further herein and described in United States Patent Publication No. 2009/0170152.

### Additional Embodiments

In another aspect of the present disclosure is a system for predicting an unmasking status of a test biological specimen treated in an unmasking process, the system comprising: (i) one or more processors, and (ii) one or more memories coupled to the one or more processors, the one or more memories to store computer-executable instructions that, when executed by the one or more processors, cause the system to perform operations comprising: obtaining test spectral data from the test biological specimen, wherein the test spectral data comprises vibrational spectral data derived from at least a portion of the biological specimen; deriving unmasking features from the obtained test spectral data using a trained unmasking status estimation engine, wherein the unmasking status estimation engine is trained using training spectral data sets acquired from a plurality of differentially unmasked training biological specimens and wherein the training spectral data sets comprise class labels of at least unmasking duration and unmasking temperature; and predicting the unmasking status of the test biological specimen based on the derived unmasking features.

In some embodiments, the unmasking status comprises at least one of a predicted duration of unmasking, a predicted temperature of unmasking, and an estimate of tissue quality. In some embodiments, the unmasking status comprises both a predicted duration of unmasking and a predicted temperature of unmasking. In some embodiments, each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions. In some embodiments, the different unmasking conditions comprise holding a temperature of unmasking constant while varying the duration of unmasking. In some embodiments, the different unmasking conditions comprise holding a duration of unmasking constant while varying the temperature of unmasking. In some embodiments, the training biological specimens comprise the same tissue type as the test biological specimen. In some embodiments, the training biological specimens comprise a different tissue type than the test biological specimen. In some embodiments, the unmasking process is an antigen retrieval process. In some embodiments, the unmasking process is a target retrieval process.

In another aspect of the present disclosure is a method for predicting an unmasking status of a test biological specimen treated in an unmasking process comprising: obtaining test spectral data from the test biological specimen, wherein the test spectral data comprises vibrational spectral data derived from at least a portion of the biological specimen; deriving unmasking features from the obtained test spectral data using a trained unmasking status estimation engine; and predicting the unmasking status of the test biological specimen based on the derived unmasking features. In some embodiments, the unmasking status comprises one of a predicted duration of unmasking or a predicted temperature of unmasking. In some embodiments, the unmasking status comprises both a predicted duration of unmasking and a predicted temperature of unmasking. In some embodiments, the unmasking status further comprises an estimate of tissue quality. In some embodiments, the unmasking status estimation engine is trained using one or more training spectral data sets, wherein each training spectral data set comprises a plurality of training vibrational spectra derived from a plurality of differentially unmasked training tissue samples, and wherein each training vibrational spectrum comprises one or more class labels. In some embodiments, the one or more class labels are selected from a known unmasking duration, a known unmasking temperature, and a qualitative assessment of an unmasking state. In some embodiments, each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions. In some embodiments, the different unmasking conditions comprise holding a temperature of unmasking constant while varying the duration of unmasking. In some embodiments, the different unmasking conditions comprise holding a duration of unmasking constant while varying the temperature of unmasking. In some embodiments, the test spectral data comprises an averaged vibrational spectrum derived from a plurality of normalized and corrected vibrational spectra.

In another aspect of the present disclosure is a non-transitory computer-readable medium storing instructions for predicting an unmasking status of a test biological specimen treated in an unmasking process comprising: obtaining test spectral data from the test biological specimen, wherein the test spectral data comprises vibrational spectral data derived from at least a portion of the biological specimen; deriving unmasking features from the obtained test spectral data using a trained unmasking status estimation engine; and predicting the unmasking status of the test biological specimen based on the derived unmasking features. In some embodiments, the unmasking status comprises one of a predicted duration of unmasking or a predicted temperature of unmasking. In some embodiments, the unmasking status comprises both a predicted duration of unmasking and a predicted temperature of unmasking. In some embodiments, the unmasking status further comprises an estimate of tissue quality. In some embodiments, the unmasking status estimation engine is trained using one or more training spectral data sets, wherein each training spectral data set comprises a plurality of training vibrational spectra derived from a plurality of differentially unmasked training tissue samples, and wherein each training vibrational spectrum comprises one or more class labels. In some embodiments, the one or more class labels are selected from a known unmasking duration, a known unmasking temperature, and a qualitative assessment of an unmasking state. **In** some embodiments, each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions. **In** some embodiments, the different unmasking conditions comprise holding a temperature of unmasking constant while varying the duration of unmasking. **In** some embodiments, the different unmasking conditions comprise holding a duration of unmasking constant while varying the temperature of unmasking. **In** some embodiments, the test spectral data comprises an averaged vibrational spectrum derived from a plurality of normalized and corrected vibrational spectra.

## Claims

1. A system (200) for predicting an unmasking status of a test biological specimen treated in an unmasking process for retrieving antigens or targets and/or improving detection of antigens, amino acids, peptides, proteins, nucleic acids, and/or other targets in fixed tissue, the system comprising: (i) one or more processors (209), and (ii) one or more memories (201) coupled to the one or more processors (209), the one or more memories (201) to store computer-executable instructions that, when executed by the one or more processors (201), cause the system (200)to perform operations comprising:
a. obtaining test spectral data from the test biological specimen, wherein the test spectral data comprises vibrational spectral data derived from at least a portion of the biological specimen;
b. deriving unmasking features from the obtained test spectral data using a trained unmasking status estimation engine (210), wherein the unmasking status estimation engine is trained using one or more training spectral data sets, wherein each training spectral data set of the one or more training spectral data sets comprises a plurality of training vibrational spectra derived from a plurality of differentially unmasked training tissue samples, and wherein each training spectral data set comprises one or more class labels, wherein the one or more class labels are selected from a known unmasking duration, a known unmasking temperature, and a qualitative assessment of an unmasking state; and
c. predicting the unmasking status of the test biological specimen based on the derived unmasking features
wherein the unmasking status comprises a predicted duration of unmasking and/or a predicted temperature of unmasking.

2. The system of claim 1, wherein the unmasking status further comprises an estimate of tissue quality.

3. The system of claim 1, wherein each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions, wherein the different unmasking conditions comprise holding a temperature of unmasking constant while varying the duration of unmasking or wherein the different unmasking conditions comprise holding a duration of unmasking constant while varying the temperature of unmasking.

4. The system of any one of the preceding claims, wherein the obtained test spectral data comprises an averaged vibrational spectrum derived from a plurality of normalized and corrected vibrational spectra.

5. The system of claim 4, wherein the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region.

6. The system of claim 5, wherein the acquired vibrational spectrum from each individual region is corrected by: (i) compensating each acquired vibrational spectrum for atmospheric effects to provide an atmospheric corrected vibrational spectrum; and (ii) compensating the atmospheric corrected vibrational spectrum for scattering.

7. The system of any one of the preceding claims, wherein the trained unmasking status estimation engine comprises a machine learning algorithm based on dimensionality reduction.

8. The system of claim 7, wherein the dimensionality reduction comprises a projection onto latent structure regression model.

9. The system of claim 7, wherein the dimensionality reduction comprises a principal component analysis plus discriminant analysis.

10. The system of any one of claims 1 to 6, wherein the trained unmasking status estimation engine comprises a neural network.

11. The system of any one of the preceding claims, further comprising operations for assessing whether test biological specimen is suitable for labeling with one or more specific binding entities.

12. The system of any one of the preceding claims, wherein the obtained test spectral data comprises vibrational spectral information for at least an amide I band.

13. The system of any one of claims 1 to 11, wherein the obtained test spectral data comprises vibrational spectral information for wavelengths ranging from between about 3200 to about 3400 cm⁻¹, about 2800 to about 2900 cm⁻¹, about 1020 to about 1100 cm⁻¹, and/or about 1520 to about 1580 cm⁻¹.

14. A non-transitory computer-readable medium storing instructions for predicting an unmasking status of a test biological specimen treated in an unmasking process for retrieving antigens or targets and/or improving detection of antigens, amino acids, peptides, proteins, nucleic acids, and/or other targets in fixed tissue comprising:
(a) obtaining test spectral data from the test biological specimen, wherein the test spectral data comprises vibrational spectral data derived from at least a portion of the biological specimen;
(b) deriving unmasking features from the obtained test spectral data using a trained unmasking status estimation engine (210), wherein the unmasking status estimation engine is trained using training spectral data sets acquired from a plurality of differentially unmasked training biological specimens and wherein the training spectral data sets comprise class labels of at least unmasking duration and unmasking temperature; and
(c) predicting the unmasking status of the test biological specimen based on the derived unmasking features,
wherein the unmasking status comprises a predicted duration of unmasking and/or a predicted temperature of unmasking.

15. The non-transitory computer-readable medium of claim 14, wherein the unmasking status comprises an estimate of tissue quality.

16. The non-transitory computer-readable medium of any one of claims 14 to 15, wherein each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions, wherein the different unmasking conditions comprise holding a temperature of unmasking constant while varying the duration of unmasking or wherein the different unmasking conditions comprise holding a duration of unmasking constant while varying the temperature of unmasking.

17. The non-transitory computer-readable medium of any one of claims 14 to 16, wherein the unmasking process is an antigen retrieval process or wherein the unmasking process is a target retrieval process.

18. A method for predicting an unmasking status of a test biological specimen treated in an unmasking process for retrieving antigens or targets and/or improving detection of antigens, amino acids, peptides, proteins, nucleic acids, and/or other targets in fixed tissue comprising:
a. obtaining test spectral data from the test biological specimen (320), wherein the test spectral data comprises vibrational spectral data derived from at least a portion of the biological specimen;
b. deriving unmasking features from the obtained test spectral data using a trained unmasking status estimation engine (340), wherein the unmasking status estimation engine is trained using training spectral data sets acquired from a plurality of differentially unmasked training biological specimens and wherein the training spectral data sets comprise class labels of at least unmasking duration and unmasking temperature; and
c. predicting at least one of a duration or a temperature of the unmasking process to which the test biological specimen was subjected based on the derived unmasking features (350),
wherein the unmasking status comprises a predicted duration of unmasking and/or a predicted temperature of unmasking.

19. The method of claim 18, wherein the unmasking process is an antigen retrieval process or wherein the unmasking process is a target retrieval process.

20. The method of any one of claims 18 to 19, further comprising estimating at least one of tissue quality and damage incurred during the unmasking process.

21. The method of any one of claims 18 to 20, wherein the trained unmasking status estimation engine comprises a machine learning algorithm based on a projection onto latent structure regression model.

22. The method of any one of claims 18 to 20, wherein the trained unmasking status estimation engine comprises a machine learning algorithm based on principal component analysis and discriminate analysis.

23. The method of any one of claims 18 to 20, wherein the trained unmasking status estimation engine comprises a neural network.

24. The method of claim 18, wherein each training spectral data set is derived by: (i) obtaining a training biological specimen; (ii) dividing the obtained training biological specimen into a plurality of training tissue samples; and (iii) unmasking each training tissue sample of the plurality of training tissue samples under different unmasking conditions, wherein the different unmasking conditions comprise holding a temperature of unmasking constant while varying the duration of unmasking or wherein the different unmasking conditions comprise holding a duration of unmasking constant while varying the temperature of unmasking..

## Patentansprüche

1. System (200) zum Vorhersagen eines Demaskierungsstatus eines biologischen Testpräparats, das in einem Demaskierungsverfahren zur Wiedergewinnung von Antigenen oder Targets und/oder zum Verbessern der Detektion von Antigenen, Aminosäuren, Peptiden, Proteinen, Nukleinsäuren und/oder anderen Targets in fixiertem Gewebe behandelt wurde, wobei das System Folgendes umfasst: (i) einen oder mehrere Prozessoren (209) und (ii) einen oder mehrere Speicher (201), die mit dem einen oder den mehreren Prozessoren (209) gekoppelt sind, wobei der eine oder die mehreren Speicher (201) computerausführbare Anweisungen speichern, die, wenn sie von dem einen oder den mehreren Prozessoren (201) ausgeführt werden, das System (200) veranlassen, Vorgänge durchzuführen, die Folgendes umfassen:
a. Erhalten von Testspektraldaten von dem biologischen Testpräparat, wobei die Testspektraldaten Vibrationsspektraldaten umfassen, die von mindestens einem Teil des biologischen Präparats abgeleitet wurden;
b. Ableiten von Demaskierungsmerkmalen aus den erhaltenen Testspektraldaten unter Verwendung einer trainierten Demaskierungsstatus-Schätzungsmaschine (210), wobei die Demaskierungsstatus-Schätzungsmaschine unter Verwendung eines oder mehrerer Trainingsspektraldatensätze trainiert wird, wobei jeder Trainingsspektraldatensatz des einen oder der mehreren Trainingsspektraldatensätze eine Vielzahl von Trainingsvibrationsspektren umfasst, die von einer Vielzahl von differentiell demaskierten Trainingsgewebeproben abgeleitet wurden, und wobei jeder Trainingsspektraldatensatz eine oder mehrere Klassenmarkierungen umfasst, wobei die eine oder die mehreren Klassenmarkierungen aus einer bekannten Demaskierungsdauer, einer bekannten Demaskierungstemperatur und einer qualitativen Beurteilung eines Demaskierungszustands ausgewählt sind; und
c. Vorhersagen des Demaskierungsstatus des biologischen Testpräparats basierend auf den abgeleiteten Demaskierungsmerkmalen,
wobei der Demaskierungsstatus eine vorhergesagte Demaskierungsdauer und/oder eine vorhergesagte Demaskierungstemperatur umfasst.

2. System nach Anspruch 1, wobei der Demaskierungsstatus ferner eine Schätzung der Gewebequalität umfasst.

3. System nach Anspruch 1, wobei jeder Trainingsspektraldatensatz abgeleitet wird durch: (i) Erhalten eines biologischen Trainingspräparats; (ii) Teilen des erhaltenen biologischen Trainingspräparats in eine Vielzahl von Trainingsgewebeproben; und (iii) Demaskieren jeder Trainingsgewebeprobe der Vielzahl von Trainingsgewebeproben unter verschiedenen Demaskierungsbedingungen, wobei die verschiedenen Demaskierungsbedingungen das Konstanthalten einer Demaskierungstemperatur umfassen, während die Demaskierungsdauer variiert wird, oder wobei die verschiedenen Demaskierungsbedingungen das Konstanthalten einer Demaskierungsdauer umfassen, während die Demaskierungstemperatur variiert wird.

4. System nach einem der vorhergehenden Ansprüche, wobei die erhaltenen Testspektraldaten ein gemitteltes Vibrationsspektrum umfassen, das von einer Vielzahl von normalisierten und korrigierten Vibrationsspektren abgeleitet wurde.

5. System nach Anspruch 4, wobei die Vielzahl von normalisierten und korrigierten Vibrationsspektren erhalten wird durch: (i) Identifizieren einer Vielzahl von räumlichen Regionen innerhalb des biologischen Testpräparats; (ii) Erfassen eines Vibrationsspektrums von jeder einzelnen Region der Vielzahl von identifizierten Regionen; (iii) Korrigieren des erfassten Vibrationsspektrums von jeder einzelnen Region, um ein korrigiertes Vibrationsspektrum für jede einzelne Region bereitzustellen; und (iv) Amplitudennormalisieren des korrigierten Vibrationsspektrums von jeder einzelnen Region auf ein vorbestimmtes globales Maximum, um ein amplitudennormalisiertes Vibrationsspektrum für jede Region bereitzustellen.

6. System nach Anspruch 5, wobei das erfasste Vibrationsspektrum von jeder einzelnen Region korrigiert wird durch: (i) Kompensieren jedes erfassten Vibrationsspektrums für atmosphärische Effekte, um ein atmosphärisches korrigiertes Vibrationsspektrum bereitzustellen; und (ii) Kompensieren des atmosphärischen korrigierten Vibrationsspektrums für Streuung.

7. System nach einem der vorhergehenden Ansprüche, wobei die trainierte Demaskierungsstatus-Schätzungsmaschine einen Maschinenlernalgorithmus, der auf Dimensionalitätsreduktion basiert, umfasst.

8. System nach Anspruch 7, wobei die Dimensionalitätsreduktion ein Projektion-auf-latente-Struktur-Regressionsmodell umfasst.

9. System nach Anspruch 7, wobei die Dimensionalitätsreduktion eine Hauptkomponentenanalyse plus Diskriminanzanalyse umfasst.

10. System nach einem der Ansprüche 1 bis 6, wobei die trainierte Demaskierungsstatus-Schätzungsmaschine ein neuronales Netzwerk umfasst.

11. System nach einem der vorhergehenden Ansprüche, ferner umfassend Vorgänge zur Beurteilung, ob ein biologisches Testpräparat zum Markieren mit einer oder mehreren spezifischen Bindungsentitäten geeignet ist.

12. System nach einem der vorhergehenden Ansprüche, wobei die erhaltenen Testspektraldaten Vibrationsspektralinformationen für mindestens eine Amid-I-Bande umfassen.

13. System nach einem der Ansprüche 1 bis 11, wobei die erhaltenen Testspektraldaten Vibrationsspektralinformationen für Wellenlängen im Bereich von etwa 3200 bis etwa 3400 cm⁻¹, etwa 2800 bis etwa 2900 cm⁻¹, etwa 1020 bis etwa 1100 cm⁻¹ und/oder etwa 1520 bis etwa 1580 cm⁻¹ umfassen.

14. Nichtflüchtiges computerlesbares Medium, das Anweisungen zum Vorhersagen eines Demaskierungsstatus eines biologischen Testpräparats speichert, das in einem Demaskierungsverfahren zur Wiedergewinnung von Antigenen oder Targets und/oder zum Verbessern der Detektion von Antigenen, Aminosäuren, Peptiden, Proteinen, Nukleinsäuren und/oder anderen Targets in fixiertem Gewebe behandelt wurde, umfassend:
(a) Erhalten von Testspektraldaten des biologischen Testpräparats, wobei die Testspektraldaten Vibrationsspektraldaten umfassen, die von mindestens einem Teil des biologischen Präparats abgeleitet wurden;
(b) Ableiten von Demaskierungsmerkmalen aus den erhaltenen Testspektraldaten unter Verwendung einer trainierten Demaskierungsstatus-Schätzungsmaschine (210), wobei die Demaskierungsstatus-Schätzungsmaschine unter Verwendung von Trainingsspektraldatensätzen trainiert wird, die von einer Vielzahl von differentiell demaskierten biologischen Trainingspräparaten erfasst werden, und wobei die Trainingsspektraldatensätze Klassenmarkierungen von zumindest Demaskierungsdauer und Demaskierungstemperatur umfassen; und
(c) Vorhersagen des Demaskierungsstatus des biologischen Testpräparats basierend auf den abgeleiteten Demaskierungsmerkmalen,
wobei der Demaskierungsstatus eine vorhergesagte Demaskierungsdauer und/oder eine vorhergesagte Demaskierungstemperatur umfasst.

15. Nichtflüchtiges computerlesbares Medium nach Anspruch 14, wobei der Demaskierungsstatus eine Schätzung der Gewebequalität umfasst.

16. Nichtflüchtiges computerlesbares Medium nach einem der Ansprüche 14 bis 15, wobei jeder Trainingsspektraldatensatz abgeleitet wird durch: (i) Erhalten eines biologischen Trainingspräparats; (ii) Teilen des erhaltenen biologischen Trainingspräparats in eine Vielzahl von Trainingsgewebeproben; und (iii) Demaskieren jeder Trainingsgewebeprobe der Vielzahl von Trainingsgewebeproben unter verschiedenen Demaskierungsbedingungen, wobei die verschiedenen Demaskierungsbedingungen das Konstanthalten einer Demaskierungstemperatur umfassen, während die Demaskierungsdauer variiert wird, oder wobei die verschiedenen Demaskierungsbedingungen das Konstanthalten einer Demaskierungsdauer umfassen, während die Demaskierungstemperatur variiert wird.

17. Nichtflüchtiges computerlesbares Medium nach einem der Ansprüche 14 bis 16, wobei das Demaskierungsverfahren ein Antigengewinnungsverfahren ist oder wobei das Demaskierungsverfahren ein Target-Wiedergewinnungsverfahren ist.

18. Verfahren zum Vorhersagen eines Demaskierungsstatus eines biologischen Testpräparats, das in einem Demaskierungsverfahren zur Wiedergewinnung von Antigenen oder Targets und/oder zum Verbessern der Detektion von Antigenen, Aminosäuren, Peptiden, Proteinen, Nukleinsäuren und/oder anderen Targets in fixiertem Gewebe behandelt wurde, umfassend:
a. Erhalten von Testspektraldaten von dem biologischen Testpräparat (320), wobei die Testspektraldaten Vibrationsspektraldaten umfassen, die von mindestens einem Teil des biologischen Präparats abgeleitet wurden;
b. Ableiten von Demaskierungsmerkmalen aus den erhaltenen Testspektraldaten unter Verwendung einer trainierten Demaskierungsstatus-Schätzungsmaschine (340), wobei die Demaskierungsstatus-Schätzungsmaschine unter Verwendung von Trainingsspektraldatensätzen trainiert wird, die von einer Vielzahl von differentiell demaskierten biologischen Trainingspräparaten erfasst werden, und wobei die Trainingsspektraldatensätze Klassenmarkierungen von zumindest Demaskierungsdauer und Demaskierungstemperatur umfassen; und
c. Vorhersagen mindestens einer Dauer oder einer Temperatur des Demaskierungsverfahrens, dem das biologische Testpräparat unterzogen wurde, basierend auf den abgeleiteten Demaskierungsmerkmalen (350),
wobei der Demaskierungsstatus eine vorhergesagte Demaskierungsdauer und/oder eine vorhergesagte Demaskierungstemperatur umfasst.

19. Verfahren nach Anspruch 18, wobei das Demaskierungsverfahren ein Antigenwiedergewinnungsverfahren ist oder wobei das Demaskierungsverfahren ein Target-Wiedergewinnungsverfahren ist.

20. Verfahren nach einem der Ansprüche 18 bis 19, ferner umfassend das Schätzen von mindestens einem von Gewebequalität und Schäden, die während des Demaskierungsverfahrens entstanden sind.

21. Verfahren nach einem der Ansprüche 18 bis 20, wobei die trainierte Demaskierungsstatus-Schätzungsmaschine einen Maschinenlernalgorithmus umfasst, der auf einem Projektion-auf-latente-Struktur-Regressionsmodell basiert.

22. Verfahren nach einem der Ansprüche 18 bis 20, wobei die trainierte Demaskierungsstatus-Schätzungsmaschine einen Maschinenlernalgorithmus umfasst, der auf Hauptkomponentenanalyse und Diskriminanzanalyse basiert.

23. Verfahren nach einem der Ansprüche 18 bis 20, wobei die trainierte Demaskierungsstatus-Schätzungsmaschine ein neuronales Netzwerk umfasst.

24. Verfahren nach Anspruch 18, wobei jeder Trainingsspektraldatensatz abgeleitet wird durch: (i) Erhalten eines biologischen Trainingspräparats; (ii) Teilen des erhaltenen biologischen Trainingspräparats in eine Vielzahl von Trainingsgewebeproben; und (iii) Demaskieren jeder Trainingsgewebeprobe der Vielzahl von Trainingsgewebeproben unter verschiedenen Demaskierungsbedingungen, wobei die verschiedenen Demaskierungsbedingungen das Konstanthalten einer Demaskierungstemperatur umfassen, während die Dauer der Demaskierung variiert wird, oder wobei die verschiedenen Demaskierungsbedingungen das Konstanthalten einer Demaskierungsdauer umfassen, während die Demaskierungstemperatur variiert wird.

## Revendications

1. Système (200) permettant de prédire un statut de démasquage d'un échantillon biologique de test traité dans un processus de démasquage pour prélever des antigènes ou des cibles et/ou améliorer la détection d'antigènes, d'acides aminés, de peptides, de protéines, d'acides nucléiques et/ou d'autres cibles dans un tissu fixé, le système comprenant : (i) un ou plusieurs processeurs (209), et (ii) une ou plusieurs mémoires (201) couplées au ou aux processeurs (209), la ou les mémoires (201) pour stocker des instructions exécutables par ordinateur qui, lorsqu'elles sont exécutées par le ou les processeurs (201), amènent le système (200) à réaliser les opérations comprenant :
a. l'obtention de données spectrales de test à partir de l'échantillon biologique de test, les données spectrales de test comprenant des données spectrales vibrationnelles dérivées d'au moins une partie de l'échantillon biologique ;
b. la dérivation de caractéristiques de démasquage à partir des données spectrales de test obtenues en utilisant un moteur d'estimation de statut de démasquage (210) entraîné, le moteur d'estimation de statut de démasquage étant entraîné en utilisant un ou plusieurs ensembles de données spectrales d'entraînement, chaque ensemble de données spectrales d'entraînement du ou des ensembles de données spectrales d'entraînement comprenant une pluralité de spectres vibrationnels d'entraînement dérivés d'une pluralité d'échantillons de tissu d'entraînement démasqués de manière différentielle, et chaque ensemble de données spectrales d'entraînement comprenant un ou plusieurs marqueurs de classe, le ou les marqueurs de classe étant choisis parmi une durée de démasquage connue, une température de démasquage connue et une évaluation qualitative d'un statut de démasquage ; et
c. la prédiction du statut de démasquage de l'échantillon biologique de test en se basant sur les caractéristiques de démasquage dérivées
dans lequel le statut de démasquage comprend une durée de démasquage prédite et/ou une température de démasquage prédite.

2. Système selon la revendication 1, dans lequel le statut de démasquage comprend en outre une estimation de la qualité du tissu.

3. Système selon la revendication 1, dans lequel chaque ensemble de données spectrales d'entraînement est dérivé par : (i) l'obtention d'un échantillon biologique d'entraînement ; (ii) la division de l'échantillon biologique d'entraînement obtenu en une pluralité d'échantillons de tissu d'entraînement; et (iii) le démasquage de chaque échantillon de tissu d'entraînement de la pluralité d'échantillons de tissu d'entraînement dans des conditions de démasquage différentes, dans lequel les conditions de démasquage différentes comprennent le maintien d'une température de démasquage constante tout en faisant varier la durée de démasquage ou dans lequel les conditions de démasquage différentes comprennent le maintien d'une durée de démasquage constante tout en faisant varier la température de démasquage.

4. Système selon l'une quelconque des revendications précédentes, dans lequel les données spectrales de test obtenues comprennent un spectre vibrationnel moyenné dérivé d'une pluralité de spectres vibrationnels normalisés et corrigés.

5. Système selon la revendication 4, dans lequel la pluralité de spectres vibrationnels normalisés et corrigés sont obtenus par : (i) l'identification d'une pluralité de régions spatiales au sein de l'échantillon biologique de test ; (ii) l'acquisition d'un spectre vibrationnel à partir de chaque région individuelle de la pluralité de régions identifiées ; (iii) la correction du spectre vibrationnel acquis à partir de chaque région individuelle pour fournir un spectre vibrationnel corrigé pour chaque région individuelle ; et (iv) la normalisation d'amplitude du spectre vibrationnel corrigé à partir de chaque région individuelle à un maximum global prédéterminé pour fournir un spectre vibrationnel normalisé en amplitude pour chaque région.

6. Système selon la revendication 5, dans lequel le spectre vibrationnel acquis à partir de chaque région individuelle est corrigé par : (i) la compression de chaque spectre vibrationnel acquis pour les effets atmosphériques pour fournir un spectre vibrationnel corrigé atmosphérique ; et (ii) la compensation du spectre vibrationnel corrigé atmosphérique vis-à-vis de la diffusion.

7. Système selon l'une quelconque des revendications précédentes, dans lequel le moteur d'estimation de statut de démasquage entraîné comprend un algorithme d'apprentissage automatique se basant sur la réduction de dimensionnalité.

8. Système selon la revendication 7, dans lequel la réduction de dimensionnalité comprend une projection sur un modèle de régression de structure latente.

9. Système selon la revendication 7, dans lequel la réduction de dimensionnalité comprend une analyse en composantes principales plus une analyse discriminante.

10. Système selon l'une quelconque des revendications 1 à 6, dans lequel le moteur d'estimation de statut de démasquage entraîné comprend un réseau neuronal.

11. Système selon l'une quelconque des revendications précédentes, comprenant en outre des opérations permettant d'évaluer si l'échantillon biologique de test est approprié pour le marquage avec une ou plusieurs entités de liaison spécifique.

12. Système selon l'une quelconque des revendications précédentes, dans lequel les données spectrales de test obtenues comprennent des informations spectrales vibrationnelles pour au moins une bande amide I.

13. Système selon l'une quelconque des revendications 1 à 11, dans lequel les données spectrales de test obtenues comprennent des informations spectrales vibrationnelles pour des longueurs d'onde comprises dans la plage allant d'environ 3200 à environ 3400 cm⁻¹, d'environ 2800 à environ 2900 cm⁻¹, d'environ 1020 à environ 1100 cm⁻¹ et/ou d'environ 1520 à environ 1580 cm⁻¹.

14. Support de stockage lisible par ordinateur non transitoire stockant des instructions permettant de prédire un statut de démasquage d'un échantillon biologique de test traité dans un processus de démasquage pour prélever des antigènes ou des cibles et/ou améliorer la détection d'antigènes, d'acides aminés, de peptides, de protéines, d'acides nucléiques et/ou d'autres cibles dans un tissu fixé comprenant :
(a) l'obtention de données spectrales de test à partir de l'échantillon biologique de test, les données spectrales de test comprenant des données spectrales vibrationnelles dérivées d'au moins une partie de l'échantillon biologique ;
(b) la dérivation de caractéristiques de démasquage à partir des données spectrales de test obtenues en utilisant un moteur d'estimation de statut de démasquage (210) entraîné, le moteur d'estimation de statut de démasquage étant entraîné en utilisant des ensembles de données spectrales d'entraînement acquises à partir d'une pluralité d'échantillons biologiques d'entraînement démasqués de manière différentielle et les ensembles de données spectrales d'entraînement comprenant des marqueurs de classe d'au moins la durée de démasquage et la température de démasquage ; et
(c) la prédiction du statut de démasquage de l'échantillon biologique de test en se basant sur les caractéristiques de démasquage dérivées,
dans lequel le statut de démasquage comprend une durée de démasquage prédite et/ou une température de démasquage prédite.

15. Support lisible par ordinateur non transitoire selon la revendication 14, dans lequel le statut de démasquage comprend une estimation de la qualité du tissu.

16. Support lisible par ordinateur non transitoire selon l'une quelconque des revendications 14 à 15, dans lequel chaque ensemble de données spectrales d'entraînement est dérivé par : (i) l'obtention d'un échantillon biologique d'entraînement ; (ii) la division de l'échantillon biologique d'entraînement obtenu en une pluralité d'échantillons de tissu d'entraînement; et (iii) le démasquage de chaque échantillon de tissu d'entraînement de la pluralité d'échantillons de tissus d'entraînement dans des conditions de démasquage différentes, dans lequel les conditions de démasquage différentes comprennent le maintien d'une température de démasquage constante tout en faisant varier la durée de démasquage ou dans lequel les conditions de démasquage différentes comprennent le maintien d'une durée de démasquage constante tout en faisant varier la température de démasquage.

17. Support lisible par ordinateur non transitoire selon l'une quelconque des revendications 14 à 16, dans lequel le processus de démasquage est un processus de prélèvement d'antigène ou dans lequel le processus de démasquage est un processus de prélèvement de cible.

18. Procédé permettant de prédire un statut de démasquage d'un échantillon biologique de test dans un processus de démasquage permettant de prélever des antigènes ou des cibles et/ou d'améliorer la détection d'antigènes, d'acides aminés, de peptides, de protéines, d'acides nucléiques et/ou d'autres cibles dans un tissu fixé comprenant :
a. l'obtention de données spectrales de test à partir de l'échantillon biologique de test (320), les données spectrales de test comprenant des données spectrales vibrationnelles dérivées d'au moins une partie de l'échantillon biologique ;
b. la dérivation de caractéristiques de démasquage à partir des données spectrales de test obtenues en utilisant un moteur d'estimation de statut de démasquage (340) entraîné, le moteur d'estimation de statut de démasquage étant entraîné en utilisant des ensembles de données spectrales d'entraînement acquises à partir d'une pluralité d'échantillons biologiques d'entraînement démasqués de manière différentielle et les ensembles de données spectrales d'entraînement comprenant des marqueurs de classe d'au moins la durée de démasquage et la température de démasquage ; et
c. la prédiction d'au moins une parmi une durée ou une température du processus de démasquage auquel l'échantillon biologique de test a été soumis en se basant sur les caractéristiques de démasquage dérivées (350),
dans lequel le statut de démasquage comprend une durée de démasquage prédite et/ou une température de démasquage prédite.

19. Procédé selon la revendication 18, dans lequel le processus de démasquage est un processus de prélèvement d'antigène ou dans lequel le processus de démasquage est un processus de prélèvement de cible.

20. Procédé selon l'une quelconque des revendications 18 à 19, comprenant en outre l'estimation d'au moins un parmi la qualité du tissu et les dommages subis pendant le processus de démasquage.

21. Procédé selon l'une quelconque des revendications 18 à 20, dans lequel le moteur d'estimation de statut de démasquage entraîné comprend un algorithme d'apprentissage machine basé sur une projection sur un modèle de régression de structure latente.

22. Procédé selon l'une quelconque des revendications 18 à 20, dans lequel le moteur d'estimation de statut de démasquage entraîné comprend un algorithme d'apprentissage automatique se basant sur une analyse en composantes principales et une analyse discriminante.

23. Procédé selon l'une quelconque des revendications 18 à 20, dans lequel le moteur d'estimation de statut de démasquage entraîné comprend un réseau neuronal.

24. Procédé selon la revendication 18, dans lequel chaque ensemble de données spectrales d'entraînement est dérivé par : (i) l'obtention d'un échantillon biologique d'entraînement ; (ii) la division de l'échantillon biologique d'entraînement obtenu en une pluralité d'échantillons de tissu d'entraînement ; et (iii) le démasquage de chaque échantillon de tissu d'entraînement de la pluralité d'échantillons de tissu d'entraînement dans des conditions de démasquage différentes, dans lequel les conditions de démasquage différentes comprennent le maintien d'une température de démasquage constante tout en faisant varier la durée de démasquage ou dans lequel les conditions de démasquage différentes comprennent le maintien d'une durée de démasquage constante tout en faisant varier la température de démasquage.
